# EUROPEAN PATENT APPLICATION

(11) **EP 4 810 484 A1**
(43) Date of publication of application: **23.09.2026**
(21) Application number: 23958572.2
(22) Date of filing: 16.11.2023
(51) Int. Cl.: C07D 213/84, A61K 31/44, A61P 25/28

(54) **NEUROACTIVE ANDROGEN RECEPTOR MODULATOR AND USE THEREOF**

(71) Applicant: Endochem Pharmaceuticals (Beijing) Co., Ltd., Beijing 100070 (CN)
(72) Inventor: HE, Yali, Beijing 102206 (CN); CUI, Mutian, Beijing 102206 (CN); SHI, Yang, Beijing 102206 (CN); WANG, Hongquan, Beijing 100070 (CN); LI, Weijian, Beijing 102206 (CN); GUO, Xiuyun, Beijing 102206 (CN); ZHENG, Hanyu, Beijing 102206 (CN); LIANG, Dongming, Beijing 102206 (CN); YU, Lijun, Beijing 100070 (CN)
(74) Representative: Metida
(86) International application number: PCT/CN2023/131994
(87) International publication number: WO 2025/102293

(57) **Abstract**

The present disclosure relates to neuroactive androgen receptor modulators and use thereof. Specifically, it relates to novel arylpropionamide compounds of Formula I or pharmaceutically acceptable salts, stereoisomers, solvates, or isotopic derivatives thereof, pharmaceutical compositions including such compounds, and the use of such compounds and pharmaceutical compositions including such compounds for the prevention and/or treatment of androgen-related diseases, particularly central nervous system diseases. The compounds of Formula I described in the present disclosure exhibit good androgen receptor binding affinity and androgen receptor modulatory capabilities, and can penetrate the blood-brain barrier, thereby exerting androgen receptor modulatory functions within the brain.

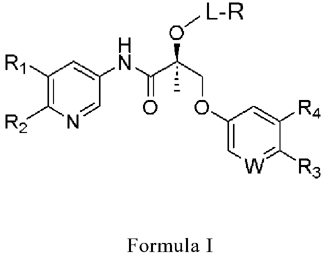

## Description

### TECHNICAL FIELD

The present disclosure relates to the field of pharmaceutical technology and relates to neuroactive androgen receptor modulators and use thereof. Specifically, the present disclosure relates to novel arylpropionamide compounds or the pharmaceutically acceptable salts, stereoisomers, solvates or isotopic derivatives thereof, to pharmaceutical compositions including such compounds, and to the use of such compounds and pharmaceutical compositions including such compounds for the prevention and/or treatment of androgen-related diseases, particularly central nervous system diseases.

### BACKGROUND

The androgen receptor (AR) belongs to the nuclear receptor family and is a receptor for ligand-induced nuclear transcription factors. As an important cellular regulatory protein, the androgen receptor plays a vital role in a series of physiological processes through endogenous androgens, including the development and maintenance of male secondary sexual characteristics, such as muscle and bone mass, male hair growth, prostate growth, and sperm development. Endogenous steroidal androgens, known as male sex hormones, include testosterone and dihydrotestosterone (DHT). Testosterone is the predominant steroidal androgen found in male serum, and is mainly secreted by the testes. In many peripheral tissues, such as the prostate and skin, testosterone can be converted into the more potent androgen i.e., dihydrotestosterone (DHT) by 5α-reductase.

Many diseases are related to androgen levels. As men age, androgen levels in the body gradually decline, accompanied by reduced muscle mass, osteoporosis, decreased sexual function, and cognitive decline. Conversely, excessively high androgen levels can also lead to androgen-related diseases such as prostate cancer, benign prostatic hyperplasia, acne, hirsutism, and hair loss.

Testosterone and its active metabolite dihydrotestosterone (DHT) exert numerous important effects in the brain, including stimulating neuronal differentiation, maintaining neuronal morphology, increasing synaptic density, modulating neuronal activity, and providing neuroprotection. The androgenic effects produced by androgens such as testosterone are regulated by the activation of androgen receptors. AR is predominantly distributed in multiple regions of the brain, including the amygdala, hippocampus, and other regions associated with learning and memory.

Androgens are believed to be involved in various aspects of cognition, including verbal fluency and spatial cognitive ability. Reduced androgen expression is often associated with cognitive impairment to a certain extent. Androgens are correlated with cognitive function not only in elderly males but also in elderly females. Many studies have shown that decreased androgen levels affect a variety of related neurobehaviors and increase the risk of brain diseases, particularly the risk of developing Alzheimer's disease (AD). Conventional androgen therapies are effective in ameliorating the above symptoms but are associated with side effects such as prostate cancer induction. In contrast, selective androgen receptor modulators (SARMs) exhibit high specificity and mild side effects, thus offering greater therapeutic advantages. However, there have been no reports in the literature regarding the use of SARMs to treat central nervous system diseases, nor have existing SRAMs been reported to penetrate the blood-brain barrier.

In recent years, arylpropionamide compounds have been identified as a class of selective androgen receptor modulators with excellent activity, and remarkable progress has been made in the treatment of prostate cancer, breast cancer and other diseases (see Ramesh, N. et al., Selective Androgen Receptor Modulators (SARMs) Negatively Regulate Triple-Negative Breast Cancer Growth and Epithelial: Mesenchymal Stem Cell Signaling, PLoS One, 2014. 9(7): e103202). At present, arylpropionamide nonsteroidal selective androgen receptor agonists such as Ostarine (also known as enobosarm, S-22, MK-2866, or GTx-024) and Andarine have been used in multiple clinical studies for various diseases (see Machek, S.B et al., Considerations, Possible Contraindications, and Potential Mechanisms for Deleterious Effect in Recreational and Athletic Use of Selective Androgen Receptor Modulators (SARMs) in Lieu of Anabolic Androgenic Steroids: A Narrative Review, Steroids, 2020(164): 108753). However, it has not been reported that these compounds can penetrate the blood-brain barrier (see Miner, JN et al., An Orally Active Selective Androgen Receptor Modulator Is Efficacious on Bone, Muscle, and Sex Function with Reduced Impact on Prostate. Endocrinology, 2007. 148(1): 363-373; Piu, F. et al., Pharmacological Characterization of AC-262536, A Novel Selective Androgen Receptor Modulator. The Journal of Steroid Biochemistry and Molecular Biology, 2008. 109(1):129-137; Pharmacokinetics and Pharmacodynamics of LGD-3303[9-Chloro-2-Ethyl-1-Methyl-3-(2,2,2-Trifluoroethyl)-3H-Pyrrolo-[3,2-f]Quinolin-7(6H)-One], An Orally Available Nonsteroidal-Selective Androgen Receptor Modulator. J Pharmacol Exp Ther, 2009. 328(2): 663-670). In addition, no compounds known as SARM have advanced clinical trials for central nervous system diseases indications. Meanwhile, the androgen receptor agonistic potency of the above compounds still leaves room for improvement. Therefore, developing neuroactive androgen receptor modulators (NARMs) that can penetrate the blood-brain barrier and possess higher agonistic efficacy could overcome the drawbacks of the above two types of therapeutic approaches, which is expected to become a novel therapeutic strategy for central nervous system diseases targeting androgen receptors.

### SUMMARY

The object of the present disclosure is to provide a class of neuroactive androgen receptor modulators (NARMs) capable of penetrating the blood-brain barrier and exhibiting higher agonistic potency. Novel structural modifications on conventional arylpropionamide compounds have been conducted through a series of modern pharmaceutical research and development techniques, so as to obtain a new class of arylpropionamide compounds. These compounds exhibit higher agonistic potency on androgen receptors, can effectively penetrate the blood-brain barrier, maintain a certain drug concentration in the brain, and exert androgen receptor regulatory functions, thus better meeting the new requirements for the treatment of central nervous system diseases.

The first aspect of the present disclosure provides compounds of Formula I or pharmaceutically acceptable salts, stereoisomers, solvates, or isotopic derivatives thereof:

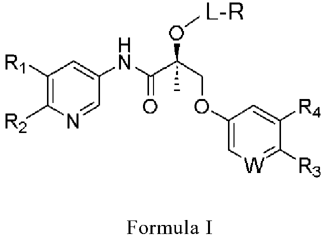

Wherein:
R₁ and R₂ are each independently cyano, halogen, C₁-C₆ haloalkyl, nitro or -NR₅R₆;
R₃ and R₄ are each independently hydrogen, cyano, halogen, C₁-C₆ haloalkyl, nitro, -NR₅R₆, -C(O)C₁-C₆ alkyl, -N(R₇)C(O)C₁-C₆ alkyl, -N(R₇)C(O)-C₁-C₆ haloalkyl, -C₁-C₆ alkyl-C(O)C₁-C₆ alkyl, -S(O)₂-C₁-C₆ alkyl, -N(R₇)-S(O)₂-C₁-C₆ alkyl, C₁-C₆ alkyl, or C₁-C₆ alkoxy.
W is CH or N;
L represents a bond, -C(O)-, -C(O)O-, -S(O)₂-, or -C(O)NH-;
R is hydrogen, C₁-C₆ alkyl, C₃-C₈ cycloalkyl, C₆-C₁₀ aryl, 3- to 12-membered heterocyclyl, 3- to 12-membered heterocyclyl-C₁-C₆ alkyl, 5- to 12-membered heteroaryl, -C₁-C₆ alkyl-NR₅R₆, glucosyl, or amino acid, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents each independently selected from C₁-C₆ alkyl, C₁-C₆ alkoxy, halogen, or cyano; and
R₅, R₆ and R₇ are each independently hydrogen or C₁-C₆ alkyl.

In some embodiments, the compound has the following Formula II: 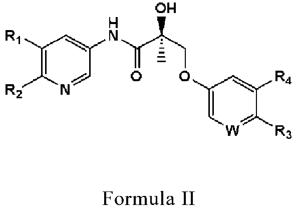 R₁, R₂, R₃, R₄ and W are as defined above for the compound of Formula I.

In some embodiments, the compound has the following Formula III: 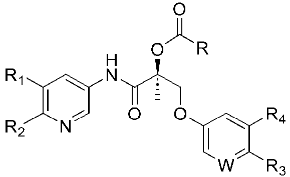 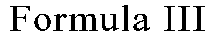 R₁, R₂, R₃, R₄, W, and R are as defined above for the compound of Formula I, provided that R is not hydrogen.

In some embodiments, the compound has the following Formula IV: 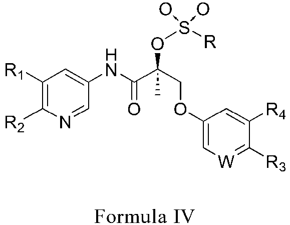 R₁, R₂, R₃, R₄, W, and R are as defined above for compounds of Formula I, provided that R is not hydrogen.

A second aspect of the disclosure provides a pharmaceutical composition including a compound of Formula I or a pharmaceutically acceptable salt, stereoisomer, solvate or isotopic derivative thereof, and one or more pharmaceutically acceptable carriers.

A third aspect of the present disclosure provides a compound of Formula I, a pharmaceutically acceptable salt, stereoisomer, solvate or isotopic derivative thereof, or a pharmaceutical composition including a compound of Formula I, a pharmaceutically acceptable salt, stereoisomer, solvate or isotopic derivative thereof and one or more pharmaceutically acceptable carriers, for use in the prevention and /or treatment of androgen-related diseases, particularly central nervous system diseases.

The fourth aspect of the disclosure provides the use of a compound of Formula I or a pharmaceutically acceptable salt, stereoisomer, solvate or isotopic derivative thereof, or a pharmaceutical composition including a compound of Formula I or a pharmaceutically acceptable salt, stereoisomer, solvate or isotopic derivative thereof and one or more pharmaceutically acceptable carriers for the prevention and/or treatment of androgen-related diseases, particularly central nervous system diseases.

The fifth aspect of the disclosure provides the use of a compound of Formula I or a pharmaceutically acceptable salt, stereoisomer, solvate or isotopic derivative thereof, or a pharmaceutical composition including a compound of Formula I or a pharmaceutically acceptable salt, stereoisomer, solvate or isotopic derivative thereof and one or more pharmaceutically acceptable carriers in the preparation of a medicament for the prevention and/or treatment of androgen-related diseases, particularly central nervous system diseases.

A sixth aspect of the disclosure provides a method for preventing and/or treating androgen - related diseases, particularly central nervous system diseases, in subjects in need thereof, the method including administering to the subject an effective amount of a compound of Formula I or a pharmaceutically acceptable salt, stereoisomer, solvate, or isotopic derivative thereof, or a pharmaceutical composition including a compound of Formula I or a pharmaceutically acceptable salt, stereoisomer, solvate, or isotopic derivative thereof and one or more pharmaceutically acceptable carriers.

In the above three to six aspects, the central nervous system diseases are dementia (including Alzheimer's disease), cognitive impairment associated with schizophrenia, Parkinson's disease, Huntington's disease, depression, anxiety, stroke, cerebral ischemia, amyotrophic lateral sclerosis, traumatic brain injury, Fragile X syndrome, Rett syndrome, brain tumor, or obesity.

### BRIEF DESCRIPTION OF DRAWINGS

FIG. 1 shows the concentration-time profile of the compound from Example 3 in mouse serum over 0-240 min after administration.
FIG. 2 shows the concentration-time profile of the compound from Example 3 in mouse brain over 0-240 min after administration.
FIG. 3 shows a comparison of the concentration-time profiles of the compound from Example 3 in the brain and serum of the mouse over 0-240 min after administration.
FIG. 4 shows the concentration-time profile of the compound from Example 3 in mouse serum over 120-480 min after administration.
FIG. 5 shows the concentration-time profile of the compound from Example 3 in mouse brain over 120-480 min after administration.
FIG. 6 shows a comparison of the concentration-time profiles of the compound from Example 3 in the brain and serum of the mouse over 120-480 min after administration.
FIG. 7 shows a comparison of the concentration-time profiles of the compound from Example 3 in the brain and serum of the mouse over 2-72 h after administration.
FIG. 8 shows a comparison of the concentration-time profiles of the compound from Example 8 in the brain and serum of the mouse over 12-72 h after administration.
FIG. 9 shows the concentration-time profiles of the compound and its metabolites from Example 13 in mouse serum over 0-240 min after administration.
FIG. 10 shows the concentration-time profiles of the compound and its metabolites from Example 13 in mouse brain over 0-240 min after administration.
FIG. 11 shows a comparison of the total concentration-time profiles of the compound and its metabolites from Example 13 in the brain and serum of the mouse over 0-240 min after administration.
FIG. 12 shows a comparison of the total concentration-time profiles of the compound and its metabolites from Example 13, versus those of the compound from Example 3 in the brain and serum of the mouse over 0-240 min after administration.
FIG. 13 shows the concentration-time profiles of the compound and its metabolites from Example 13 in mouse serum over 120-480 min after administration.
FIG. 14 shows the concentration-time profiles of the compound and its metabolites from Example 13 in mouse brain over 120-480 min after administration.
FIG. 15 shows a comparison of the total concentration-time profiles of the compound and its metabolites from Example 13 in the brain and serum of the mouse over 120-480 min after administration.
FIG. 16 shows a comparison of the total concentration-time profiles of the compound and its metabolites from Example 13, versus those of the compound from Example 3 in the brain and serum of the mouse over 120-480 min after administration.
FIG. 17 shows the concentration-time profiles of the compound and its metabolites from Example 15 in mouse serum over 0-240 min after administration.
FIG. 18 shows the concentration-time profiles of the compound and its metabolites from Example 15 in mouse brain over 0-240 min after administration.
FIG. 19 shows a comparison of the total concentration-time profiles of the compound and its metabolites from Example 15 in the brain and serum of the mouse over 0-240 min after administration.
FIG. 20 shows the concentration-time profiles of the compound and its metabolites from Example 21 in mouse serum over 120-480 min after administration.
FIG. 21 shows the concentration-time profiles of the compound and its metabolites from Example 21 in mouse brain over 120-480 min after administration.
FIG. 22 shows a comparison of the total concentration-time profiles of the compound and its metabolites from Example 21 in the brain and serum of the mouse over 120-480 min after administration.
FIG. 23 shows the concentration-time profiles of the compound and its metabolites from Example 22 in mouse serum over 120-480 min after administration.
FIG. 24 shows the concentration-time profiles of the compound and its metabolites from Example 22 in mouse brain over 120-480 min after administration.
FIG. 25 shows a comparison of the total concentration-time profiles of the compound and its metabolites from Example 22 in the brain and serum of the mouse over 120-480 min after administration.
FIG. 26 shows a comparison of the total concentration-time profiles of the compound and its metabolites from Example 24 in the brain and serum of the mouse over 2-72 h after administration.
FIG. 27 shows a comparison of the total concentration-time profiles of the compound and its metabolites from Example 24, versus those of the compound from Example 3 in the brain and serum of the mouse over 2-72 h after administration.
FIG. 28 shows the concentration-time profiles of the compound and its metabolites from Example 26 in mouse serum over 0-240 min after administration.
FIG. 29 shows the concentration-time profiles of the compound and its metabolites from Example 26 in mouse brain over 0-240 min after administration.
FIG. 30 shows a comparison of the total concentration-time profiles of the compound and its metabolites from Example 26 in the brain and serum of the mouse over 0-240 min after administration.
FIG. 31 shows the concentration-time profiles of the compound and its metabolites from Example 28 in mouse serum over 120-480 min after administration.
FIG. 32 shows the concentration-time profiles of the compound and its metabolites from Example 28 in mouse brain over 120-480 min after administration.
FIG. 33 shows a comparison of the total concentration-time profiles of the compound and its metabolites from Example 28 in the brain and serum of the mouse over 120-480 min after administration.
FIG. 34 shows a comparison of the total concentration-time profiles of the compound and its metabolites from Example 28, versus those of the compound from Example 3 in the brain and serum of the mouse over 120-480 min after administration.

### DESCRIPTION OF EMBODIMENTS

### Definitions

Unless otherwise stated, the following terms used in the specification and claims have the meanings set forth below. It should be understood that, in the absence of a specific definition herein, a term should be given a meaning that is well-known in the field. Furthermore, it should be understood that the terminology used herein is for the purpose of describing particular embodiments only and is not intended to limit the scope of the disclosure in any way. Unless otherwise specified, where there is a discrepancy between the structural formula and chemical name of a compound described herein, the structural formula shall prevail.

As used herein, the prefix "Cx-Cy" for a group indicates the range of the number of carbon atoms contained in the group, where x and y are each an integer. For example, C₃-C₈ cycloalkyl refers to cycloalkyl having 3 to 8 carbon atoms, i.e., cycloalkyl having 3, 4, 5, 6, 7, or 8 carbon atoms. It is also to be understood that "C₃-C₈" includes any subrange therein, such as C₃-C₇, C₃-C₆, C₄-C₇, C₄-C₆, C₅-C₆, etc.

The term "alkyl" as used herein refers to saturated linear or branched-chain monovalent hydrocarbon radicals that contain the indicated number of carbon atoms. Alkyl typically contains 1-6 carbon atoms ("C₁-C₆ alkyl"), preferably 1-5 carbon atoms ("C₁-C₅ alkyl"), more preferably 1-4 carbon atoms ("C₁-C₄ alkyl"), 1-3 carbon atoms ("C₁-C₃ alkyl"), or 1-2 carbon atoms ("C₁-C₂ alkyl"). Examples of alkyl include, but are not limited to, methyl, ethyl, n-propyl, isopropyl, n-butyl, sec-butyl, isobutyl, tert-butyl, n-pentyl, isopentyl, neopentyl, and n-hexyl.

The term "alkoxy" as used herein refers to an alkyl linked to a parent molecule via an oxygen atom (i.e.,"-O- alkyl"), wherein the alkyl is as defined above. Alkoxy typically contains 1-6 carbon atoms ("C₁-C₆ alkoxy"), more preferably 1-4 carbon atoms ("C₁-C₄ alkoxy"). Examples of alkoxy include, but are not limited to, methoxy, ethoxy, n-propoxy, isopropoxy, n-butoxy, tert-butoxy, pentoxy, and hexoxy.

The term "halogen" as used herein refers to fluorine, chlorine, bromine, and iodine, with fluorine and chlorine being preferred.

As used herein, the term "halo" means that one or more hydrogen atoms in a substituent are replaced by one or more identical or different halogen atoms as defined above. For example,"C₁-C₆ haloalkyl" means "C₁-C₆ alkyl" in which one or more hydrogen atoms are replaced by one or more identical or different halogen atoms, where "C₁-C₆ alkyl" is as defined above. Examples of C₁-C₆ haloalkyl include, but are not limited to, chloromethyl, fluoromethyl, trifluoromethyl, trichloromethyl, difluoromethyl, and dichloromethyl.

The term "cyano" as used herein refers to the -CN group.

The term "nitro" as used herein refers to the -NO₂ group.

The term "hydroxyl" as used herein refers to the -OH group.

As used herein, the term "aryl" refers to a monovalent hydrocarbon radical derived from a monocyclic, fused bicyclic or polycyclic ring system (where at least one ring contains a fully conjugated π-electron system) having well-known aromatic characteristics. Fused aryl may include aryl rings fused with a saturated or partially unsaturated carbocyclic or heterocyclyl ring, or fused to another aryl or heteroaryl ring, provided that the point of attachment to the parent molecule in such a fused ring system is an atom of the aromatic portion of the ring system. Aryl typically contains 6-14 carbon atoms ("C₆-C₁₄ aryl"), more preferably 6-10 carbon atoms ("C₆-C₁₀ aryl"). Examples of aryl include, but are not limited to, phenyl, naphthyl, anthracenyl, phenanthryl, indanyl, indenyl, and tetrahydronaphthyl.

As used herein, the term "cycloalkyl" refers to a monovalent hydrocarbon group derived from a non-aromatic saturated carbocyclic system containing a indicated number of carbon atoms. Cycloalkyl typically contains 3-8 carbon atoms ("C₃-C₈ cycloalkyl"), preferably 3-7 carbon atoms ("C₃-C₇ cycloalkyl") or 3-6 carbon atoms ("C₃-C₆ cycloalkyl"). Examples of cycloalkyl include, but are not limited to, cyclopropyl, cyclobutyl, cyclopentyl, cyclohexyl, cycloheptyl, and cyclooctyl.

The term "heteroatom" as used herein refers to N, O, or S atoms.

As used herein, the term "heterocyclyl" refers to a monovalent radical derived from a saturated or partially unsaturated non-aromatic ring structure containing the indicated number of carbon atoms and at least one heteroatom, preferably one to four heteroatoms, as ring members. Heterocyclyl includes spirocyclic, bridged, or fused rings formed with one or more other heterocycles or carbocyclic rings, provided that the point of attachment to the parent molecule is an atom of the heterocyclic portion of such ring system. Heterocyclyl typically contains 3 to 12 ring atoms (i.e., 3 to 12-membered heterocyclyl), preferably 4 to 7 ring atoms (i.e., 4 to 7-membered heterocyclyl), and most preferably 5 or 6 ring atoms (i.e., 5 or 6-membered heterocyclyl). Examples of heterocyclyl include, but are not limited to, aziridinyl, oxiranyl, thiiranyl, azetidinyl, oxetanyl, thietanyl, tetrahydrofuranyl, tetrahydropyranyl, tetrahydrothiophenyl, tetrahydrothiopyranyl, pyrrolidinyl, imidazolidinyl, pyrazolidinyl, oxazolidinyl, isoxazolidinyl, thiazolidinyl, isothiazolidinyl, dihydrofuranyl, dihydrothiophenyl, dihydrooxazolyl, dihydrothiazolyl, dihydroisothiazolyl, dihydropyrrolyl, dihydroimidazolyl, dihydropyrazolyl, dihydropyridyl, dihydropyrimidinyl, dihydropyrazinyl, dihydropyridazinyl, piperidinyl, piperazinyl, dioxacyclohexyl, oxathianyl, azepanyl, diazepanyl, morpholinyl, thiomorpholinyl, indolinyl, and isoindolinyl.

As used herein, the term "heteroaryl" refers to a monovalent radical having an aromatic ring structure derived from an indicated number of carbon atoms and at least one heteroatom, preferably one to four heteroatoms, as ring members. Heteroaryl typically contains 5-12 ring atoms ("5-12-membered heteroaryl"), preferably 5-10 ring atoms ("5-10-membered heteroaryl"), and more preferably 5 or 6 ring atoms ("5 or 6-membered heteroaryl"). Heteroaryl may also be fused with another aryl or heteroaryl ring, or with a saturated or partially unsaturated carbon ring or heterocycle, provided that the point of attachment to the parent molecule in such a fused ring system is an atom of the heteroaryl portion of the ring system. Examples of heteroaryl include, but are not limited to, pyrrolyl, furyl, thienyl, pyrazolyl, imidazolyl, oxazolyl, isoxazolyl, thiazolyl, isothiazolyl, oxadiazolyl, thiadiazolyl, triazolyl, tetrazolyl, pyridinyl, pyridazinyl, pyrimidinyl, pyrazinyl, triazinyl, indolyl, isoindolyl, indolizinyl, benzofuranyl, benzothiophenyl, indazolyl, benzimidazolyl, benzoxazolyl, benzoisoxazolyl, benzothiazolyl, benzoisothiazolyl, benzoxadiazolyl, benzothiadiazolyl, benzotriazolyl, imidazopyridyl, imidazopyrimidinyl, imidazopyridazinyl, purinyl, furopyridinyl, thienopyridyl, benzopyranyl, quinolinyl, isoquinolinyl, quindazinyl, quinazolinyl, quinoxalinyl, benzopyridazinyl, cinolinyl, naphthyridinyl, pteridinyl, carbazolyl, carolinyl, phenanthridinyl, acridinyl, phenanthrolinyl, phenazinyl, phenoxazinyl, and phenothiazinyl.

As used herein, the term "optional" or "optionally" means that the subsequently described element may or may not be present. For example, "piperidinyl optionally substituted by methyl" encompasses both "piperidinyl unsubstituted by methyl" and "piperidinyl substituted by methyl".

As used herein, the term "stereoisomer" refers to an isomer arising from a different spatial arrangement of atoms in a molecule. Enantiomers are formed when a compound contains asymmetric carbon atoms; cis-trans isomers are formed when a compound contains carbon-carbon double bonds or a cyclic structure. The scope of the present disclosure includes all enantiomers, diastereomers, racemates, cis-trans isomers, geometric isomers, epimers, and mixtures thereof of compounds of Formula I.

As used herein, the term "solvate" refers to a molecular complex including a compound of Formula I and one or more pharmaceutically acceptable solvent molecules (e.g., ethanol). When the solvent is water, the term "hydrate" is used.

As used herein, the term "isotopic derivative" refers to compound labeled with isotopes, i.e., a compound in which one or more atoms are replaced by atoms having the same atomic number but a different atomic mass or mass number than those normally found in nature. Examples of isotopes suitable for incorporation into compounds include, but are not limited to: hydrogen (such as ²H and ³H), carbon (such as ¹¹C, ¹³C and ¹⁴C), chlorine (such as ³⁶Cl), fluorine (such as ¹⁸F), iodine (such as ¹²³I, ¹²⁴I and ¹²⁵I), nitrogen (such as ¹³N and ¹⁵N), oxygen (such as ¹⁵O, ¹⁷O and ¹⁸O), phosphorus (such as ³²P), and sulfur (such as ³⁵S). The isotopically labeled compounds of the present disclosure can be prepared using conventional techniques known to those skilled in the art or by methods similar to those described in the schemes and examples herein, using appropriate isotopically labeled reagents and/or intermediates, without the need for excessive experimentation.

As used herein, the term "pharmaceutically acceptable" means that a substance or material is suitable for contact with the tissues of a subject, such as a human or other mammal, without causing excessive toxicity, irritation, allergic reactions or other problems, within the bounds of reasonable medical judgment, and has a proportionate benefit/risk ratio.

As used herein, the term "pharmaceutically acceptable salt" refers to a salt formed by the reaction of a pharmaceutically acceptable acid or base with a compound of Formula I of the present disclosure, including, for example, hydrochlorides, acetates, hydrobromides, sulfates, bisulfates, carbonates, bicarbonates, sulfites, phosphates, hydrogen phosphates, oxalates, malonates, valerates, borates, p-toluenesulfonates, methanesulfonates, tartrates, benzoates, lactates, citrates, maleates, fumarates, malates, salicylates, mandelates, succinates, gluconates, lactobionates, alkali metal salts (e.g., lithium, sodium, potassium, rubidium, cesium salts), alkaline earth metal salts (e.g., magnesium, calcium, strontium, barium salts), aluminum salts, etc. These salts can be prepared by methods well known to those skilled in the art.

The terms "prevent," "preventing," and "prevention" as used herein refer to reducing or eliminating the possibility of disease.

The terms "treat," "treating," and "treatment" as used herein refer to the complete or partial elimination of the disease and / or its accompanying symptoms.

As used herein, the term "subject" refers to an animal, preferably a mammal, that is intended to be an experimental or therapeutic individual, including but not limited to primates (e.g., apes and humans), equines (e.g., horses), canines (e.g., dogs), felines, domesticated animals (e.g., pigs, goats, sheep, etc.), as well as domestic pets and animals kept in zoos, preferably humans.

The term "androgen-related disease" as used herein refers to diseases that can be treated or prevented by targeting androgen receptors or by regulating androgen levels.

The first aspect of the present disclosure provides a series of compounds of Formula I or pharmaceutically acceptable salts, stereoisomers, solvates, or isotopic derivatives thereof:

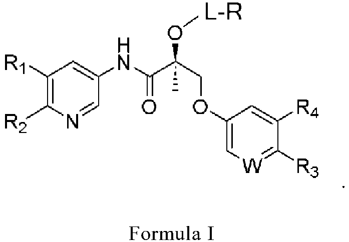

R₁, R₂, R₃, R₄, W, L, and R are as defined above for the compound of Formula I.

In some embodiments, L represents a bond, -C(O)-, or -S(O)₂-.

In some embodiments, L is a bond.

In some embodiments, L is -C(O)-.

In some embodiments, L is -S(O)₂-.

In some embodiments, R is hydrogen.

In some embodiments, R is selected from C₁-C₆ alkyl, C₃-C₈ cycloalkyl, C₆-C₁₀ aryl, 3- to 12-membered heterocyclyl, 3- to 12-membered heterocyclyl-C₁-C₆ alkyl, 5- to 12-membered heteroaryl, and -C₁-C₆ alkyl- NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents each independently selected from C₁-C₆ alkyl, C₁-C₆ alkoxy, halogen, and cyano.

In some embodiments, R is C₁-C₆ alkyl.

In some embodiments, R is methyl, ethyl, n-propyl, or isopropyl.

In some embodiments, R is methyl.

In some embodiments, R is C₃-C₈ cycloalkyl, which is optionally substituted by 1 or 2 substituents each independently selected from C₁-C₆ alkyl, C₁-C₆ alkoxy, halogen, and cyano.

In some embodiments, R is cyclopropyl, cyclobutyl, cyclopentyl, or cyclohexyl that is optionally substituted by 1 or 2 substituents each independently selected from C₁-C₆ alkyl, C₁-C₆ alkoxy, halogen, or cyano.

In some embodiments, R is unsubstituted cyclopentyl.

In some embodiments, R is C₆-C₁₀ aryl, which is optionally substituted by 1 or 2 substituents each independently selected from C₁-C₆ alkyl, C₁-C₆ alkoxy, halogen, and cyano.

In some embodiments, R is phenyl or naphthyl, which is optionally substituted by 1 or 2 substituents each independently selected from C₁-C₆ alkyl, C₁-C₆ alkoxy, halogen, and cyano.

In some embodiments, R is unsubstituted phenyl.

In some embodiments, R is 3- to 12- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, R is 4- to 7- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, R is 5- to 6- membered heterocyclyl, wherein the heterocyclyl contains 1 or 2 heteroatoms each independently selected from N, O, or S and optionally substituted by 1 or 2 substituents each independently selected from C₁-C₆ alkyl, C₁-C₆ alkoxy, halogen, or cyano.

In some embodiments, R is azetidinyl, oxetanyl, thietanyl, tetrahydrofuranyl, tetrahydropyranyl, tetrahydrothiophenyl, tetrahydrothiopyranyl, pyrrolidinyl, imidazolidinyl, pyrazolidinyl, oxazolidinyl, isoxazolidinyl, thiazolidinyl, isothiazolidinyl, dihydrofuranyl, dihydrothiophenyl, dihydrooxazolyl, dihydrothiazolyl, dihydroisothiazolyl, dihydropyrrolyl, dihydroimidazolyl, dihydropyrazolyl, dihydropyridyl, dihydropyrimidinyl, dihydropyrazinyl, dihydropyridazinyl, piperidinyl, piperazinyl, dioxacyclohexyl, oxathianyl, azepanyl, diazepanyl, morpholinyl, thiomorpholinyl, indolinyl, or isoindolinyl, wherein said group is optionally substituted by 1 or 2 substituents each independently selected from C₁-C₆ alkyl, C₁-C₆ alkoxy, halogen, or cyano.

In some embodiments, R is pyrrolidinyl, piperidinyl, or dihydropyridinyl that is optionally substituted by 1 or 2 substituents each independently selected from methyl, ethyl, methoxy, ethoxy, fluorine, chlorine, bromine, iodine, or cyano.

In some embodiments, R is pyrrolidinyl optionally substituted by methyl.

In some embodiments, R is piperidinyl optionally substituted by methyl.

In some embodiments, R is dihydropyridyl optionally substituted by methyl.

In some embodiments, R is 3- to 12- membered heterocyclyl-C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, R is 4- to 7- membered heterocyclyl-C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, R is 5- to 6-membered heterocyclyl-C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, R is

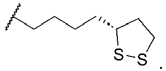

In some embodiments, R is 5- to 10-membered heteroaryl, wherein the heteroaryl contains 1 or 2 heteroatoms each independently selected from N, O, or S and optionally substituted by 1 or 2 substituents each independently selected from C₁-C₆ alkyl, C₁-C₆ alkoxy, halogen, or cyano.

In some embodiments, R is 5- to 6- membered heteroaryl, wherein the heteroaryl contains 1 or 2 heteroatoms each independently selected from N, O, or S and optionally substituted by 1 or 2 substituents each independently selected from C₁-C₆ alkyl, C₁-C₆ alkoxy, halogen, or cyano.

In some embodiments, R is pyrrolyl, furyl, thienyl, pyrazolyl, imidazolyl, oxazolyl, isoxazolyl, thiazolyl, isothiazolyl, pyridinyl, pyridazinyl, pyrimidinyl, pyrazinyl, indolyl, isoindolyl, indolizinyl, benzofuranyl, benzothiophenyl, indazolyl, benzimidazolyl, benzoxazolyl, benzoisoxazolyl, benzothiazolyl, benzoisothiazolyl, imidazopyridinyl, furopyridinyl, thienopyridinyl, benzopyranyl, quinolinyl, isoquinolinyl, quinazolinyl, quinoxalinyl, benzopyridazinyl, cinnolinyl, or naphthyridinyl, wherein said group is optionally substituted by 1 or 2 substituents each independently selected from C₁-C₆ alkyl, C₁-C₆ alkoxy, halogen, or cyano.

In some embodiments, R is unsubstituted pyridinyl.

In some embodiments, R is -C₁-C₆ alkyl- NR₅R₆, wherein R₅ and R₆ are each independently hydrogen or C₁-C₆ alkyl.

In some embodiments, R is -C₁-C₆ alkyl-NH₂.

In some embodiments, R is -C₁-C₆ alkyl-NH(C₁-C₆ alkyl).

In some embodiments, R is -C₁-C₆ alkyl-N (C₁-C₆ alkyl)₂.

In some embodiments, R is dimethylaminoethyl.

In some embodiments, R₁ and R₂ are each independently cyano, halogen, C₁-C₆ haloalkyl, nitro, or -NR₅R₆.

In some embodiments, R₁ and R₂ are each independently cyano, fluorine, chlorine, bromine, iodine, trifluoromethyl, difluoromethyl, fluoromethyl, trichloromethyl, dichloromethyl, chloromethyl, tribromomethyl, dibromomethyl, bromomethyl, nitro, -NH₂, -NH(C₁-C₆ alkyl) or -N(C₁-C₆ alkyl)₂.

In some embodiments, R₁ is cyano, halogen, trifluoromethyl, difluoromethyl, trichloromethyl, dichloromethyl, nitro, or -NH₂.

In some embodiments, R₁ is cyano, halogen, trifluoromethyl, difluoromethyl, nitro, or -NH₂.

In some embodiments, R₁ is trifluoromethyl or halogen.

In some embodiments, R₂ is cyano, halogen, trifluoromethyl, difluoromethyl, trichloromethyl, dichloromethyl, nitro, or -NH₂.

In some embodiments, R₂ is cyano, halogen, trifluoromethyl, difluoromethyl, nitro, or -NH₂.

In some embodiments, R₂ is cyano.

In some embodiments, W stands for CH.

In some embodiments, W is N.

In some embodiments, R₃ and R₄ are each independently hydrogen, cyano, halogen, C₁-C₆ haloalkyl, nitro, -NR₅R₆, -C(O)C₁-C₆ alkyl, -N(R₇)C(O)C₁-C₆ alkyl, -N(R₇)C(O)-C₁-C₆ haloalkyl, - C₁-C₆ alkyl-C(O)C₁-C₆ alkyl, -S(O)₂-C₁-C₆ alkyl, -N(R₇)-S(O)₂-C₁-C₆ alkyl, C₁-C₆ alkyl, or C₁-C₆ alkoxy.

In some embodiments, R₃ and R₄ are each independently hydrogen, cyano, halogen, C₁-C₆ haloalkyl, nitro, -NR₅R₆, -C(O)C₁-C₆ alkyl, -N(R₇)C(O)C₁-C₆ alkyl, -N(R₇)C(O)-C₁-C₆ haloalkyl, - S(O)₂-C₁-C₆ alkyl, or -N(R₇)-S(O)₂-C₁-C₆ alkyl.

In some embodiments, R₃ and R₄ are each independently hydrogen, cyano, fluorine, chlorine, bromine, iodine, trifluoromethyl, difluoromethyl, trichloromethyl, dichloromethyl, nitro, -NH₂, - NH(C₁-C₆ alkyl), -N(C₁ -C₆ alkyl)₂, -C(O)C₁-C₆ alkyl, -NHC(O)C₁-C₆ alkyl, -N(C₁-C₆ alkyl)C(O)C₁-C₆ alkyl, -S(O)₂-C₁-C₆ alkyl, -NH-S(O)₂-C₁-C₆ alkyl, or -N(C₁-C₆ alkyl)-S(O)₂-C₁-C₆ alkyl.

In some embodiments, R₃ is cyano, halogen, C₁-C₆ haloalkyl, nitro, -NH₂, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl.

In some embodiments, R₃ is cyano, fluorine, chlorine, bromine, iodine, trifluoromethyl, nitro, -NH₂, acetyl, acetamido, ethanesulfonyl, or ethanesulfonamide.

In some embodiments, R₃ is cyano, fluorine, chlorine, bromine, or iodine.

In some embodiments, R₃ is cyano.

In some embodiments, R₄ is hydrogen, fluorine, chlorine, bromine, or iodine.

In some embodiments, R₄ is hydrogen.

In some embodiments, L is a bond and R is hydrogen, thus the compounds have Formula II:

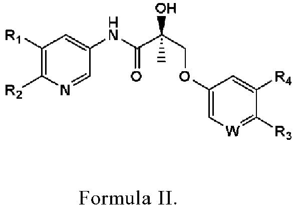

In some embodiments, L is -C(O)-, thus the compounds have Formula III:

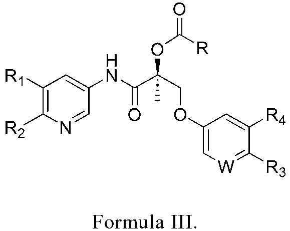

In some embodiments, L is -C(O)-, and R is C₁-C₆ alkyl, C₃-C₈ cycloalkyl, C₆-C₁₀ aryl, 3- to 12-membered heterocyclyl, 3- to 12-membered heterocyclyl -C₁-C₆ alkyl, 5- to 12-membered heteroaryl, and -C₁-C₆ alkyl-NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents each independently selected from C₁-C₆ alkyl, C₁-C₆ alkoxy, halogen, and cyano.

In some embodiments, L is -C(O)-and R is C₁-C₆ alkyl.

In some embodiments, L is -C(O)-, and R is methyl, ethyl, n-propyl, or isopropyl.

In some embodiments, L is -C(O)-and R is methyl.

In some embodiments, L is -C(O)-and R is C₃-C₈ cycloalkyl, which is optionally substituted by 1 or 2 substituents each independently selected from C₁-C₆ alkyl, C₁-C₆ alkoxy, halogen, and cyano.

In some embodiments, L is -C(O)-and R is cyclopropyl, cyclobutyl, cyclopentyl, or cyclohexyl, wherein the group is optionally substituted by 1 or 2 substituents each independently selected from C₁-C₆ alkyl, C₁-C₆ alkoxy, halogen, or cyano.

In some embodiments, L is -C(O)-and R is cyclopentyl.

In some embodiments, L is -C(O)-and R is C₆-C₁₀ aryl, wherein the aryl is optionally substituted by 1 or 2 substituents each independently selected from C₁-C₆ alkyl, C₁-C₆ alkoxy, halogen, or cyano.

In some embodiments, L is -C(O)-and R is phenyl or naphthyl, the group optionally being substituted by 1 or 2 substituents each independently selected from C₁-C₆ alkyl, C₁-C₆ alkoxy, halogen, and cyano.

In some embodiments, L is -C(O)-and R is unsubstituted phenyl.

In some embodiments, L is -C(O)-and R is 3- to 12- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-and R is 4- to 7- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.
1. In some embodiments, L is -C(O)-and R is 5- to 6- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-, and R is azetidinyl, oxetanyl, thietanyl, tetrahydrofuranyl, tetrahydropyranyl, tetrahydrothiophenyl, tetrahydrothiopyranyl, pyrrolidinyl, imidazolidinyl, pyrazolidinyl, oxazolidinyl, isoxazolidinyl, thiazolidinyl, isothiazolidinyl, dihydrofuranyl, dihydrothiophenyl, dihydrooxazolyl, dihydrothiazolyl, dihydroisothiazolyl, dihydropyrrolyl, dihydroimidazolyl, dihydropyrazolyl, dihydropyridyl, dihydropyrimidinyl, dihydropyrazinyl, dihydropyridazinyl, piperidinyl, piperazinyl, dioxacyclohexyl, oxathianyl, azepanyl, diazepanyl, morpholinyl, thiomorpholinyl, indolinyl, or isoindolinyl, wherein the groups are optionally substituted by 1 or 2 substituents each independently selected from C₁-C₆ alkyl, C₁-C₆ alkoxy, halogen, or cyano.

In some embodiments, L is -C(O)-and R is pyrrolidinyl, piperidinyl, or dihydropyridinyl, wherein the group is optionally substituted by 1 or 2 substituents each independently selected from methyl, ethyl, methoxy, ethoxy, fluorine, chlorine, bromine, iodine, or cyano.

In some embodiments, L is -C(O)-, and R is pyrrolidinyl optionally substituted by methyl.

In some embodiments, L is -C(O)-, and R is piperidinyl optionally substituted by methyl.

In some embodiments, L is -C(O)-, and R is dihydropyridyl optionally substituted by methyl.

In some embodiments, L is -C(O)-and R is 3- to 12-membered heterocyclyl -C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-and R is 4- to 7- membered heterocyclyl -C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-and R is 5- to 6- membered heterocyclyl -C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-, and R is

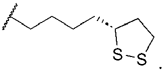

In some embodiments, L is -C(O)-and R is 5- to 10-membered heteroaryl, wherein the heteroaryl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-and R is 5- to 6- membered heteroaryl, wherein the heteroaryl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -C(O)-, and R is pyrrolyl, furyl, thienyl, pyrazolyl, imidazolyl, oxazolyl, isoxazolyl, thiazolyl, isothiazolyl, pyridinyl, pyridazinyl, pyrimidinyl, pyrazinyl, indolyl, isoindolyl, indolizinyl, benzofuranyl, benzothiophenyl, indazolyl, benzimidazolyl, benzoxazolyl, benzoisoxazolyl, benzothiazolyl, benzoisothiazolyl, imidazopyridinyl, furopyridinyl, thienopyridyl, benzopyranyl, quinolinyl, isoquinolinyl, quinazolinyl, quinoxalinyl, benzopyridazinyl, cinnolinyl, or naphthyridinyl, wherein said group is optionally substituted by 1 or 2 substituents each independently selected from C₁-C₆ alkyl, C₁-C₆ alkoxy, halogen, or cyano.

In some embodiments, L is -C(O)-and R is pyridyl, wherein the pyridyl is optionally substituted by 1 or 2 substituents each independently selected from methyl, ethyl, methoxy, ethoxy, fluorine, chlorine, bromine, iodine, and cyano.

In some embodiments, L is -C(O)-and R is unsubstituted pyridinyl.

In some embodiments, L is -C(O)-and R is -C₁-C₆ alkyl-NR₅R₆, wherein R₅ and R₆ are each independently hydrogen or C₁-C₆ alkyl.

In some embodiments, L is -C(O)-and R is -C₁-C₆ alkyl-NH₂.

In some embodiments, L is -C(O)-and R is -C₁-C₆ alkyl-NH(C₁-C₆ alkyl).

In some embodiments, L is -C(O)-and R is -C₁-C₆ alkyl-N (C₁-C₆ alkyl)₂.

In some embodiments, L is -C(O)-and R is dimethylaminoethyl.

In some embodiments, L is -S(O)₂-, thus the compounds have the Formula IV:

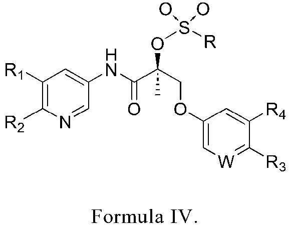

In some embodiments, L is -S(O)₂-, and R is C₁-C₆ alkyl, C₃-C₈ cycloalkyl, C₆-C₁₀ aryl, 3- to 12-membered heterocyclyl, 3- to 12-membered heterocyclyl-C₁-C₆ alkyl, 5- to 12-membered heteroaryl, and -C₁-C₆ alkyl- NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents each independently selected from C₁-C₆ alkyl, C₁-C₆ alkoxy, halogen, and cyano.

In some embodiments, L is -S(O)₂- and R is C₁-C₆ alkyl.

In some embodiments, L is -S(O)₂-, and R is methyl, ethyl, n-propyl, or isopropyl.

In some embodiments, L is -S(O)₂- and R is methyl.

In some embodiments, L is -S(O)₂- and R is C₃-C₈ cycloalkyl, which is optionally substituted by 1 or 2 substituents each independently selected from C₁-C₆ alkyl, C₁-C₆ alkoxy, halogen, and cyano.

In some embodiments, L is -S(O)₂-, and R is cyclopropyl, cyclobutyl, cyclopentyl, or cyclohexyl, wherein the group is optionally substituted by 1 or 2 substituents each independently selected from C₁-C₆ alkyl, C₁-C₆ alkoxy, halogen, or cyano.

In some embodiments, L is -S(O)₂- and R is cyclopentyl.

In some embodiments, L is -S(O)₂- and R is C₆-C₁₀ aryl, wherein the aryl is optionally substituted by 1 or 2 substituents each independently selected from C₁-C₆ alkyl, C₁-C₆ alkoxy, halogen, or cyano.

In some embodiments, L is -S(O)₂- and R is phenyl or naphthyl, the group optionally being substituted by 1 or 2 substituents each independently selected from C₁-C₆ alkyl, C₁-C₆ alkoxy, halogen, and cyano.

In some embodiments, L is -S(O)₂- and R is unsubstituted phenyl.

In some embodiments, L is -S(O)₂- and R is 3- to 12- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂- and R is 4- to 7- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂- and R is 5- to 6- membered heterocyclyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂-, and R is azetidinyl, oxetanyl, thietanyl, tetrahydrofuranyl, tetrahydropyranyl, tetrahydrothiophenyl, tetrahydrothiopyranyl, pyrrolidinyl, imidazolidinyl, pyrazolidinyl, oxazolidinyl, isoxazolidinyl, thiazolidinyl, isothiazolidinyl, dihydrofuranyl, dihydrothiophenyl, dihydrooxazolyl, dihydrothiazolyl, dihydroisothiazolyl, dihydropyrrolyl, dihydroimidazolyl, dihydropyrazolyl, dihydropyridinyl, dihydropyrimidinyl, dihydropyrazinyl, dihydropyridazinyl, piperidinyl, piperazinyl, dioxacyclohexyl, oxathianyl, azepanyl, diazepanyl, morpholinyl, thiomorpholinyl, indolinyl, or isoindolinyl, wherein the groups are optionally substituted by 1 or 2 substituents each independently selected from C₁-C₆ alkyl, C₁-C₆ alkoxy, halogen, or cyano.

In some embodiments, L is -S(O)₂- and R is pyrrolidinyl, piperidinyl, or dihydropyridinyl, wherein the group is optionally substituted by 1 or 2 substituents each independently selected from methyl, ethyl, methoxy, ethoxy, fluorine, chlorine, bromine, iodine, or cyano.

In some embodiments, L is -S(O)₂-, and R is pyrrolidinyl optionally substituted by methyl.

In some embodiments, L is -S(O)₂-, and R is piperidinyl optionally substituted by methyl.

In some embodiments, L is -S(O)₂-, and R is dihydropyridyl optionally substituted by methyl.

In some embodiments, L is -S(O)₂- and R is 3- to 12-membered heterocyclyl -C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂- and R is 4- to 7-membered heterocyclyl -C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂- and R is 5- to 6-membered heterocyclyl -C₁-C₆ alkyl, wherein the heterocyclyl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂-, and R is

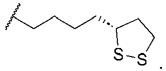

In some embodiments, L is -S(O)₂- and R is 5- to 10-membered heteroaryl, wherein the heteroaryl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂- and R is 5- to 6- membered heteroaryl, wherein the heteroaryl contains 1 or 2 heteroatoms each independently selected from N, O or S and optionally substituted by 1 or 2 substituents each independently selected from C₁-C₆ alkyl, C₁-C₆ alkoxy, halogen or cyano.

In some embodiments, L is -S(O)₂-, and R is pyrrolyl, furyl, thienyl, pyrazolyl, imidazolyl, oxazolyl, isoxazolyl, thiazolyl, isothiazolyl, pyridinyl, pyridazinyl, pyrimidinyl, pyrazinyl, indolyl, isoindolyl, indolizinyl, benzofuranyl, benzothiophenyl, indazolyl, benzimidazolyl, benzoxazolyl, benzoisoxazolyl, benzothiazolyl, benzoisothiazolyl, imidazopyridinyl, furopyridinyl, thienopyridinyl, benzopyranyl, quinolinyl, isoquinolinyl, quinazolinyl, quinoxalinyl, benzopyridazinyl, cinnolinyl, or naphthyridinyl, wherein said group is optionally substituted by 1 or 2 substituents each independently selected from C₁-C₆ alkyl, C₁-C₆ alkoxy, halogen, or cyano.

In some embodiments, L is -S(O)₂- and R is pyridyl, wherein the pyridyl is optionally substituted by 1 or 2 substituents each independently selected from methyl, ethyl, methoxy, ethoxy, fluorine, chlorine, bromine, iodine, and cyano.

In some embodiments, L is -S(O)₂- and R is unsubstituted pyridinyl.

In some embodiments, L is -S(O)₂- and R is -C₁-C₆ alkyl-NR₅R₆, wherein R₅ and R₆ are each independently hydrogen or C₁-C₆ alkyl.

In some embodiments, L is -S(O)₂- and R is -C₁-C₆ alkyl-NH₂.

In some embodiments, L is -S(O)₂- and R is -C₁-C₆ alkyl-NH(C₁-C₆ alkyl).

In some embodiments, L is -S(O)₂- and R is -C₁-C₆ alkyl-N (C₁-C₆ alkyl)₂.

In some embodiments, L is -S(O)₂- and R is dimethylaminoethyl.

In some embodiments, R₁ and R₂ are each independently cyano, halogen, trifluoromethyl, difluoromethyl, nitro, or -NH₂.

In some embodiments, R₁ is cyano and R₂ is trifluoromethyl.

In some embodiments, R₁ is trifluoromethyl and R₂ is cyano.

In some embodiments, R₁ is nitro and R₂ is trifluoromethyl.

In some embodiments, R₁ is trifluoromethyl and R₂ is nitro.

In some embodiments, R₁ is halogen and R₂ is cyano.

In some embodiments, R₁ is cyano and R₂ is halogen.

In some embodiments, R₃ is cyano, halogen, C₁-C₆ alkyl, nitro, -NR₅R₆, -C(O)C₁-C₆ alkyl, - N(R₇)C(O)C₁-C₆ alkyl, -N(R₇)C(O)-C₁-C₆ haloalkyl, -C₁-C₆ alkyl-C(O)C₁-C₆ alkyl, -S(O)₂-C₁-C₆ alkyl, -N(R₇)-S(O)₂-C₁-C₆ alkyl, C₁-C₆ alkyl, or C₁-C₆ alkoxy, and R₄ is hydrogen or halogen.

In some embodiments, R₃ is cyano, fluorine, chlorine, bromine, iodine, trifluoromethyl, difluoromethyl, trichloromethyl, dichloromethyl, nitro, -NH₂, -NH(C₁ -C₆ alkyl), -N(C₁-C₆ alkyl)₂, - C(O)C₁-C₆ alkyl, -NHC(O)C₁-C₆ alkyl, -N(C₁-C₆ alkyl)-C(O)C₁-C₆ alkyl, -S(O)₂-C₁- C₆ alkyl, -NH-S(O)₂-C₁-C₆ alkyl or -N(C₁-C₆ alkyl)-S(O)₂-C₁-C₆ alkyl, and R₄ is hydrogen, fluorine, chlorine, bromine or iodine.

In some embodiments, R₃ is cyano, fluorine, chlorine, bromine, iodine, trifluoromethyl, nitro, -NH₂, acetyl, acetamido, ethanesulfonyl, or ethanesulfonamido, and R₄ is hydrogen, fluorine, chlorine, bromine, or iodine.

In some embodiments, R₃ is cyano, fluorine, chlorine, bromine, or iodine, and R₄ is hydrogen, fluorine, chlorine, bromine, or iodine.

In some embodiments, R₃ is cyano and R₄ is hydrogen.

In some embodiments, R₃ is cyano and R₄ is fluorine.

In some embodiments, R₃ is chlorine and R₄ is hydrogen.

In some embodiments, a compound of Formula I is provided, wherein:
R₁ and R₂ are each independently cyano, halogen, trifluoromethyl, difluoromethyl, nitro or - NH₂;
R₃ is cyano, halogen, C₁-C₆ haloalkyl, nitro, -C(O)C₁-C₆ alkyl, -NHC(O)C₁-C₆ alkyl, - NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L represents a bond, -C(O)-or -S(O)₂-;
R is hydrogen, C₁-C₆ alkyl, C₃-C₈ cycloalkyl, C₆-C₁₀ aryl, 4- to 7-membered heterocyclyl, 4-to 7-membered heterocyclyl-C₁-C₆ alkyl, 5- to 10- membered heteroaryl, or -C₁-C₆ alkyl- NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₆ alkyl.

In some embodiments, a compound of Formula I is provided, wherein:
R₁ and R₂ are each independently cyano, halogen, trifluoromethyl, difluoromethyl, nitro or - NH₂;
R₃ is cyano, halogen, C₁-C₆ haloalkyl, nitro, -C(O)C₁-C₆ alkyl, -NHC(O)C₁-C₆ alkyl, - NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L is a bond; and
R stands for hydrogen.

In some embodiments, a compound of Formula I is provided, wherein:
R₁ and R₂ are each independently cyano, halogen, trifluoromethyl, difluoromethyl, nitro or - NH₂;
R₃ is cyano, halogen, C₁-C₆ haloalkyl, nitro, -C(O)C₁-C₆ alkyl, -NHC(O)C₁-C₆ alkyl, - NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L is -C(O)-;
R is C₁-C₆ alkyl, C₃-C₈ cycloalkyl, C₆-C₁₀ aryl, 4- to 7-membered heterocyclyl, 4- to 7-membered heterocyclyl-C₁-C₆ alkyl, 5- to 10-membered heteroaryl, or -C₁-C₆ alkyl-NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₆ alkyl.

In some embodiments, a compound of Formula I is provided, wherein:
R₁ and R₂ are each independently cyano, halogen, trifluoromethyl, difluoromethyl, nitro or - NH₂;
R₃ is cyano, halogen, C₁-C₆ haloalkyl, nitro, -C(O)C₁-C₆ alkyl, -NHC(O)C₁-C₆ alkyl, - NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L is -S(O)₂-;
R is C₁-C₆ alkyl, C₃-C₈ cycloalkyl, C₆-C₁₀ aryl, 4- to 7-membered heterocyclyl, 4- to 7-membered heterocyclyl-C₁-C₆ alkyl, 5- to 10-membered heteroaryl, or -C₁-C₆ alkyl-NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₆ alkyl.

In some embodiments, a compound of Formula I is provided, wherein:
R₁ and R₂ are each independently cyano, halogen, trifluoromethyl or nitro;
R₃ is cyano, halogen, nitro, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L represents a bond, -C(O)-or -S(O)₂-;
R is hydrogen, C₁-C₆ alkyl, C₃-C₈ cycloalkyl, phenyl, 5- to 6- membered heterocyclyl, 5- to 6 -membered heterocyclyl-C₁-C₄ alkyl, 5- to 6 -membered heteroaryl, or -C₁-C₆ alkyl-NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₄ alkyl.

In some embodiments, a compound of Formula I is provided, wherein:
R₁ and R₂ are each independently cyano, halogen, trifluoromethyl or nitro;
R₃ is cyano, halogen, nitro, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L represents a bond;
R stands for hydrogen.

In some embodiments, a compound of Formula I is provided, wherein:
R₁ and R₂ are each independently cyano, halogen, trifluoromethyl or nitro;
R₃ is cyano, halogen, nitro, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L is -C(O)-;
R is C₁-C₆ alkyl, C₃-C₈ cycloalkyl, phenyl, 5- to 6- membered heterocyclyl, 5- to 6 - membered heterocyclyl-C₁-C₄ alkyl, 5- to 6 -membered heteroaryl, or -C₁-C₆ alkyl-NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₄ alkyl.

In some embodiments, a compound of Formula I is provided, wherein:
R₁ and R₂ are each independently cyano, halogen, trifluoromethyl or nitro;
R₃ is cyano, halogen, nitro, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L is -S(O)₂-;
R is C₁-C₆ alkyl, C₃-C₈ cycloalkyl, phenyl, 5- to 6- membered heterocyclyl, 5- to 6 - membered heterocyclyl-C₁-C₄ alkyl, 5- to 6 -membered heteroaryl, or -C₁-C₆ alkyl-NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₄ alkyl.

In some embodiments, a compound of Formula I is provided, wherein:
R₁ is trifluoromethyl;
R₂ is cyano;
R₃ is cyano or halogen;
R₄ is hydrogen or halogen;
W is CH or N;
L represents a bond, -C(O)-or -S(O)₂-;
R is hydrogen, methyl, ethyl, n-propyl, isopropyl, cyclopentyl, phenyl, piperidinyl (optionally substituted by methyl), pyrrolidinyl (optionally substituted by methyl), dihydropyridinyl (optionally substituted by methyl), pyridinyl, dimethylaminoethyl, or

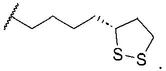

In some embodiments, compounds selected from the following or pharmaceutically acceptable salts, stereoisomers, solvates, or isotopic derivatives thereof are provided:
2. 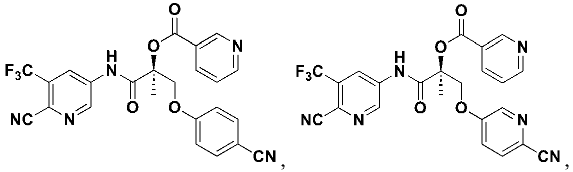 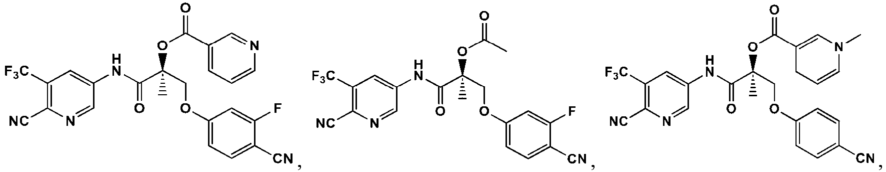 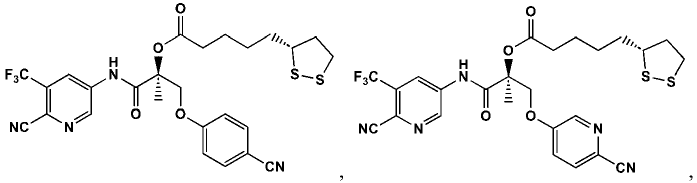 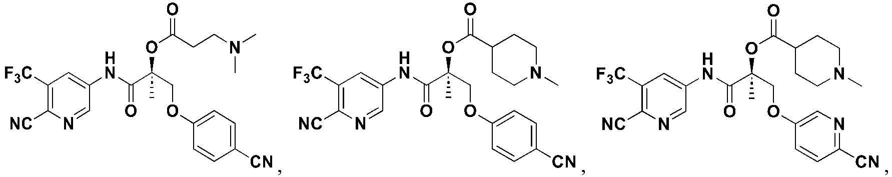 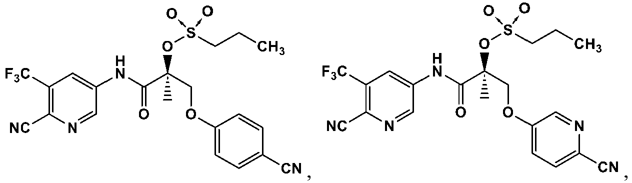 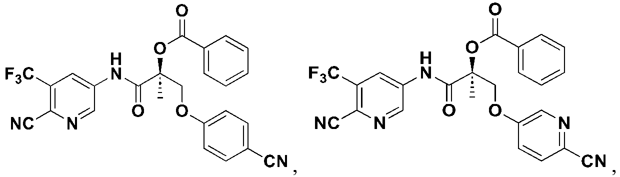 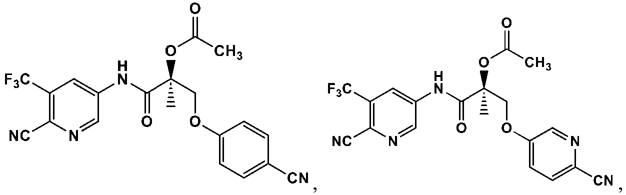 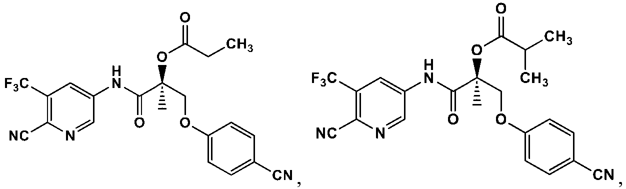 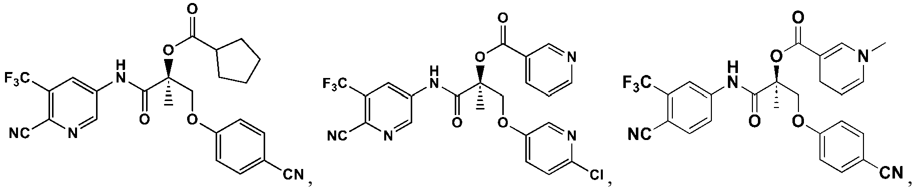
3. 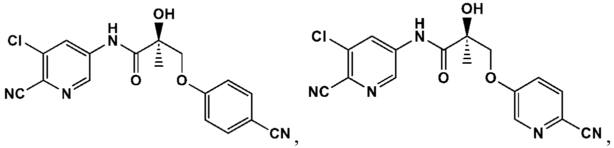
4.

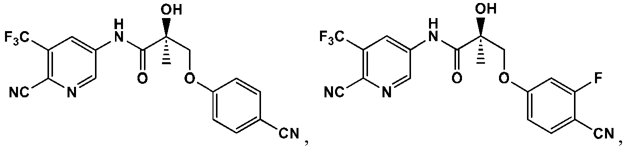

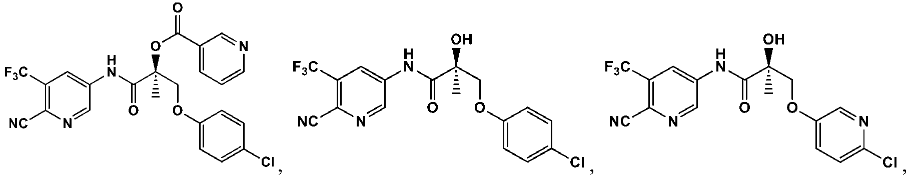

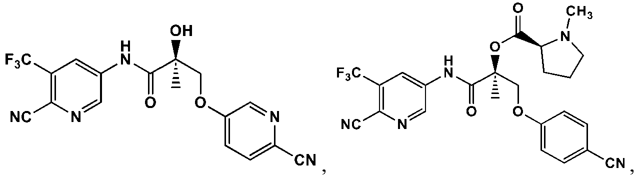

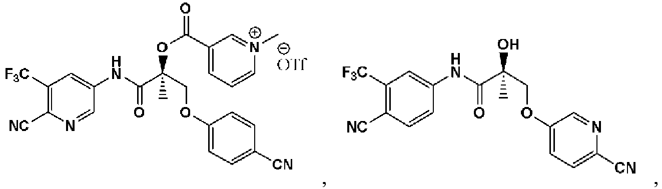

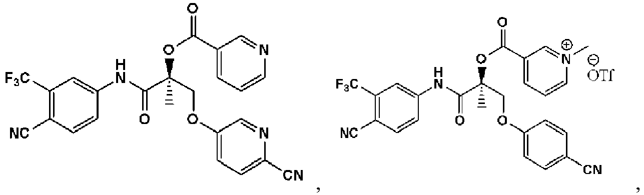

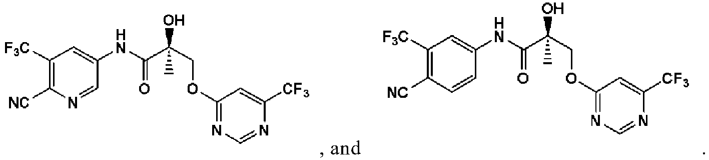

The second aspect of the disclosure provides a pharmaceutical composition including a compound of Formula I or a pharmaceutically acceptable salt, stereoisomer, solvate or isotopic derivative thereof, and one or more pharmaceutically acceptable carriers.

In some embodiments, the pharmaceutical composition includes 0.1 wt% to 99.5 wt% of a compound of Formula I or a pharmaceutically acceptable salt, stereoisomer, solvate, or isotopic derivative thereof as an active ingredient, preferably 0.5 wt% to 99.5 wt%, more preferably 1 wt% to 50 wt%, for example 1 wt%, 1.5 wt%, 2 wt%, 5 wt%, 10 wt%, 15 wt%, 20 wt%, 25 wt%, 30 wt%, or 50 wt% of the active ingredient. The balance of the pharmaceutical composition is a pharmaceutically acceptable carrier.

In some embodiments, the pharmaceutical composition includes two, three, or more pharmaceutically acceptable carriers. These pharmaceutically acceptable carriers include those conventionally used in the pharmaceutical field, such as diluents, fillers, binders, disintegrants, lubricants, wetting agents, solubilizers, solvents, colorants, fragrances, absorption enhancers, surfactants, and adsorbents. Examples include, but are not limited to, starch, pregelatinized starch, sodium carboxymethyl starch, powdered sugar, lactose, calcium phosphate, magnesium stearate, talc, micronized silica gel, dextrin, cellulose and its derivatives (e.g., hydroxyethyl cellulose, hydroxypropyl cellulose, hydroxypropyl methyl cellulose (HPMC), sodium carboxymethyl cellulose, etc.), microcrystalline cellulose, mannitol, sorbitol, polysorbate 80, polyethylene glycol, water, water for injection, physiological saline, glucose solution, etc. The pharmaceutical composition may also contain various other commonly used additives, such as preservatives, emulsifiers, suspending agents, and flavoring agents.

The pharmaceutical composition can be prepared into any pharmaceutically acceptable dosage form using any conventional technique in the art, including but not limited to tablets, capsules, pills, granules, syrups, injections, solutions, suspensions, powders (including sterile powders for injection), etc. The pharmaceutical composition of the present disclosure can be administered to a subject (e.g., human or non-human mammal) via any route of administration, including, for example, oral, intravenous, intraperitoneal, intramuscular, local, transdermal, ocular, nasal, inhalation, subcutaneous, buccal, sublingual, rectal, etc. The effective amount of the compound of Formula I of the present disclosure or a pharmaceutically acceptable salt thereof depends on a variety of factors, including but not limited to: the specific compound to be administered; the species, size, age, and general health condition of the mammal; the severity of the disease; the individual patient's response; the route of administration; the bioavailability characteristics of the administered formulation; the selected dosage regimen; the use of other concomitant medications, etc., which can generally be determined by the attending physician according to routine practice. Generally, the effective amount is typically in the range of about 0.001 to about 100 mg/kg body weight per day, preferably about 0.01 to about 50 mg/kg body weight per day. In some cases, dose levels below the lower limit of the above range may be more than sufficient, while in other cases, a larger dose may be required without causing any harmful side effects. Such a larger dose is usually divided into several smaller doses to be administered throughout the day.

A third aspect of the present disclosure provides a compound of Formula I or a pharmaceutically acceptable salt, stereoisomer, solvate or isotopic derivative thereof, or a pharmaceutical composition including a compound of Formula I or a pharmaceutically acceptable salt, stereoisomer, solvate or isotopic derivative thereof and one or more pharmaceutically acceptable carriers, for the prevention and/or treatment of androgen-related diseases, particularly central nervous system diseases.

The fourth aspect of the disclosure provides the use of a compound of Formula I or a pharmaceutically acceptable salt, stereoisomer, solvate or isotopic derivative thereof, or a pharmaceutical composition including a compound of Formula I or a pharmaceutically acceptable salt, stereoisomer, solvate or isotopic derivative thereof and one or more pharmaceutically acceptable carriers for the prevention and/or treatment of androgen-related diseases, particularly central nervous system diseases.

The fifth aspect of the disclosure provides the use of a compound of Formula I or a pharmaceutically acceptable salt, stereoisomer, solvate or isotopic derivative thereof, or a pharmaceutical composition including a compound of Formula I or a pharmaceutically acceptable salt, stereoisomer, solvate or isotopic derivative thereof and one or more pharmaceutically acceptable carriers in the preparation of a medicament for the prevention and/or treatment of androgen-related diseases, particularly central nervous system diseases.

The sixth aspect of the disclosure provides a method for preventing and/or treating androgen-related diseases, particularly central nervous system diseases, in subjects in need of such treatment, the method including administering to the subject an effective amount of a compound of Formula I or a pharmaceutically acceptable salt, stereoisomer, solvate, or isotopic derivative thereof, or a pharmaceutical composition including a compound of Formula I or a pharmaceutically acceptable salt, stereoisomer, solvate, or isotopic derivative thereof and one or more pharmaceutically acceptable carriers.

In the above third to sixth aspects, the central nervous system diseases mentioned are dementia (including Alzheimer's disease), cognitive deficits of schizophrenia, Parkinson's disease, Huntington's disease, depression, anxiety, stroke, cerebral ischemia, amyotrophic lateral sclerosis, traumatic brain injury, Fragile X syndrome, Rett syndrome, brain tumor, or obesity.

In some embodiments, the central nervous system disease is Alzheimer's disease.

The compounds or pharmaceutical compositions of the present disclosure can be administered to a subject (e.g., a human or a non-human mammal) via any route of administration, including, for example, oral, intravenous, intraperitoneal, intramuscular, local, transdermal, ocular, nasal, inhalation, subcutaneous, buccal, sublingual, rectal, etc.

The attending physician may adjust the dosage and frequency of administration of the compounds or pharmaceutical compositions of the present disclosure, taking into account factors such as the patient's age, general health condition, body weight, and severity of the symptoms to be treated. Generally, the total daily dose of the compounds or pharmaceutical compositions of the present disclosure is typically from about 0.1 to about 1000 mg of active ingredient per day, for example, from about 1 to about 800 mg per day, from about 10 to about 600 mg per day, or from about 50 to about 500 mg per day, administered as a single dose or in 2, 3, or 4 divided doses.

The compounds of the present disclosure provide the following key beneficial effects:
(1) The compounds of the present disclosure exhibit good binding ability to androgen receptors, and some of the compounds show nanomolar protein affinity (KD).
(2) The compounds of the present disclosure possess good androgen receptor agonist potency, and some of the compounds show nanomolar agonist activity while achieving nanomolar agonist activity comparable to that of dihydrotestosterone.
(3) The compounds of the present disclosure exhibit favorable blood-brain barrier permeability, can penetrate through the blood-brain barrier well, and sustain effective brain concentrations for a long period of time.

The novel N-arylpropionamide compounds of the present disclosure are a new class of nonsteroidal androgen receptor modulator that can penetrate the blood-brain barrier. This series of compounds not only exhibit good binding ability for and modulatory activity on the androgen receptor, but also demonstrates good ability to penetrate the blood-brain barrier, thereby possessing the potential to serve as therapeutic agents for central nervous system diseases targeting androgen receptors.

### General method for preparing the compounds of the present disclosure

The compounds of Formula I may be prepared from commercially available or readily prepared starting materials according to synthetic and purification methods known to those skilled in the art of organic synthesis. Exemplary methods for preparing compounds of Formula I are described in the following schemes and examples. It should be understood that these exemplary methods do not constitute any limitation on the disclosure, and those skilled in the art of organic synthesis will understand alternative synthetic routes.

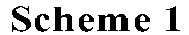

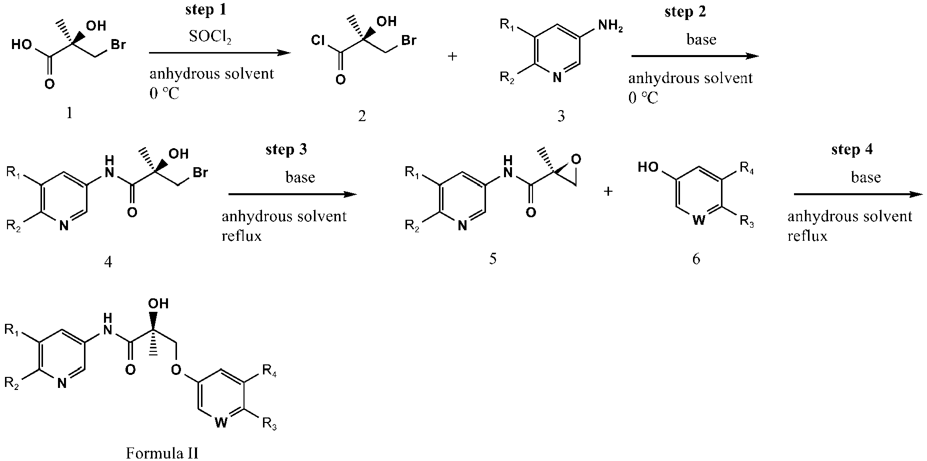

Scheme 1 illustrates a general method for preparing compounds of Formula I in which L is a bond and R is hydrogen, wherein the variables R₁, R₂, R₃, R₄ and W are as defined above for compound of Formula I (i.e., the aforementioned compound of Formula II).

Step 1: Compound 1 (commercially available) was treated with SOCl₂ at 0 °C in an anhydrous solvent to obtain Compound 2.

Step 2: Compound 2 and Compound 3 (commercially available) were reacted at 0 °C in an anhydrous solvent in the presence of a base to afford Compound 4.

Step 3: Compound 4 was heated in an anhydrous solvent in the presence of a base to afford Compound 5.

Step 4: Compound 5 and Compound 6 (commercially available) were reacted in an anhydrous solvent in the presence of a base to afford the compound of Formula II.

In the above reaction steps, the base may be selected from sodium tert-butoxide, sodium methoxide, potassium tert-butoxide, trimethylammonium hydroxide, guanidines, 1,8-diazabicyclo[5.4.0]undec-7-ene (DBU), triethylamine, pyridine, 4-dimethylaminopyridine, N,N-diisopropylethylamine, etc., and the anhydrous solvent is selected from pyridine, tetrahydrofuran, acetonitrile, toluene, acetone, 2-butanone, ethyl acetate, dioxane, N,N-dimethylformamide, and N,N-dimethylacetamide.

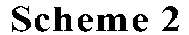

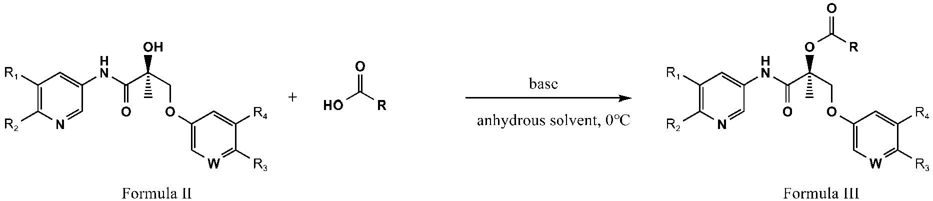

Scheme 2 illustrates a general method for preparing compounds of Formula I in which L is -C(O)-, wherein the variables R₁, R₂, R₃, R₄, W and R are as defined above for compound of Formula I (i.e., the aforementioned compound of Formula III).

The compound of Formula II was prepared as described in Scheme 1, and then reacted with a carboxylic acid compound (which may also be the corresponding acid anhydride or acyl chloride) in an anhydrous solvent in the presence of a base at 0 °C to obtain a compound of Formula III. The base may be selected from sodium tert-butoxide, sodium methoxide, potassium tert-butoxide, trimethylammonium hydroxide, guanidines, 1,8-diazabicyclo[5.4.0]undec-7-ene (DBU), triethylamine, pyridine, 4-dimethylaminopyridine, N,N-diisopropylethylamine, etc., and the anhydrous solvent is selected from pyridine, tetrahydrofuran, acetonitrile, toluene, acetone, 2 - butanone, ethyl acetate, dioxane, N,N-dimethylformamide, and N,N-dimethylacetamide.

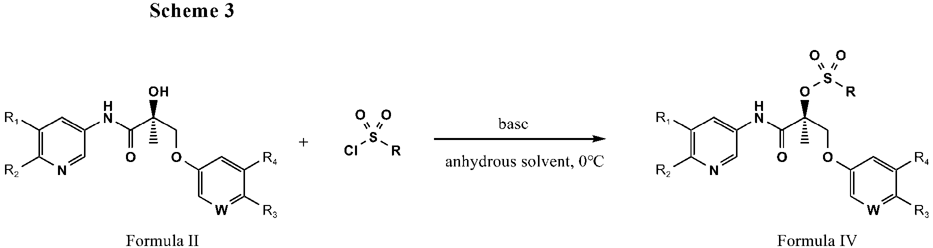

Scheme 3 illustrates a general method for preparing compounds of Formula I in which L is - S(O)₂-, wherein the variables R₁, R₂, R₃, R₄, W, and R are as defined above for the compound of Formula I (i.e., the aforementioned compound of Formula IV).

The compound of Formula II is prepared as described in Scheme 1, and then reacted with an acyl chloride compound in the presence of a base in an anhydrous solvent at 0 °C to obtain a compound of Formula IV. The base is selected from sodium tert-butoxide, sodium methoxide, potassium tert-butoxide, trimethylammonium hydroxide, guanidines, 1,8-diazabicyclo[5.4.0]undec-7-ene (DBU), triethylamine, pyridine, 4-dimethylaminopyridine, N,N-diisopropylethylamine, etc., and the anhydrous solvent is selected from pyridine, tetrahydrofuran, acetonitrile, toluene, acetone, 2-butanone, ethyl acetate, dioxane, N,N-dimethylformamide, and N,N-dimethylacetamide.

### Embodiments

The present disclosure will be further described in detail below with reference to embodiments. Obviously, the described embodiments are only a part of the present disclosure, and not all of it. These embodiments are only used to illustrate the present disclosure and should not be construed as limiting the scope of protection of the present disclosure. Based on the embodiments of the present disclosure, all other technical solutions obtained by those skilled in the art without creative effort are within the scope of protection of the present disclosure.

In the examples, the abbreviations THF represent tetrahydrofuran, DMSO-d₆ represent deuterated dimethyl sulfoxide, DMSO represent dimethyl sulfoxide, CDCl₃ represents deuterated chloroform, eq represents equivalent, HPLC represents high-performance liquid chromatography, PPh₃ represents triphenylphosphine, PCy₃ represents tricyclohexylphosphine, K₃PO₄ represents tripotassium phosphate, DMF represents dimethylformamide, Pd(PPh₃)₄ represents tetra(triphenylphosphine)palladium, and DME represents dimethoxyethane.

¹H NMR measurements were performed using a Bruker AVANCE II 400MHz NMR spectrometer, where s represents a singlet, bs or brs represents a broad singlet, d represents a doublet, t represents a triplet, q represents a quartet, dd represents doublet of doublets, dt represents a double triplet, dq represents a double quartet, m represents a multiplet, and Ar represents aryl. Mass spectrometry was performed using a Bruker amaZon SL mass spectrometer. High-resolution mass spectrometry was performed using a Thermo TSQ. High-performance liquid chromatography (HPLC) was performed using an Agilent/1260 Infinity II. Thin-layer chromatography (TLC) was performed using Silica gel 60F254 plates (Merck).

### Preparation Examples of the Compounds

Example 1 Preparation of (S)-N-(6-cyano-5-(trifluoromethyl)pyridin-3-yl)-2-hydroxy-2-methyl-3-((6-(trifluoromethyl)pyrimidin-4-yl)oxy)propionamide

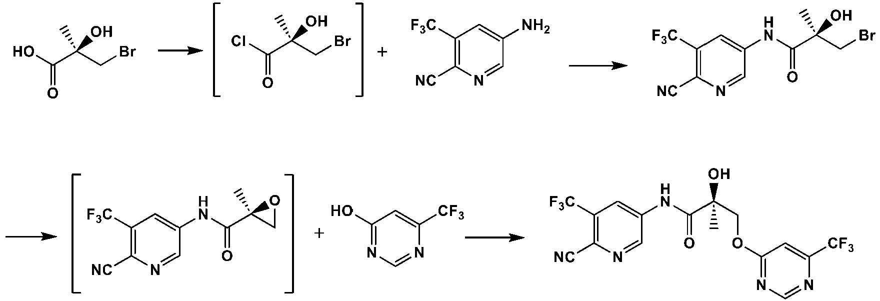

### First and Second Reaction Steps

Under nitrogen atmosphere, (2R)-3-bromo-2-hydroxy-2-methylpropionic acid (2.7 mmol) was added to 10 mL of anhydrous THF. Then, under ice bath conditions, thionyl chloride (3.28 mmol) was slowly added while maintaining the temperature inside the reaction vessel below 0 °C. The reaction solution was gradually heated to room temperature and reacted for 1.5 h. The reaction solution was then placed back under ice bath conditions, and triethylamine (3.55 mmol) was added dropwise while maintaining the temperature inside the reaction vessel below 0 °C. Subsequently, a THF solution of 5-amino-3-(trifluoromethyl)cyanopyridine (2.7 mmol) was added dropwise. The temperature inside the reaction vessel was maintained below 0 °C. After the addition was complete, the mixture was slowly raised to room temperature, then heated to 50 °C and reacted for 2 h. TLC indicated completion of the reaction. The reaction mixture was cooled to room temperature, then quenched by the addition of water and extracted with 40 mL of ethyl acetate. The organic phase was washed with water and saturated brine, then dried over anhydrous sodium sulfate, filtered, and concentrated under reduced pressure to give compound (R)-3-bromo-N-(6-cyano-5-(trifluoromethyl)pyridin-3-yl)-2-hydroxy-2-methylpropionamide.

¹H NMR (400 MHz, DMSO-d₆) δ 10.83 (s, 1H, NH), 9.41 (d, J = 2.2 Hz, 1H, CH), 8.91 (d, J = 2.3 Hz, 1H, CH), 6.53 (s, 1H, CH), 3.93 - 3.80 (m, 2H, 1/2*CH₂), 3.71 - 3.52 (m, 2H, 1/2*CH₂), 1.50 (s, 3H, CH₃). δ LRMS (-ESI) m/z: 350.0/352.0, [(M-H)⁻, 99/100%].

### Third and Fourth Reaction Steps

Under nitrogen atmosphere, (R)-3-bromo-N-(6-cyano-5-(trifluoromethyl)pyridin-3-yl)-2-hydroxy-2-methylpropionamide (0.7 mmol) and 4-hydroxy-6-(trifluoromethyl)pyrimidine (0.8 mmol) were added to anhydrous butanone (10 mL) and stirred until dissolved. Anhydrous K₂CO₃ (2.1 mmol) was added to the solution, and the reaction mixture was heated to 80-85 °C and stirred for 3 h. TLC indicated completion of the reaction. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound (S)-N-(6-cyano-5-(trifluoromethyl)pyridin-3-yl)-2-hydroxy-2-methyl-3-((6-(trifluoromethyl)pyrimidin-4-yl)oxy)propionamide as a white powdery solid, with a yield of 65%.

¹H NMR (400 MHz, DMSO -d₆) δ 10.74 (s, 1H, NH), 9.27 (d, J = 2.3 Hz, 1H, CH), 8.76 (d, J = 2.3 Hz, 1H, CH), 8.49 (s, 1H, CH), 6.90 (s, 1H, CH), 6.61 (br, 1H, OH), 4.23 (s, 2H, CH₂), 1.39 (s, 3H, CH₃). LRMS (-ESI) 433.99 m/z: [(M-H)⁻, 100%].

### Example 2 Preparation of (S)-N-(4-cyano-3-(trifluoromethyl)phenyl)-2-hydroxy-2-methyl-3-((6-(trifluoromethyl)pyrimidin-4-yl)oxy)propionamide

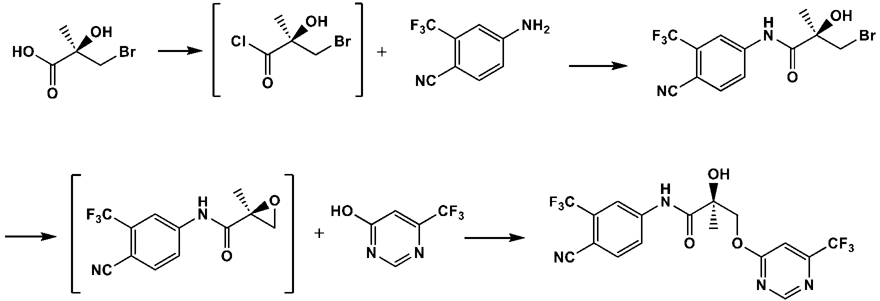

### First and Second Reaction Steps

Under nitrogen atmosphere, (2R)-3-bromo-2-hydroxy-2-methylpropionic acid (2.7 mmol) was added to 10 mL of anhydrous THF. Then, under ice bath conditions, thionyl chloride (3.28 mmol) was slowly added while maintaining the temperature inside the reaction vessel below 0 °C. The reaction solution was gradually heated to room temperature and reacted for 1.5 h. The reaction solution was then placed back under ice bath conditions, and triethylamine (3.55 mmol) was added dropwise while maintaining the temperature inside the reaction vessel below 0 °C. Subsequently, a THF solution (2 mL) of 4-cyano-3-trifluoromethyl-aniline (2.7 mmol) was added dropwise, while continuing to maintain the temperature inside the reaction vessel below 0 °C. After the addition was complete, the mixture was slowly heated to room temperature, then heated to 50 °C and reacted for 2 h. TLC indicated completion of the reaction. The reaction mixture was cooled to room temperature, then quenched by the addition of water and extracted with 40 mL of ethyl acetate. The organic phase was washed with water and saturated brine, then dried over anhydrous sodium sulfate, filtered, and concentrated under reduced pressure to obtain the compound (R)-3-bromo-N-(4-cyano- 3-(trifluoromethyl)phenyl)-2-hydroxy-2-methylpropionamide.

¹H NMR (500 MHz, DMSO-d₆) δ 10.54 (s, 1H, NH), 8.55 (d, J = 2.2 Hz, 1H, CH), 8.31 (dd, J = 8.6, 2.1 Hz, 1H, CH), 8.12 (d, J = 8.6 Hz, 1H, CH), 6.42 (s, 1H, CH), 3.83 (d, J = 10.4 Hz, 1H, 1/2*CH₂), 3.59 (d, J = 10.4 Hz, 1H, 1/2*CH₂), 1.49 (s, 3H, CH₃). LRMS (+ESI) m/z: 349.0/351.0, [(M-H)⁻, 99/100%].

### Third and Fourth Reaction Steps

Under nitrogen atmosphere, (R)-3-bromo-N-(4-cyano-3-(trifluoromethyl)phenyl)-2-hydroxy-2-methylpropionamide (0.7 mmol) and 4-hydroxy-6-(trifluoromethyl)pyrimidine (0.8 mmol) were added to anhydrous butanone (10 mL) and stirred until dissolved. Anhydrous K₂CO₃ (2.1 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound (S)-N-(4-cyano-3-(trifluoromethyl)phenyl)-2-hydroxy-2-methyl-3-((6-(trifluoromethyl)pyrimidin-4-yl)oxy)propionamide as a white powdery solid with a yield of 60%.

¹H NMR (400 MHz, DMSO-d₆) δ 10.49 (s, 1H, NH), 8.48 (s, 1H, CH), 8.41 (d, J = 2.1 Hz, 1H, CH), 8.20 (dd, J = 8.6, 2.1 Hz, 1H, CH), 8.08 (d, J = 8.6 Hz, 1H, CH), 6.90 (s, 1H, CH), 6.50 (s, 1H, OH), 4.28 (d, J = 13.6 Hz, 1H, 1/2*CH₂), 4.19 (d, J = 13.6 Hz, 1H, 1/2*CH₂), 1.37 (s, 3H, CH₃). LRMS (-ESI) 433.12 m/z: [(M-H)⁻, 100%].

### Example 3 Preparation of (S)-N-(6-cyano-5-(trifluoromethyl)pyridin-3-yl)-3-(4-cyanophenoxy)-2-hydroxy-2-methylpropionamide

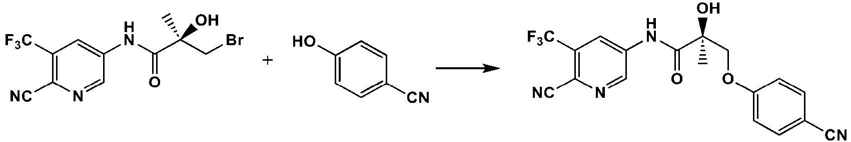

Under nitrogen atmosphere, (R)-3-bromo-N-(6-cyano-5-(trifluoromethyl)pyridin-3-yl)-2-hydroxy-2-methylpropionamide (0.3 mmol) and 4-hydroxybenzonitrile (0.3 mmol) were added to anhydrous butanone (10 mL) and stirred until dissolved. Anhydrous K₂CO₃ (0.8 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine, and dried over anhydrous sodium sulfate. After concentration under reduced pressure, the crude product was obtained, which was then purified by column chromatography to give the target compound (S)-N-(6-cyano-5-(trifluoromethyl)pyridin-3-yl)-3-(4-cyanophenoxy)-2-hydroxy-2-methylpropionamide as a white powdery solid, with a yield of 65%.

¹H NMR (400 MHz, DMSO-d₆) δ 10.88 (s, 1H, NH), 9.39 (d, J = 2.2 Hz, 1H, CH), 8.88 (d, J = 2.3 Hz, 1H, CH), 7.81 - 7.64 (m, 2H, 2*CH), 7.16 - 7.00 (m, 2H, 2*CH), 6.44 (s, 1H, OH), 4.32 (d, J = 9.9 Hz, 1H, 1/2*CH₂), 4.09 (d, J = 9.9 Hz, 1H, 1/2*CH₂), 1.44 (s, 3H, CH₃). LRMS (+ESI) 389.04 m/z: [(M+H)⁺, 100%].

### Example 4 Preparation of (S)-N-(4-cyano-3-(trifluoromethyl)phenyl)-3-((6-cyanopyridin-3-yl)oxy)-2-hydroxy-2-methylpropionamide

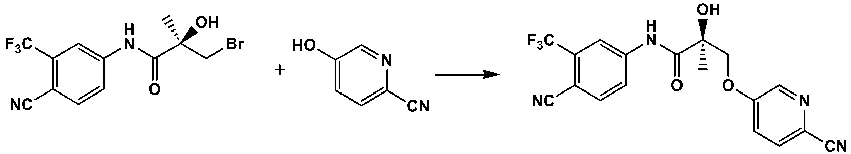

Under nitrogen atmosphere, (R)-3-bromo-N-(4-cyano-3-(trifluoromethyl)phenyl)-2-hydroxy-2-methylpropionamide (0.3 mmol) and 5-hydroxypyridine-2-carbonitrile (0.3 mmol) were added to anhydrous butanone (10 mL) and stirred until dissolved. Anhydrous K₂CO₃ (0.8 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound as a white powdery solid, with a yield of 75%.

¹H NMR (400 MHz, DMSO-d₆) δ 10.61 (s, 1H, NH), 8.53 (d, J = 2.1 Hz, 1H, CH), 8.40 (d, J = 2.9 Hz, 1H, CH), 8.29 (dd, J = 8.6, 2.1 Hz, 1H, CH), 8.09 (d, J = 8.6 Hz, 1H, CH), 7.97 (d, J = 8.7 Hz, 1H, CH), 7.61 (dd, J = 8.8, 3.0 Hz, 1H, CH), 6.40 (s, 1H, OH), 4.43 (d, J = 10.2 Hz, 1H, 1/2*CH₂), 4.18 (d, J = 10.1 Hz, 1H, 1/2*CH₂). LRMS (+ESI) 390.05 m/z: [(M+H)⁺, 100%].

### Example 5 Preparation of (S)-N-(6-cyano-5-(trifluoromethyl)pyridin-3-yl)-3-((6-cyanopyridin-3-yl)oxy)-2-hydroxy-2-methylpropionamide

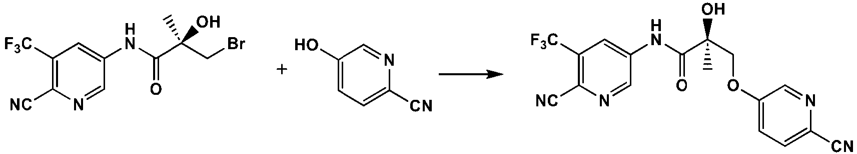

Under nitrogen atmosphere, (R)-3-bromo-N-(6-cyano-5-(trifluoromethyl)pyridin-3-yl)-2-hydroxy-2-methylpropionamide (0.3 mmol) and 5-hydroxypyridine-2-carbonitrile (0.3 mmol) were added to anhydrous butanone (10 mL) and stirred until dissolved. Anhydrous K₂CO₃ (0.8 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound as a white powdery solid, with a yield of 68%.

¹H NMR (400 MHz, DMSO-d₆) δ 10.89 (s, 1H, NH), 9.39 (d, J = 2.3 Hz, 1H, CH), 8.88 (d, J = 2.3 Hz, 1H, CH), 8.41 (dd, J = 3.0, 0.6 Hz, 1H, CH), 7.98 (dd, J = 8.7, 0.7 Hz, 1H, CH), 7.61 (dd, J = 8.8, 2.9 Hz, 1H, CH), 6.49 (s, 1H, OH), 4.42 (d, J = 10.1 Hz, 1H, 1/2*CH₂), 4.20 (d, J = 10.2 Hz, 1H, 1/2*CH₂). LRMS (-ESI) 390.05 m/z: [(M-H)⁻, 100%].

### Example 6 Preparation of (S)-3-(4-chlorophenoxy)-N-(6-cyano-5-(trifluoromethyl)pyridin-3-yl)-2-hydroxy-2-methylpropionamide

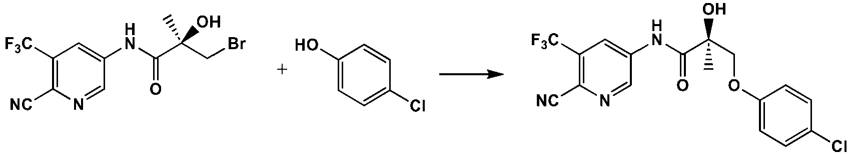

Under nitrogen atmosphere, (R)-3-bromo-N-(6-cyano-5-(trifluoromethyl)pyridin-3-yl)-2-hydroxy-2-methylpropionamide (0.3 mmol) and 4-chlorophenol (0.3 mmol) were added to anhydrous butanone (10 mL) and stirred until dissolved. Anhydrous K₂CO₃ (0.8 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound (S)-3-(4-chlorophenoxy)-N-(6-cyano-5-(trifluoromethyl)pyridin-3-yl)-2-hydroxy-2-methylpropionamide as a white powdery solid, with a yield of 77%.

¹H NMR (500 MHz, DMSO-d₆) δ 10.88 (s, 1H, NH), 9.44 - 9.39 (m, 1H, CH), 8.91 (t, J = 1.6 Hz, 1H, CH), 7.33 - 7.25 (m, 2H, 2*CH), 6.99 - 6.92 (m, 2H, 2*CH), 6.40 (s, 1H, CH), 4.23 (d, J = 9.7 Hz, 1H, 1/2*CH), 4.00 (d, J = 9.8 Hz, 1H, 1/2*CH), 1.47 - 1.43 (m, 3H, CH₃). LRMS (-ESI) m/z: 398.0/399.0/400.0, [(M-H)⁻, 100/20/33%].

### Example 7 Preparation of (S)-3-((6-chloropyridin-3-yl)oxy)-N-(6-cyano-5-(trifluoromethyl)pyridin-3-yl)-2-hydroxy-2-methylpropionamide

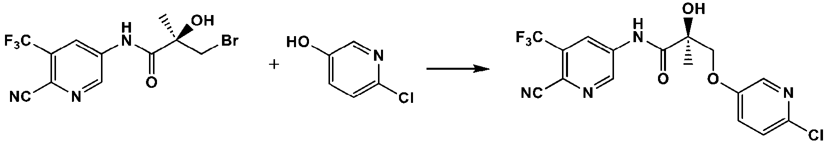

Under nitrogen atmosphere, (R)-3-bromo-N-(6-cyano-5-(trifluoromethyl)pyridin-3-yl)-2-hydroxy-2-methylpropionamide (0.3 mmol) and 2-chloro-5-hydroxypyridine (0.3 mmol) were added to anhydrous butanone (10 mL) and stirred until dissolved. Anhydrous K₂CO₃ (0.8 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound as a white powdery solid, with a yield of 75%.

¹H NMR (400 MHz, DMSO-d₆) δ 10.87 (s, 1H, NH), 9.39 (d, J = 2.2 Hz, 1H, CH), 8.89 (d, J = 2.2 Hz, 1H, CH), 8.10 (d, J = 3.1 Hz, 1H, CH), 7.52 - 7.43 (m, 1H, CH), 7.39 (d, J = 8.8 Hz, 1H, CH), 6.43 (s, 1H, OH), 4.32 (d, J = 10.0 Hz, 1H, 1/2*CH₂), 4.08 (d, J = 10.0 Hz, 1H, 1/2*CH₂), 1.43 (s, 3H, CH₃). LRMS (+ESI) 401.1 m/z: [(M+H)⁺, 100%].

### Example 8 Preparation of (S)-3-(4-cyano-3-fluorophenoxy)-N-(6-cyano-5-(trifluoromethyl)pyridin-3-yl)-2-hydroxy-2-methylpropionamide

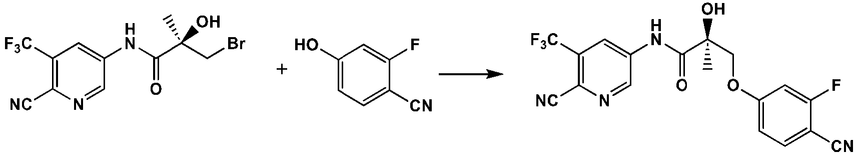

Under nitrogen atmosphere, (R)-3-bromo-N-(6-cyano-5-(trifluoromethyl)pyridin-3-yl)-2-hydroxy-2-methylpropionamide (0.3 mmol) and 2-fluoro-4-hydroxybenzonitrile (0.3 mmol) were added to anhydrous butanone (10 mL) and stirred until dissolved. Anhydrous K₂CO₃ (0.8 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound as a white powdery solid, with a yield of 81%.

¹H NMR (400 MHz, DMSO) δ 10.89 (s, 1H, NH), 9.42 (d, J = 2.3 Hz, 1H, CH), 8.91 (d, J = 2.3 Hz, 1H, CH), 7.85 - 7.76 (m, 1H, CH), 7.19 (dd, J = 11.9, 2.4 Hz, 1H, CH), 6.97 (dd, J = 8.8, 2.4 Hz, 1H, CH), 6.49 (s, 1H, OH), 4.38 (d, J = 10.1 Hz, 1H, 1/2*CH₂), 4.15 (d, J = 10.1 Hz, 1H, 1/2*CH₂), 1.46 (s, 3H, CH₃). LRMS (-ESI) 407.0 m/z: [(M-H)⁻, 100%].

### Example 9 Preparation of (S)-N-(5-chloro-6-cyanopyridin-3-yl)-3-(4-cyanophenoxy)-2-hydroxy-2-methylpropionamide

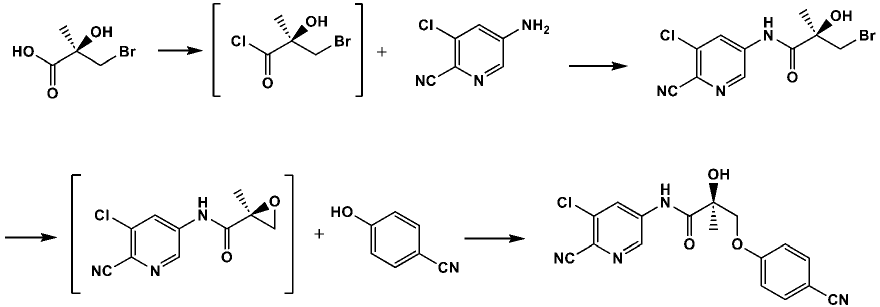

### First and Second Reaction Steps

Under nitrogen atmosphere, (2R)-3-bromo-2-hydroxy-2-methylpropionic acid (2.7 mmol) was added to 10 mL of anhydrous THF. Then, under ice bath conditions, thionyl chloride (3.28 mmol) was slowly added while maintaining the temperature inside the reaction vessel below 0 °C. The reaction solution was gradually heated to room temperature and reacted for 1.5 h. The reaction solution was then placed back under ice bath conditions, and triethylamine (3.55 mmol) was added dropwise while maintaining the temperature inside the reaction vessel below 0 °C. Subsequently, a THF solution of 5-amino-3-chloro-2-pyridinecarbonitrile (2.7 mmol) (2 mL) was added dropwise, while continuing to maintain the temperature inside the reaction vessel below 0 °C. After the addition was complete, the mixture was slowly heated to room temperature, then heated to 50 °C and reacted for 2 h. TLC indicated completion of the reaction. The reaction mixture was cooled to room temperature, then quenched by the addition of water and extracted with 40 mL of ethyl acetate. The organic phase was washed with water and saturated brine, then dried over anhydrous sodium sulfate, filtered, and concentrated under reduced pressure to obtain compound (R)-3-bromo-N-(5-chloro-6-cyanopyridin-3-yl)-2-hydroxy-2-methylpropionamide.

¹H NMR (400 MHz, DMSO-d₆) δ 10.71 (s, 1H, NH), 9.16 (d, J = 2.1 Hz, 1H, CH), 8.71 (d, J = 2.1 Hz, 1H, CH), 6.54 (s, 1H, OH), 3.88 (d, J = 10.4 Hz, 1H, 1/2*CH₂), 3.65 (d, J = 10.6 Hz, 1H, 1/2*CH₂), 1.55 (s, 3H, CH₃). δ LRMS (-ESI) 315.9/317.9/319.9 m/z: [(M-H)⁻, 78%/100%/33%].

### Third and Fourth Reaction Steps

Under nitrogen atmosphere, (R)-3-bromo-N-(5-chloro-6-cyanopyridin-3-yl)-2-hydroxy-2-methylpropionamide (0.7 mmol) and 4-hydroxybenzonitrile (0.8 mmol) were added to anhydrous butanone (10 mL) and stirred until dissolved. Anhydrous K₂CO₃ (2.1 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound (S)-N-(5-chloro-6-cyanopyridin-3-yl)-3-(4-cyanophenoxy)-2-hydroxy-2-methylpropionamide as a white powdery solid, with a yield of 62%.

δ LRMS (-ESI) 355.0 m/z: [(M-H)⁻, 100%].

### Example 10 Preparation of (S)-N-(5-chloro-6-cyanopyridin-3-yl)-3-((6-cyanopyridin-3-yl)oxy)-2-hydroxy-2-methylpropionamide

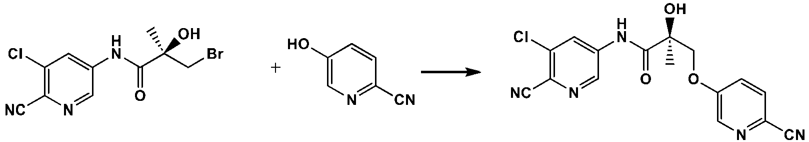

Under nitrogen atmosphere, (R)-3-bromo-N-(5-chloro-6-cyanopyridin-3-yl)-2-hydroxy-2-methylpropionamide (0.7 mmol) and 5-hydroxypyridine-2-carbonitrile (0.8 mmol) were added to anhydrous butanone (10 mL) and stirred until dissolved. Anhydrous K₂CO₃ (2.1 mmol) was added to the solution, and the reaction mixture was heated to 80-85 °C and stirred for 3 h. TLC indicated the reaction was complete. The reaction mixture was filtered to remove K₂CO₃, and the filtrate was washed with water and saturated brine and dried over anhydrous sodium sulfate. After concentration under reduced pressure, the crude product was obtained, which was then purified by column chromatography to give the target compound (S)-N-(5-chloro-6-cyanopyridin-3-yl)-3-((6-cyanopyridin-3-yl)oxy)-2-hydroxy-2-methylpropionamide as a white powdery solid, with a yield of 55%.

δ LRMS (-ESI) 356.1 m/z: [(M-H)⁻, 100%].

### Example 11 Preparation of (S)-1-((4-cyano-3-(trifluoromethyl)phenyl)amino)-3-((6-cyanopyridin-3-yl)oxy)-2-methyl-1-oxopropan-2-yl nicotinate

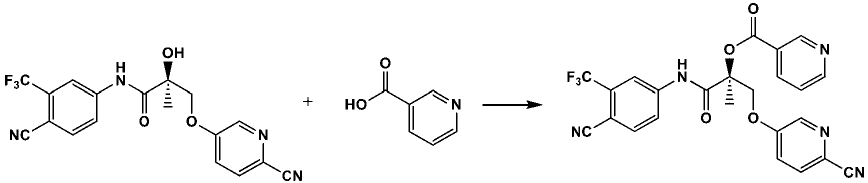

Under nitrogen atmosphere and in an ice bath, (S)-N-(4-cyano-3-(trifluoromethyl)phenyl)-3-((6-cyanopyridin-3-yl)oxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and nicotinic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a white powdery solid, with a yield of 81%.

¹H NMR (400 MHz, DMSO -d₆) δ 10.59 (s, 1H, NH), 9.08 (dd, J = 2.3, 0.8 Hz, 1H, CH), 8.84 (dd, J = 4.8, 1.7 Hz, 1H, CH), 8.51 (d, J = 2.8 Hz, 1H, CH), 8.31 - 8.22 (m, 2H, 2*CH), 8.18 - 8.05 (m, 2H, 2*CH), 8.00 (d, J = 8.7 Hz, 1H, CH), 7.72 (dd, J = 8.8, 2.9 Hz, 1H, CH), 7.58 (ddd, J = 8.0, 4.9, 0.9 Hz, 1H, CH), 4.76 (q, J = 10.8 Hz, 2H, CH₂), 1.87 (s, 3H, CH₃). LRMS (+ESI) 496.13 m/z: [(M+H)⁺, 100%].

### Example 12 Preparation of (S)-1-((6-cyano-5-(trifluoromethyl)pyridin-3-yl)amino)-3-((6-cyanopyridin-3-yl)oxy)-2-methyl-1-oxopropan-2-yl nicotinate

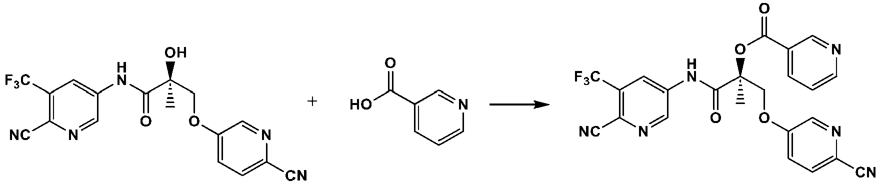

Under nitrogen atmosphere and in an ice bath, (S)-N-(6-cyano-5-(trifluoromethyl)pyridin-3-yl)-3-((6-cyanopyridin-3-yl)oxy)-2-hydroxy-2-methylpropionamide (0.1 mmol), triethylamine (0.2 mmol), and 2,4,6-trichlorobenzoyl chloride (0.2 mmol) were added to toluene (2 mL) and stirred until dissolved. Subsequently, 4-dimethylaminopyridine (0.4 mmol) and nicotinic acid (0.4 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a white powdery solid, with a yield of 3%.

LRMS (-ESI) 495.0 m/z: [(M-H)⁻, 100%].

### Example 13 Preparation of (S)-1-((6-cyano-5-(trifluoromethyl)pyridin-3-yl)amino)-3-(4-cyanophenoxy)-2-methyl-1-oxopropan-2-yl nicotinate

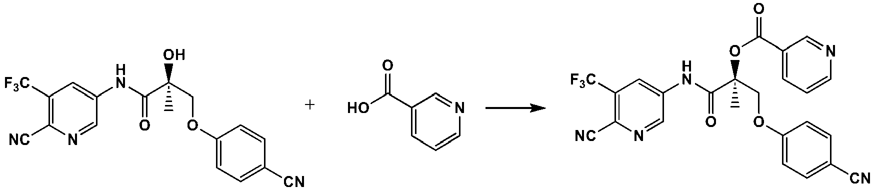

Under nitrogen atmosphere and in an ice bath, (S)-N-(6-cyano-5-(trifluoromethyl)pyridin-3-yl)-3-(4-cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and nicotinic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a white powdery solid, with a yield of 75%.

¹H NMR (400 MHz, DMSO-d₆) δ 10.83 (s, 1H, NH), 9.21 (s, 1H, CH), 9.12 - 9.05 (m, 1H, CH), 8.82 (ddt, J = 15.7, 5.1, 1.5 Hz, 1H, CH), 8.64 (d, J = 2.3 Hz, 1H, CH), 8.28 (dd, J = 8.0, 2.2 Hz, 1H, CH), 7.84 - 7.72 (m, 2H, 2*CH), 7.62 - 7.52 (m, 1H, CH), 7.23 - 7.16 (m, 1H, CH), 7.18 (s, 1H, CH), 4.74 - 4.59 (m, 2H, CH₂), 1.87 (s, 3H, CH₃). LRMS (+ESI) 496.1 m/z: [(M+H)⁺, 100%].

### Example 14 Preparation of (S)-1-((6-cyano-5-(trifluoromethyl)pyridin-3-yl)amino)-3-(4-cyanophenoxy)-2-methyl-1-oxopropyl-2-yl 3-(dimethylamino)propanate

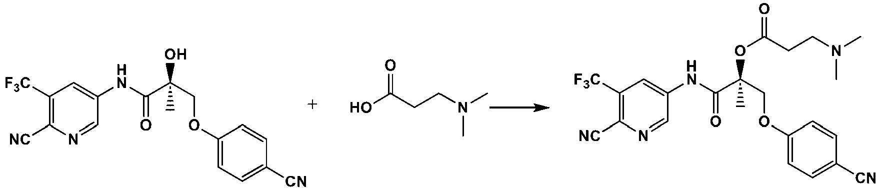

Under nitrogen atmosphere and in an ice bath, (S)-N-(6-cyano-5-(trifluoromethyl)pyridin-3-yl)-3-(4-cyanophenoxy)-2-hydroxy-2-methylpropionamide (0.1 mmol), triethylamine (0.2 mmol), and 2,4,6-trichlorobenzoyl chloride (0.2 mmol) were added to toluene (2 mL) and stirred until dissolved. Subsequently, 4-dimethylaminopyridine (0.4 mmol) and 3-dimethylaminopropylamine hydrochloride (0.4 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a white powdery solid, with a yield of 3%.

LRMS (+ESI) 490.2 m/z: [(M+H)⁺, 100%].

### Example 15 Preparation of (S)-1-((6-cyano-5-(trifluoromethyl)pyridin-3-yl)amino)-3-(4-cyanophenoxy)-2-methyl-1-oxopropyl-2-yl 5-((R)-1,2-dithiolan-3-yl) pentanoate

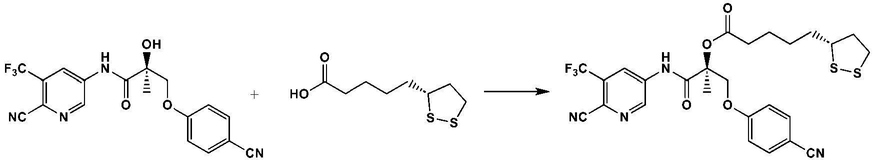

Under nitrogen atmosphere and in an ice bath, (S)-N-(6-cyano-5-(trifluoromethyl)pyridin-3-yl)-3-(4-cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and R-(+)-lipoic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a white powdery solid, with a yield of 35%.

¹H NMR (400 MHz, DMSO -d₆) δ 10.77 (s, 1H, NH), 9.23 (d, J = 2.3 Hz, 1H, CH), 8.66 (d, J = 2.3 Hz, 1H, CH), 7.85 - 7.68 (m, 2H, CH₂), 7.24 - 7.07 (m, 2H, CH₂), 4.55 (d, J = 10.6 Hz, 1H, 1/2*CH₂), 4.48 (d, J = 10.5 Hz, 1H, 1/2*CH₂), 3.48 (dq, J = 8.8, 6.2 Hz, 1H, CH), 3.18 - 2.99 (m, 2H, CH₂), 2.42 (td, J = 7.1, 2.1 Hz, 2H, CH₂), 2.32 (dtd, J = 12.9, 6.5, 5.6 Hz, 1H, 1/2*CH), 1.77 (dd, J = 12.8, 6.7 Hz, 1H, 1/2*CH), 1.72 (s, 3H, CH₃), 1.59 (ddd, J = 13.8, 8.2, 4.4 Hz, 1H, 1/2*CH), 1.52 - 1.40 (m, 1H, 1/2*CH), 1.37 - 1.23 (m, 3H, CH, CH₂).1.22 (d, J = 6.2 Hz, 1H, 1/2*CH). LRMS (+ESI) 579.2 m/z: [(M+H)⁺, 100%].

### Example 16 Preparation of (S)-1-((6-cyano-5-(trifluoromethyl)pyridin-3-yl)amino)-3-((6-cyanopyridin-3-yl)oxy)-2-methyl-1-oxopropyl-2-yl 5-((R)-1,2-dithiolan-3-yl) pentanoate

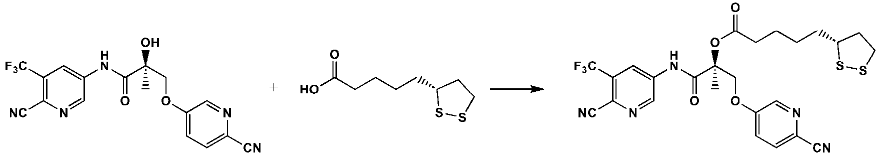

Under nitrogen atmosphere and in an ice bath, (S)-N-(6-cyano-5-(trifluoromethyl)pyridin-3-yl)-3-((6-cyanopyridin-3-yl)oxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and R-(+)-lipoic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a white powdery solid, with a yield of 21%.

¹H NMR (400 MHz, DMSO-d₆) δ 10.76 (s, 1H, NH), 9.22 (d, J = 2.3 Hz, 1H, CH), 8.65 (d, J = 2.3 Hz, 1H, CH), 8.52 - 8.41 (m, 1H, CH), 8.00 (d, J = 0.6 Hz, 1H, CH), 7.67 (dd, J = 8.7, 2.9 Hz, 1H, CH), 4.66 - 4.55 (m, 2H, CH₂), 3.49 (dq, J = 8.8, 6.2 Hz, 1H, CH), 3.18 - 3.00 (m, 2H, CH₂), 2.42 (t, J = 7.1 Hz, 2H, CH₂), 2.32 (dq, J = 12.5, 6.3 Hz, 1H, 1/2*CH₂), 1.84 - 1.74 (m, 1H, 1/2*CH₂), 1.73 (s, 3H, 1/2*CH₂, CH₂), 1.70 - 1.40 (m, 3H, 1/2*CH₂, CH₂), 1.36 - 1.23 (m, 3H, CH₃). LRMS (+ESI) 578.0 m/z: [(M+H)⁺, 100%].

### Example 17 Preparation of (S)-1-((6-cyano-5-(trifluoromethyl)pyridin-3-yl)amino)-3-(4-cyanophenoxy)-2-methyl-1-oxopropan-2-yl propane-1-sulfonate

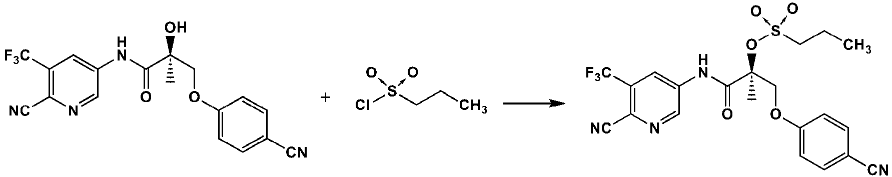

Under nitrogen atmosphere and in an ice bath, (S)-N-(6-cyano-5-(trifluoromethyl)pyridin-3-yl)-3-(4-cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and propylsulfonyl chloride (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound as a pale yellow oil, with a yield of 5%.

LRMS (+ESI) 497.0 m/z: [(M+H)⁺, 100%].

### Example 18 Preparation of (S)-1-((6-cyano-5-(trifluoromethyl)pyridin-3-yl)amino)-3-((6-cyanopyridin-3-yl)oxy)-2-methyl-1-oxopropan-2-yl propane-1-sulfonate

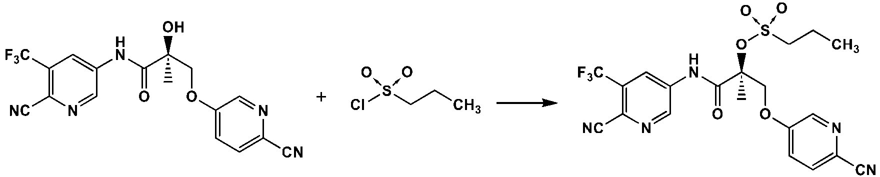

Under nitrogen atmosphere and in an ice bath, (S)-N-(6-cyano-5-(trifluoromethyl)pyridin-3-yl)-3-((6-cyanopyridin-3-yl)oxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and propylsulfonyl chloride (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-((6-cyanopyridin-3-yl)oxy)-2-methyl-1-oxopropan-2-yl propane-1-sulfonate as a pale yellow oil, with a yield of 7%.

LRMS (-ESI) 496.0 m/z: [(M-H)⁻, 100%].

### Example 19 Preparation of (S)-1-((6-cyano-5-(trifluoromethyl)pyridin-3-yl)amino)-3-(4-cyanophenoxy)-2-methyl-1-oxopropyl-2-yl-1-methylpiperidine-4-carboxylate

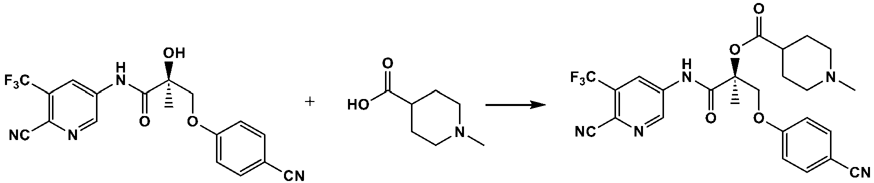

Under nitrogen atmosphere and in an ice bath, (S)-N-(6-cyano-5-(trifluoromethyl)pyridin -3-yl)-3-(4-cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and 1-methylpiperidin-4-carboxylic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropyl-2-yl-1-methylpiperidine-4-carboxylate as a pale yellow powdery solid, with a yield of 15%.

¹H NMR (400 MHz, DMSO-d₆) δ 10.88 (s, 1H, NH), 9.40 (d, J = 2.3 Hz, 1H, CH), 8.89 (d, J = 2.3 Hz, 1H, CH), 7.75 (m, 2H, 2*CH), 7.19 - 7.03 (m, 2H, 2*CH), 4.32 (d, J = 10.0 Hz, 1H, 1/2*CH₂), 4.09 (d, J = 10.0 Hz, 1H, 1/2*CH₂), 2.68 (d, J = 15.1 Hz, 2H, CH₂), 2.19 - 2.10 (m, 3H, CH₃), 1.95 (s, 2H, CH₂), 1.77 (d, J = 8.9 Hz, 2H, CH₂), 1.61 - 1.47 (m, 2H, CH₂), 1.44 (s, 3H, CH₃). LRMS (+ESI) 516.2 m/z: [(M+H)⁺, 100%].

### Example 20 Preparation of (S)-1-((6-cyano-5-(trifluoromethyl)pyridin-3-yl)amino)-3-((6-cyanopyridin-3-yl)oxy)-2-methyl-1-oxopropan-2-yl-1-methylpiperidine- 4-carboxylate

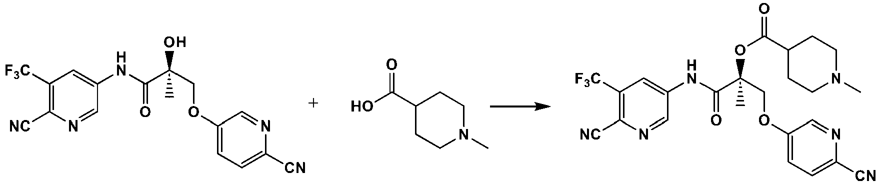

Under nitrogen atmosphere and in an ice bath, (S)-N-(6-cyano-5-(trifluoromethyl)pyridin-3-yl)-3-((6-cyanopyridin-3-yl)oxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and 1-methylpiperidin-4-carboxylic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-((6-cyanopyridin-3-yl)oxy)-2-methyl-1-oxopropan-2-yl-1-methylpiperidine-4-carboxylate as a pale yellow powdery solid, with a yield of 10%.

LRMS (+ESI) 517.1 m/z: [(M+H)⁺, 100%].

### Example 21 Preparation of (S)-1-((6-cyano-5-(trifluoromethyl)pyridin-3-yl)amino)-3-(4-cyanophenoxy)-2-methyl-1-oxopropan-2-yl isobutyrate

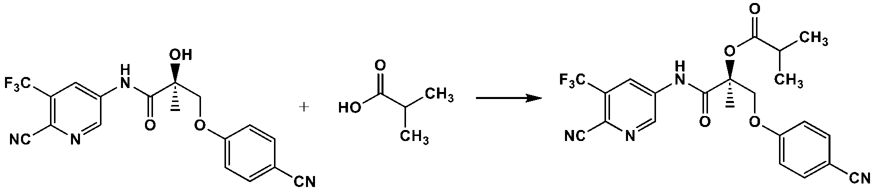

Under nitrogen atmosphere and in an ice bath, (S)-N-(6-cyano-5-(trifluoromethyl)pyridin-3-yl)-3-(4- cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and isobutyric acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl isobutyrate, which is a white powdery solid, with a yield of 25%.

¹H NMR (500 MHz, DMSO-d₆) δ 10.70 (s, 1H, NH), 9.24 (d, J = 2.3 Hz, 1H, CH), 8.68 (d, J = 2.3 Hz, 1H, CH), 7.82 - 7.75 (m, 2H, 2*CH), 7.17 (d, J = 8.4 Hz, 2H, 2*CH), 4.57 (d, J = 10.4 Hz, 1H, 1/2*CH₂), 4.51 (d, J = 10.5 Hz, 1H, 1/2*CH₂), 1.74 (s, 3H, CH₃), 1.11 - 1.05 (m, 6H, 2*CH₃). LRMS (+ESI) 461.1 m/z: [(M+H)⁺, 100%], LRMS (-ESI) 459.1 m/z: [(M-H)⁻, 100%].

### Example 22 Preparation of (S)-1-((6-cyano-5-(trifluoromethyl)pyridin-3-yl)amino) -3- (4-cyanophenoxy)-2-methyl-1-oxopropan-2-yl cyclopentanecarboxylate

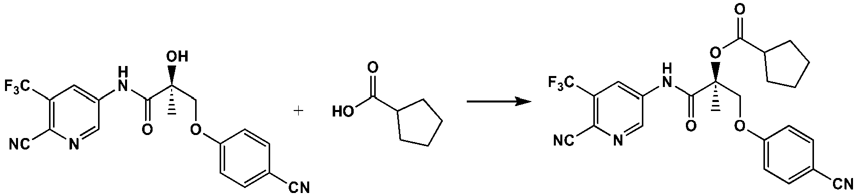

Under nitrogen atmosphere and in an ice bath, (S)-N-(6-cyano-5-(trifluoromethyl)pyridin-3-yl)-3-(4- cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and cyclopentanecarboxylic acid (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl cyclopentanecarboxylate as a white powdery solid, with a yield of 28%.

¹H NMR (500 MHz, DMSO-d₆) δ 10.71 (s, 1H, NH), 9.23 (d, J = 2.3 Hz, 1H, CH), 8.68 (d, J = 2.3 Hz, 1H, CH), 7.82 - 7.75 (m, 2H, CH₂), 7.20 - 7.14 (m, 2H, CH₂), 4.57 (d, J = 10.5 Hz, 1H, 1/2*CH₂), 4.50 (d, J = 10.5 Hz, 1H, 1/2*CH₂), 1.89 - 1.79 (m, 2H, CH₂), 1.74 (s, 3H, CH₃), 1.67 (ddt, J = 11.5, 7.3, 3.8 Hz, 2H, CH₂), 1.53 (tt, J = 7.5, 3.3 Hz, 4H, 2*CH₂). LRMS (+ESI) 487.1 m/z: [(M+H)⁺, 100%], LRMS (-ESI) 485.1 m/z: [(M-H)⁻, 100%].

### Example 23 Preparation of (S)-1-((6-cyano-5-(trifluoromethyl)pyridin-3-yl)amino)-3-(4-cyanophenoxy)-2-methyl-1-oxopropan-2-yl N-methyl-L-prolinate

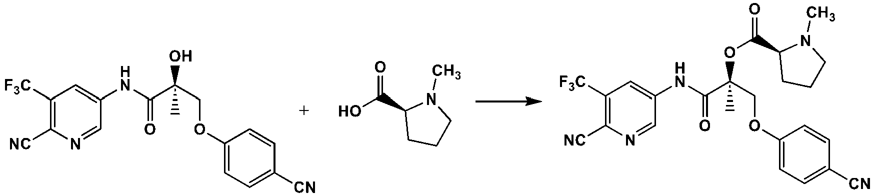

Under nitrogen atmosphere and in an ice bath, (S)-N-(6-cyano-5-(trifluoromethyl)pyridin -3-yl)-3-(4- cyanophenoxy)-2-hydroxy-2-methylpropionamide (0.8 mmol), triethylamine (1.9 mmol), and 2,4,6-trichlorobenzoyl chloride (1.9 mmol) were added to toluene (10 mL) and stirred until dissolved. Subsequently, 4-dimethylaminopyridine (3.8 mmol) and N-methyl-L-proline (1.6 mmol) were added to the solution, and the reaction mixture was maintained below 0 °C and stirred for 30 min. TLC indicated the reaction was complete. The reaction solution was quenched with a saturated aqueous sodium bicarbonate solution and extracted with ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl N-methyl-L-prolinate as a white powdery solid, with a yield of 36%.

¹H NMR (500 MHz, DMSO-d₆) δ 10.75 (s, 1H, NH), 9.22 (s, 1H, CH), 8.69 (s, 1H, CH), 7.79 (d, J = 8.5 Hz, 2H, 2*CH), 7.17 (d, J = 8.3 Hz, 2H, 2*CH), 4.60 (d, J = 10.5 Hz, 1H, 1/2*CH₂), 4.52 (d, J = 10.4 Hz, 1H, 1/2*CH₂), 3.13 (t, J = 7.5 Hz, 1H, 1/2*CH₂), 2.95 - 2.87 (m, 1H, 1/2*CH₂), 2.29 (s, 3H, CH₃), 2.07 (dt, J = 17.0, 8.7 Hz, 1H, 1/2*CH₂), 1.83 (dq, J = 11.9, 5.7 Hz, 1H, 1/2*CH₂), 1.77 (s, 3H, CH₃), 1.72 (dd, J = 8.4, 3.8 Hz, 1H, 1/2*CH₂), 1.68 (d, J = 9.0 Hz, 1H, 1/2*CH₂). LRMS (+ESI) 502.2 m/z: [(M+H)⁺, 100%], LRMS (-ESI) 500.1 m/z: [(M-H)⁻, 100%].

### Example 24 Preparation of (S)-1-((6-cyano-5-(trifluoromethyl)pyridin-3-yl)amino)-3-(4-cyanophenoxy)-2-methyl-1-oxopropan-2-yl acetate (SARM-46)

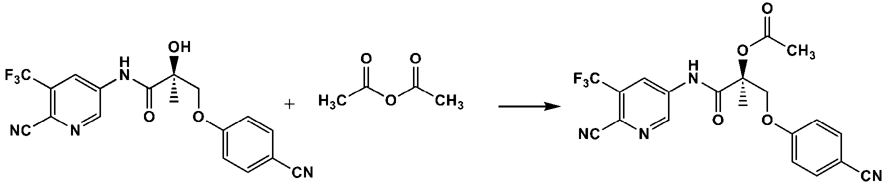

Under nitrogen atmosphere, (S)-N-(6-cyano-5-(trifluoromethyl)pyridin-3-yl)-3-(4-cyanophenoxy)-2-hydroxy-2-methylpropionamide (0.3 mmol), 4-dimethylaminopyridine (0.4 mmol), and acetic anhydride (0.6 mmol) were added to 5 mL of pyridine and stirred to dissolve. The reaction mixture was heated to 80-85 °C and stirred for 30 min. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl acetate as a white powdery solid, with a yield of 77%.

¹H NMR (400 MHz, DMSO-d₆) δ 10.76 (s, 1H, NH), 9.23 (d, J = 2.3 Hz, 1H, CH), 8.65 (d, J = 2.3 Hz, 1H, CH), 7.85 - 7.70 (m, 2H, 2*CH), 7.24 - 7.07 (m, 2H, 2*CH), 4.49 (h, J = 9.5 Hz, 2H, CH₂), 2.11 (s, 3H, CH₃), 1.71 (s, 3H, CH₃). LRMS (-ESI) 431.0 m/z: [(M-H)⁻, 100%].

### Example 25 Preparation of (S)-1-((6-cyano-5-(trifluoromethyl)pyridin-3-yl)amino)-3-((6-cyanopyridin-3-yl)oxy)-2-methyl-1-oxopropyl-2-yl acetate

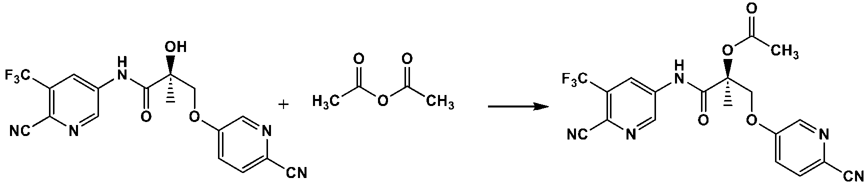

Under nitrogen atmosphere, (S)-N-(6-cyano-5-(trifluoromethyl)pyridin-3-yl)-3-((6-cyanopyridin-3-yl)oxy)-2-hydroxy-2-methylpropionamide (0.3 mmol), 4-dimethylaminopyridine (0.4 mmol), and acetic anhydride (0.6 mmol) were added to 5 mL of pyridine and stirred to dissolve. The reaction mixture was heated to 80-85 °C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-((6-cyanopyridin-3-yl)oxy)-2-methyl-1-oxopropyl-2-yl acetate as a white powdery solid, with a yield of 90%.

¹H NMR (400 MHz, DMSO-d₆) δ 10.78 (s, 1H, CH), 9.24 (d, J = 2.2 Hz, 1H, CH), 8.65 (d, J = 2.3 Hz, 1H, CH), 8.49 (dd, J = 2.9, 0.6 Hz, 1H, CH), 8.01 (dd, J = 8.7, 0.6 Hz, 1H, CH), 7.67 (dd, J = 8.7, 2.9 Hz, 1H, CH), 4.65 4.53 (m, 2H, CH₂), 2.11 (s, 3H, CH₃), 1.72 (s, 3H, CH₃). LRMS (-ESI) 432.0 m/z: [(M-H)⁻, 100%].

### Example 26 Preparation of (S)-1-((6-cyano-5-(trifluoromethyl)pyridin-3-yl)amino)-3-(4-cyanophenoxy)-2-methyl-1-oxopropan-2-yl benzoate

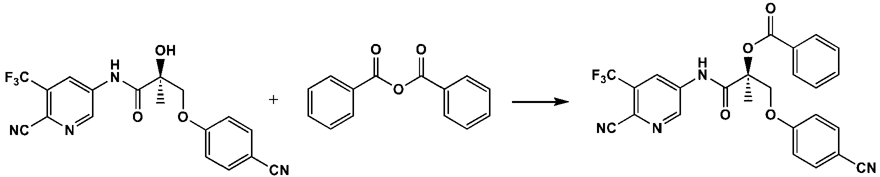

Under nitrogen atmosphere, (S)-N-(6-cyano-5-(trifluoromethyl)pyridin-3-yl)-3-(4-cyanophenoxy)-2-hydroxy-2-methylpropionamide (0.3 mmol), 4-dimethylaminopyridine (0.4 mmol), and benzoic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl benzoate as a white powdery solid, with a yield of 78%.

¹H NMR (400 MHz, DMSO-d₆) δ 10.85 (s, 1H, NH), 9.23 (d, J = 2.3 Hz, 1H, CH), 8.66 (d, J = 2.3 Hz, 1H, CH), 8.03 - 7.87 (m, 2H, 2*CH), 7.84 - 7.73 (m, 1H, CH), 7.73 (d, J = 15.3 Hz, 1H, CH), 7.72 - 7.57 (m, 1H, CH), 7.57 - 7.44 (m, 2H, 2*CH), 7.19 (d, J = 6.9 Hz, 1H, CH), 4.69 (d, J = 10.8 Hz, 1H, 1/2*CH₂), 4.63 (d, J = 10.8 Hz, 1H, 1/2*CH₂), 1.85 (s, 3H, CH₃). LRMS (-ESI) 493.0 m/z: [(M-H)⁻, 100%].

### Example 27 Preparation of (S)-1-((6-cyano-5-(trifluoromethyl)pyridin-3-yl)amino)-3-((6-cyanopyridin-3-yl)oxy)-2-methyl-1-oxopropan-2-yl benzoate

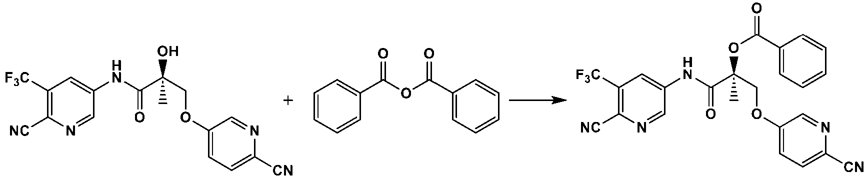

Under nitrogen atmosphere, (S)-N-(6-cyano-5-(trifluoromethyl)pyridin-3-yl)-3-((6-cyanopyridin-3-yl)oxy)-2-hydroxy-2-methylpropionamide (0.3 mmol), 4-dimethylaminopyridine (0.4 mmol), and benzoic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-((6-cyanopyridin-3-yl)oxy)-2-methyl-1-oxopropan-2-yl benzoate as a white powdery solid, with a yield of 91%.

¹H NMR (400 MHz, DMSO-d₆) δ 10.86 (s, 1H, NH), 9.24 (s, 1H, CH), 8.65 (s, 1H, CH), 8.51 (d, J = 2.8 Hz, 1H, CH), 8.01 (d, J = 8.7 Hz, 1H, CH), 7.94 (d, J = 7.6 Hz, 2H, 2*CH), 7.76 - 7.65 (m, 2H, 2*CH), 7.53 (t, J = 7.7 Hz, 2H, 2*CH), 4.81 - 4.69 (m, 2H, CH₂), 1.86 (s, 3H, CH₃). LRMS (-ESI) 494.0 m/z: [(M-H)⁻, 100%].

### Example 28 Preparation of (S)-1-((6-cyano-5-(trifluoromethyl)pyridin-3-yl)amino)-3-(4-cyanophenoxy)-2-methyl-1-oxopropan-2-yl-propionate

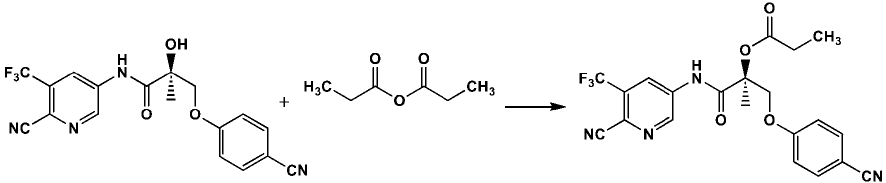

Under nitrogen atmosphere, (S)-N-(6-cyano-5-(trifluoromethyl)pyridin-3-yl)-3-(4-cyanophenoxy)-2-hydroxy-2-methylpropionamide (0.3 mmol), 4-dimethylaminopyridine (0.4 mmol), and propionic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl propionate as a white powdery solid, with a yield of 95%.

¹H NMR (500 MHz, DMSO-d₆) δ 10.74 (s, 1H, NH), 9.25 (d, J = 2.2 Hz, 1H, CH), 8.68 (d, J = 2.3 Hz, 1H, CH), 7.81 - 7.76 (m, 2H, 2*CH), 7.21 - 7.14 (m, 2H, 2*CH), 4.58 - 4.53 (m, 1H, 1/2*CH₂), 4.50 (dd, J = 10.5, 1.9 Hz, 1H, 1/2*CH₂), 2.45 (qd, J = 7.5, 1.8 Hz, 2H, CH₂), 1.74 (d, J = 2.0 Hz, 3H, CH₃), 1.01 (td, J = 7.5, 1.9 Hz, 3H, CH₃). LRMS (+ESI) 447.1 m/z: [(M+H)⁺, 100%], LRMS (-ESI) 445.1 m/z: [(MH)-, 100%].

### Example 29 Preparation of (S)-1-((6-cyano-5-(trifluoromethyl)pyridin-3-yl)amino)-3-(4-chlorophenoxy)-2-methyl-1-oxopropan-2-yl nicotinate

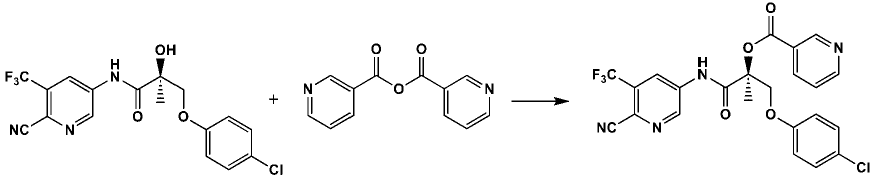

Under nitrogen atmosphere, (S)-3-(4-chlorophenoxy)-N-(6-cyano-5-(trifluoromethyl)pyridin-3-yl)-2-hydroxy-2-methylpropionamide (0.3 mmol), 4-dimethylaminopyridine (0.4 mmol), and nicotinic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-chlorophenoxy) -2-methyl-1-oxopropan-2-ylnicotinate as a white powdery solid, with a yield of 91%.

¹H NMR (500 MHz, DMSO-d₆) δ 10.83 (s, 1H, NH), 9.24 (s, 1H, CH), 9.12 (d, J = 2.2 Hz, 1H, CH), 8.86 (dt, J = 4.8, 1.5 Hz, 1H, CH), 8.66 (d, J = 2.4 Hz, 1H, CH), 8.29 (dq, J = 8.1, 1.8 Hz, 1H, CH), 7.60 (dd, J = 8.0, 4.9 Hz, 1H, CH), 7.36 - 7.29 (m, 2H, 2*CH), 7.09 - 7.02 (m, 2H, 2*CH), 4.62 (d, J = 10.7 Hz, 1H, 1/2*CH₂), 4.57 (d, J = 10.8 Hz, 1H, 1/2*CH₂), 1.88 (d, J = 1.3 Hz, 3H, CH₃). LRMS (-ESI) m/z: 503.1/504.1/505.1, [(M-H)⁻, 100/30%/33%].

### Example 30 Preparation of (S)-1-((6-cyano-5-(trifluoromethyl)pyridin-3-yl)amino) -3- ((6-chloropyridin- 3 -yl) oxy)-2-methyl-1-oxopropan-2-yl nicotinate

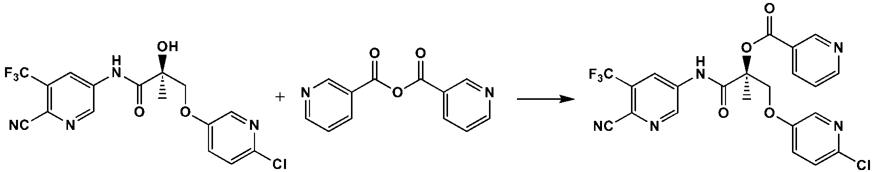

Under nitrogen atmosphere, (S)-3-((6-chloropyridin-3-yl)oxy)-N-(6-cyano-5-(trifluoromethyl)pyridin-3-yl)-2-hydroxy-2-methylpropionamide (0.3 mmol), 4-dimethylaminopyridine (0.4 mmol), and nicotinic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-((6- chloropyridin- 3 -yl) oxy)-2-methyl-1-oxopropan-2-ylnicotinate as a white powdery solid, with a yield of 91%.

¹H NMR (500 MHz, DMSO-d₆) δ 10.85 (s, 1H, NH), 9.25 (d, J = 2.0 Hz, 1H, CH), 9.12 (dd, J = 2.2, 0.8 Hz, 1H, CH), 8.87 (dd, J = 4.8, 1.7 Hz, 1H, CH), 8.66 (d, J = 2.3 Hz, 1H, CH), 8.30 (dt, J = 8.0, 2.0 Hz, 1H, CH), 8.22 (d, J = 3.1 Hz, 1H, CH), 7.64 - 7.58 (m, 2H, 2*CH), 7.44 (d, J = 8.8 Hz, 1H, CH), 4.74 - 4.65 (m, 2H, CH₂), 1.89 (s, 3H, CH₃). LRMS (-ESI) m/z: 506.1/507.1/508.1, [(M-H)⁻, 100/20%/33%].

### Example 31 Preparation of (S)-3-(4-cyano-3-fluorophenoxy)-1-((6-cyano-5-(trifluoromethyl)pyridin-3-yl)amino)-2-methyl-1-oxopropyl-2-yl nicotinate

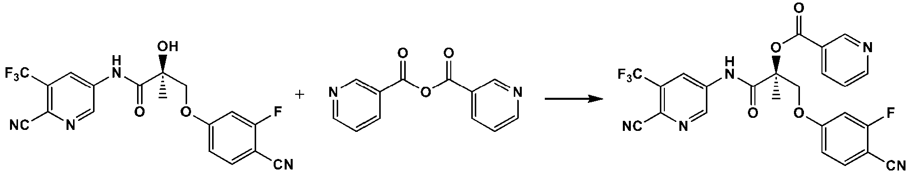

Under nitrogen atmosphere, (S)-3-(4-cyano-3-fluorophenoxy)-N-(6-cyano-5-(trifluoromethyl)pyridin-3-yl)-2-hydroxy-2-methylpropionamide (0.3 mmol), 4-dimethylaminopyridine (0.4 mmol), and nicotinic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-3-(4-cyano-3-fluorophenoxy)-1-((6-cyano-5-(trifluoromethyl)pyridin-3-yl)amino)-2-methyl-1-oxopropyl-2-yl nicotinate as a white powdery solid, with a yield of 88%.

LRMS (-ESI) 512.0 m/z: [(M-H)⁻, 100%].

### Example 32 Preparation of (S)-3-(4-cyano-3-fluorophenoxy)-1-((6-cyano-5-(trifluoromethyl)pyridin-3-yl)amino)-2-methyl-1-oxopropyl-2-yl acetate

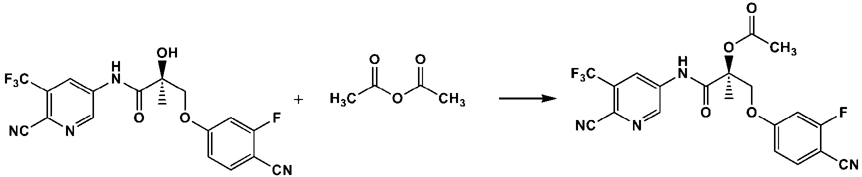

Under nitrogen atmosphere, (S)-3-(4-cyano-3-fluorophenoxy)-N-(6-cyano-5-(trifluoromethyl)pyridin-3-yl)-2-hydroxy-2-methylpropionamide (0.3 mmol), 4-dimethylaminopyridine (0.4 mmol), and acetic anhydride (0.6 mmol) were added to 5 mL of pyridine and stirred to dissolve. The reaction mixture was heated to 80-85 ° C and stirred for 3 h. TLC indicated completion of the reaction. The reaction solution was added to water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-3-(4-cyano-3-fluorophenoxy)-1-((6-cyano-5-(trifluoromethyl)pyridin-3-yl)amino)-2-methyl-1-oxopropyl-2-yl acetate as a white powdery solid, with a yield of 80%.

LRMS (-ESI) 449.1 m/z: [(M-H)⁻, 100%].

### Example 33 Preparation of (S)-3-(((1-((4-cyano-3-(trifluoromethyl)phenyl)amino)-3-(4-cyanophenoxy)-2-methyl-1-oxopropan-2-yl)oxy)carbonyl)-1-methylpyridin-1-ium trifluoromethanesulfonate

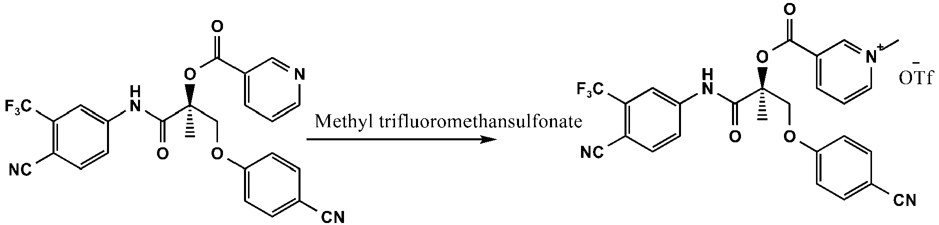

Under an inert gas atmosphere, (S)-1-((4-cyano-3-(trifluoromethyl)phenyl)amino)-3-(4-cyanophenoxy)-2-methyl-1-oxopropyl-2-yl nicotinate (0.2 mmol) and methyl trifluoromethanesulfonate (0.3 mmol) were added to DCM and stirred at room temperature for 3 h. TLC indicated the reaction was complete. 5 mL of diethyl ether was added to the solution, and the mixture was stirred and filtered. The filter cake was washed with 10 mL of diethyl ether and dried to obtain the target compound (S)-3-(((1-((4-cyano-3-(trifluoromethyl)phenyl)amino)-3-(4-cyanophenoxy)-2-methyl-1-oxopropan-2-yl)oxy)carbonyl)-1-methylpyridin-1-ium trifluoromethanesulfonate onium salt as a white powdery solid, with a yield of 85%.

¹H NMR (400 MHz, DMSO-d₆) δ 10.60 (s, 1H, NH), 9.56 (s, 1H, CH), 9.20 (d, J = 6.1 Hz, 1H, CH), 8.99 (d, J = 8.1 Hz, 1H, CH), 8.28 (dd, J = 8.1, 6.1 Hz, 1H, CH), 8.22 (s, 1H, CH), 8.13 (d, J = 1.2 Hz, 2H, 2*CH), 7.86 - 7.70 (m, 2H, 2*CH), 7.27 - 7.10 (m, 2H, 2*CH), 4.75 - 4.63 (m, 2H, 2*CH), 4.41 (s, 3H, CH₃), 1.92 (s, 3H, CH₃). LRMS (-ESI) 509.0 m/z: [(M-H)⁻, 100%].

### Example 34 Preparation of (S)-3-(((1-((6-cyano-5-(trifluoromethyl)pyridin-3-yl)amino)-3-(4-cyanophenoxy)-2-methyl-1-oxopropan-2-yl)oxy)carbonyl)-1-methylpyridin-1-ium trifluoromethanesulfonate

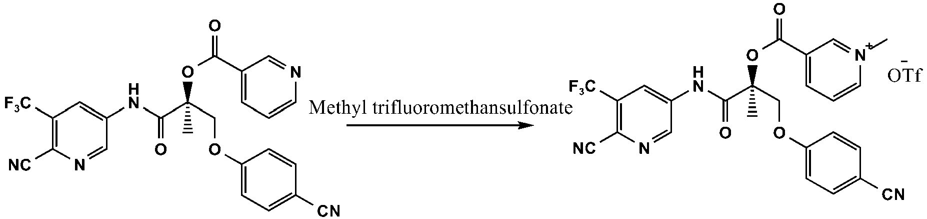

Under an inert gas atmosphere, (S)-1-((6-cyano-5-(trifluoromethyl)pyridin-3-yl)amino)-3-(4-cyanophenoxy)-2-methyl-1-oxopropyl-2-yl nicotinate (0.2 mmol) and methyl trifluoromethanesulfonate (0.3 mmol) were added to DCM, and the mixture was stirred at room temperature for 3 h. TLC indicated the reaction was complete. 5 mL of diethyl ether was added to the solution, and the mixture was stirred and filtered. The filter cake was washed with 10 mL of diethyl ether and dried to obtain the target compound as a white powdery solid, with a yield of 60%.

¹H NMR (500 MHz, DMSO-d₆) δ 10.89 (s, 1H, NH), 9.60 (d, J = 11.1 Hz, 1H, CH), 9.28 - 9.21 (m, 2H, 2*CH), 9.03 (t, J = 7.7 Hz, 1H, CH), 8.32 (ddd, J = 11.6, 8.0, 6.0 Hz, 1H, CH), 7.82 (dd, J = 15.0, 8.7 Hz, 2H, 2*CH), 7.24 - 7.17 (m, 2H, 2*CH), 4.74 - 4.67 (m, 2H, CH₂), 4.44 (d, J = 9.0 Hz, 3H, CH₃), 1.96 (d, J = 5.3 Hz, 3H, CH₃). LRMS (+ESI) 510.1/511.1 m/z: [(M+H)⁺, 100/33%].

### Example 35 Preparation of (S)-1-((4-cyano-3-(trifluoromethyl)phenyl)amino)-3-(4-cyanophenoxy)-2-methyl-1-oxopropan-2-yl 1-methyl-1,4-dihydropyridine-3-carboxylate

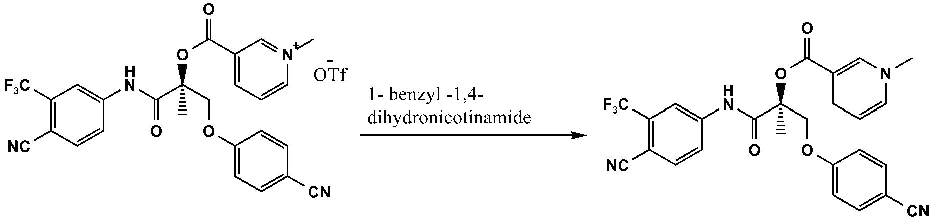

Under nitrogen atmosphere, (S)-3-(((1-((4-cyano-3-(trifluoromethyl)phenyl)amino)-3-(4-cyanophenoxy)-2-methyl-1-oxopropan-2-yl)oxy)carbonyl)-1-methylpyridinium-1-trifluoromethanesulfonate (0.1 mmol) and 1-benzyl-1,4-dihydronicotinamide (0.1 mmol) were added to DCM, and the mixture was stirred overnight at room temperature. TLC indicated the reaction was complete. The reaction solution was added to 10 mL of water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((4-cyano-3-(trifluoromethyl)phenyl)amino)-3-(4-cyanophenoxy)-2-methyl-1-oxopropan-2-yl 1-methyl-1,4-dihydropyridine-3-carboxylate as a yellow powdery solid, with a yield of 91%.

¹H NMR (400 MHz, DMSO-d₆) δ 10.33 (s, 1H, NH), 8.30 (d, J = 2.0 Hz, 1H, CH), 8.18 - 8.11 (m, 1H, CH), 8.11 - 8.02 (m, 2H, 2*CH), 7.85 - 7.69 (m, 2H, 2*CH), 7.15 (d, J = 8.8 Hz, 2H, 2*CH), 7.03 (d, J = 1.6 Hz, 1H, CH), 5.87 - 5.80 (m, 1H, CH), 4.73 (dt, J = 7.7, 3.5 Hz, 1H, CH), 4.54 (d, J = 10.6 Hz, 1H, 1/2*CH₂), 4.45 (d, J = 10.6 Hz, 1H, 1/2*CH₂), 2.92 (s, 3H, CH₃), 2.89 (d, J = 3.4 Hz, 2H, CH₃), 1.66 (s, 3H, CH₃). LRMS (- ESI) 510.0 m/z: [(M-H)⁻, 100%].

### Example 36 Preparation of (S)-1-((6-cyano-5-(trifluoromethyl)pyridin-3-yl)amino)-3-(4-cyanophenoxy)-2-methyl-1-oxopropan-2-yl 1-methyl-1,4-dihydropyridine-3- carboxylate

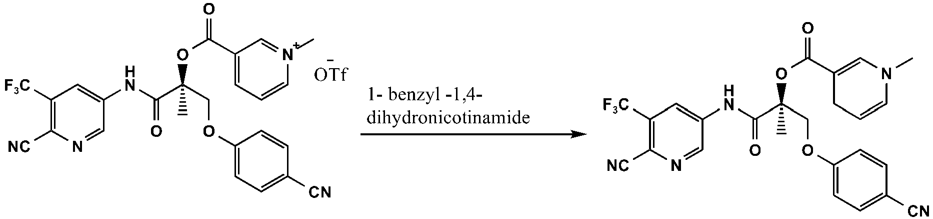

Under nitrogen atmosphere, (S)-3-(((1-((6-cyano-5-(trifluoromethyl)pyridin-3-yl)amino)-3-(4-cyanophenoxy)-2-methyl-1-oxopropan-2-yl)oxy)carbonyl)-1-methylpyridin-1-trifluoromethanesulfonate (0.1 mmol) and 1-benzyl-1,4-dihydronicotinamide (0.1 mmol) were added to DCM, and the mixture was stirred overnight at room temperature. TLC indicated the reaction was complete. The reaction solution was added to 10 mL of water and extracted with 20 mL of ethyl acetate. The organic phase was washed with water and saturated brine and dried over anhydrous sodium sulfate. After concentration under reduced pressure, the crude product was obtained, which was then purified by column chromatography to give the target compound (S)-1-((6-cyano-5-(trifluoromethyl)pyridin-3-yl)amino)-3-(4-cyanophenoxy)-2-methyl-1-oxopropan-2-yl 1-methyl-1,4-dihydropyridine-3-carboxylate as a yellow powdery solid, with a yield of 75%.

LRMS (+ESI) 512.1 m/z: [(M+H)⁺, 100%], LRMS (-ESI) 510.1/511.1 m/z: [(M-H)⁻, 100/30%].

### II Formulation Examples

Formulation Example A: Preparation of Injectable Formulation
(a) Batch formulation composition:

| | |
|---|---|
| Compound from Example 19 | 20 g |
| Polysorbate 80 | 20 g |
| Mannitol | 10 g |
| Water for injection | 5000 mL |
| 1000 vials | |

### (II) Preparation method:

According to the formulation, the compound from Example 19, polysorbate 80, and mannitol were added to 4000 mL of water for injection, and the mixture was stirred until dissolved. Water for injection was added to adjust the total volume to 5000 mL, and stirring was continued. The solution was subjected to sterile filtration through a 0.22 µm microporous membrane. The filtrate was aseptically filled into 5 mL ampoules at 5 mL per vial (specification: 20 mg per vial), and the ampoules were sealed and sterilized.

Formulation Example B: Preparation of Tablets
(a) Formula composition (dosage per 1000 tablets)

| | |
|---|---|
| Compound from Example 19 | 20 g |
| lactose | 100 g |
| Microcrystalline cellulose | 60 g |
| Pregelatinized starch | 40 g |
| Sodium carboxymethyl starch | 40 g |
| Micronized silica | 4 g |
| Magnesium stearate | 4 g |
| 0.3% HPMC | q.s. |
| 1000 pieces | |

### (II) Preparation process

The compound from Example 19, lactose, and a portion of microcrystalline cellulose were micronized in a ratio of 200:100:40. The remaining microcrystalline cellulose, lactose, pregelatinized starch, micronized silica gel, and sodium carboxymethyl starch (which had passed through an 80-mesh sieve) were added according to the formula ratio. After mixing evenly, an appropriate amount of 0.3% HPMC solution was added to the mixture to form a soft mass. Granulation was performed using an 18-mesh sieve, and the granules were dried at 60 °C (controlling the moisture content of the granules to around 3%). Magnesium stearate (which had passed through an 80-mesh sieve) was added and mixed evenly with the granules. The granules were then sized using a 16-mesh sieve, compressed into tablets, and packaged.

Formulation Example C: Preparation of Tablets
(a) Formula composition (dosage per 1000 tablets)

| | |
|---|---|
| Compound from Example 19 | 40 g |
| lactose | 50 g |
| Microcrystalline cellulose | 15 g |
| Pregelatinized starch | 10 g |
| Sodium carboxymethyl starch | 10 g |
| Micronized silica | 0.1 g |
| Magnesium stearate | 1 g |
| 0.3% HPMC | q.s. |
| 1000 pieces | |

### (II) Preparation process

The tablets of Formulation Example C were prepared in a similar manner to those of Formulation Example B above.

Formulation Example D: Preparation of Tablets
(a) Formula composition (dosage per 1000 tablets)

| | |
|---|---|
| Compound from Example 19 | 10 g |
| lactose | 10 g |
| Microcrystalline cellulose | 3 g |
| Pregelatinized starch | 2 g |
| Sodium carboxymethyl starch | 2 g |
| Micronized silica | 0.02 g |
| Magnesium stearate | 0.2 g |
| 0.3% HPMC | q.s. |
| 1000 pieces | |

### (II) Preparation process

The tablets of Formulation Example D were prepared in a similar manner to those of Formulation Example B above.

### III. Bioactivity Detection

### Small molecule-protein affinity (protein-ligand coupling) assay

### (1) Experimental equipment

Biacore ^{™} T200 by Cytiva^{®}, CM 5 series chip (purchased from Cytiva^{®}, catalog number: 29149604), recombinant human androgen receptor (AR) protein (CLOUD-CLONE CORP. WUHAN, RPB252Hu03, Ile673-His918 with N-terminal His Tag).

### (2) Experimental procedure

1) pH screening: The suitable ligand buffer pH was screened from 10 mM sodium acetate solutions at pH 5.5, 5.0, 4.5, and 4.0, with ligand concentrations ranging from 10 to 100 µg/mL. Contact time = 180s, flow rate = 5 µL/min. The surface was regenerated with 50 mM NaOH.
2) Ligand immobilization: Immobilization was performed using the direct amino coupling method. The ligands were first activated and immobilized using EDC/NHS, followed by blocking with ethanolamine. Automatic coupling was performed using the aim-for-immobilized-level mode with a target level of 1000 RU. After coupling, the coupling amount was detected using a response bound.
3) Surface testing: β₂-microglobulin solutions with concentrations of 85 nM, 8.5 nM, and 0.85 nM, and glycine-HCl regeneration solution were prepared and placed on the sample holder. Contact time = 180 s, wait time = 60s, the corresponding location of the regeneration solution was selected, contact time = 30 seconds, and the surface test-regeneration process was repeated for three samples. The binding between the analyte and the ligand, the suitability of the injection and dissociation time, and the estimated KD value were examined by the sensorgram results of channel 1 subtracted by channel 2.
4) Regeneration condition Selection: Regeneration was performed using glycine-HCl solutions with different pH values. The low rate of the regeneration solution was 30 µL/min.
5) A reasonable concentration gradient of the analyte was set according to the previous experimental results, and kinetic detection was performed. Kinetic/affinity calculation method was selected. For kinetic analysis, the Rₘₐₓ value should be less than 100 RU.

### (3) Experimental results

The binding affinity test results of compounds of the present disclosure as androgen receptor ligands with androgen receptor proteins are shown in the table below, with Ostarine as the control compound.

**Table 1. Results of in vitro small molecule-protein affinity of the compounds**

| Compound | Log P | Affinity KD (µM) |
|---|---|---|
| Ostarine | 3.49 | N/A |
| Ex. 1 | 2.62 | No binding |
| Ex. 2 | 3.53 | No binding |
| Ex. 3 | 2.53 | 27.3 |
| Ex. 4 | 2.53 | 3.7 |
| Ex. 5 | 1.61 | 0.2 |
| Ex. 11 | 3.32 | 0.0021 |
| Ex. 13 | 3.32 | 1706 |
| Ex. 14 | 2.86 | NT |
| Ex. 15 | 5.15 | 0.024 |
| Ex. 17 | 3.07 | NT |
| Ex. 19 | 3.20 | NT |
| Ex. 24 | 2.76 | 14.4 |
| Ex. 26 | 4.65 | 55.0 |
| Ex. 33 | NT | NT |
| Ex. 35 | 4.45 | NT |

| | | |
|---|---|---|
| Note: NT means Untested. | | |

Under the present experimental conditions, the compounds of Examples 3, 4, 5, 11, 15, 24, and 26 exhibited strong affinity to the androgen receptor proteins. In contrast, the control compound Ostarine showed no binding affinity to the androgen receptor proteins.

### Androgen Receptor Reporter Gene Assay (AR Reporter Gene Assay)

### 2.1 Experimental Overview

This experiment was performed to investigate the agonistic activity of the compounds of the present disclosure on androgen receptors. The positive control compound used in the experiment was dihydrotestosterone (DHT, MCE, HY-A0120). The experiment mainly included: seeding plasmid-transfected HEK293T cells (ATCC, CRL-3216) into well plates containing dilutions of the corresponding concentrations of the compounds; measuring the fluorescence values of different compounds using CellTiter-Fluor^{™} cell viability assay (Promega, G6081) and BritelitePlus luciferase assay (PerkinElmer, 6066769); calculating the relative receptor agonistic activity of the compounds of the present disclosure according to the formula, with DHT as a positive control; and simultaneously calculating the EC₅₀ by fitting the %activity values and the logarithm of the compound concentrations to a nonlinear regression.

### 2.2 Experimental Procedure

### (1) Preparation of Compounds

All test compounds were serially diluted 10-fold from 10 mM at a ratio of 1:3 in DMSO to a final concentration of 0.000508053 mM, with three replicates for each concentration. The positive control dihydrotestosterone was serially diluted 10-fold from 0.1 mM in DMSO at a 1: 3 ratio to a final concentration of 5.08053 E⁻⁰⁶ mM, with three replicates for each concentration. A 1000× positive control (0.1 mM dihydrotestosterone) and a 1000× vehicle control (100% DMSO) were prepared.

### (2) Experimental Operation

1) HEK293T cells were cultured according to ATCC recommendations and assayed during the exponential growth phase;
2) The culture medium was removed from the flask;
3) The cells were rinsed with PBS;
4) TrypLE solution was added to the flask and detach the cells. The cells were washed once with complete growth medium;
5) The cells were pelleted and washed twice with PBS to remove phenol red, and then resuspended in the culture medium to an appropriate concentration;
6) Only cells with a viability greater than 90% were used for the assay;
7) 6*10⁶ HEK293T cells were seeded into a 100 mm culture dish;
8) The cells were cultured at 37 °C under 5% CO₂ for 16 h;
9) The plasmid were transfected into the cells, and the cells were cultured at 37 °C under 5% CO₂ for 5-6 h;
10) 25 µL of the compound dilution was transferred to a 384-well plate using Echo 655;
11) HEK293T cells were seeded into 384-well assay plates at a density of 17,000 cells/well;
12) The cells were cultured at 37 °C under 5% CO₂ for 18-20 h;
13) 25 µL of CellTiter-Fluor ^{™} cell Viability Assay was added to each well of a 384-well plate, and the cells were cultured at 37 °C under 5% CO₂ for 30 min;
14) The values were read using ex 380 nm/em 510 nm;
15) 25 µL britelite plus luciferase assay reagent was added to each well of the 384-well assay plate, and the luminescence values were recorded on an Envision plate reader.

### (3) Data Processing

The EC₅₀ was calculated according to the following formula:
$Y = Bottom + \left(Top-Bottom\right) / \left(1+{10}^{∧}\left(\left({LogEC}_{50}-X\right)*Hillslope\right)\right)$

Wherein:
X represents the logarithm of the activator concentration; Y represents the inhibition percentage; Bottom represents the lowest plateau value of the curve; Top represents the highest plateau value of the curve; and Hillslope represents the slope of the curve.

The % relative effectiveness was calculated according to the following formula: 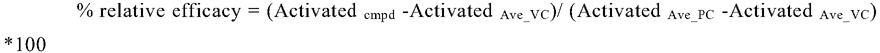 % relative efficacy = (Activated cmpd -Activated Ave_VC)/ (Activated Ave_PC -Activated Ave_VC) *100

Wherein:
Activated _{cmpd}: mean activation value of the compound signal across the entire plate.
Activated _{Ave_PC}: mean activation value of the positive control across the entire plate.
Activated _{Ave_VC}: mean activation value of the vehicle control across the entire plate.

### 2.3 Experimental Results

The results of androgen receptor agonist ability tests of the control compound and some example compounds are shown in Table 2 below.

**Table 2. Test results of the androgen receptor agonist ability of the compounds**

| Compound | Eₘₐₓ | EC₅₀ (nM) | % relative efficacy |
|---|---|---|---|
| DHT | 95.38 | 0.24 | 100 |
| Ostarine | 78.38 | 1.86 | 42.3 |
| Ex. 1 | 75.18 | >10 | 0 |
| Ex. 2 | NA | >10 | >10 |
| Ex. 3 | 90.11 | 1.05 | 23.1 |
| Ex. 4 | 67.67 | 3.06 | 7.9 |
| Ex. 5 | 82.43 | 1.38 | 17.6 |
| Ex. 6 | 88.26 | 3.45 | 22.8 |
| Ex. 7 | 78.99 | 2.76 | 28.4 |
| Ex. 8 | 96.25 | 0.81 | 97.2 |
| Ex. 11 | 60.79 | 6.89 | 3.5 |
| Ex. 13 | 93.18 | 2.93 | 26.8 |
| Ex. 15 | 107.90 | 22.01 | 3.6 |
| Ex. 21 | 86.51 | 14.12 | 5.6 |
| Ex. 22 | 92.22 | 34.10 | 2.3 |
| Ex. 24 | 100.10 | 3.01 | 26.1 |
| Ex. 25 | 82.94 | 8.53 | 9.2 |
| Ex. 28 | 95.73 | 5.77 | 13.6 |
| Ex. 30 | 88.03 | 5.41 | 14.5 |

Under the present experimental conditions, the compounds of Examples 3, 5, 8, 13, 15, and 24 exhibited excellent androgen receptor protein binding affinity and agonistic activity, while the remaining compounds also showed favorable binding affinity and agonistic activity. Among them, the nanomolar agonist activity (E_{MAX}) of the compound of Example 15 can reach up to 113% of that of DHT, indicating that it is a full androgen receptor agonist. Compared to Ostarine, a SARM-based compound currently undergoing clinical trials, all of the above compounds, except for those of Examples 1, 2, 4, and 11, demonstrate superior agonistic efficacy.

Furthermore, the above results also confirm that, compared with the corresponding arylpropionamide compounds in which the A-ring is a benzene ring, the novel arylpropionamide compounds of the present disclosure in which the A-ring is a pyridine ring have significantly improved androgen receptor agonistic potency, as shown below (wherein the KD results can be found in Table 1 above):

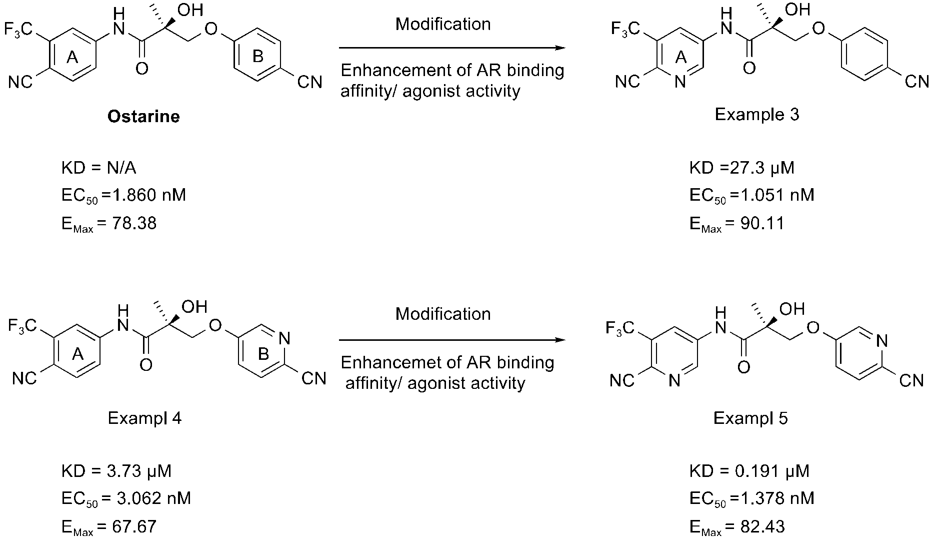

Meanwhile, compared with arylpropionamide compounds that retain free hydroxyl (i.e., L is a bond and R is hydrogen in Formula I), further modification of the hydroxyl can further enhance the maximum agonistic potency of the compound against the androgen receptor while maintaining relatively excellent agonistic potency (EC₅₀ < 20 nM), as shown below (where E_{MAX} results can be found in Table 2 above):

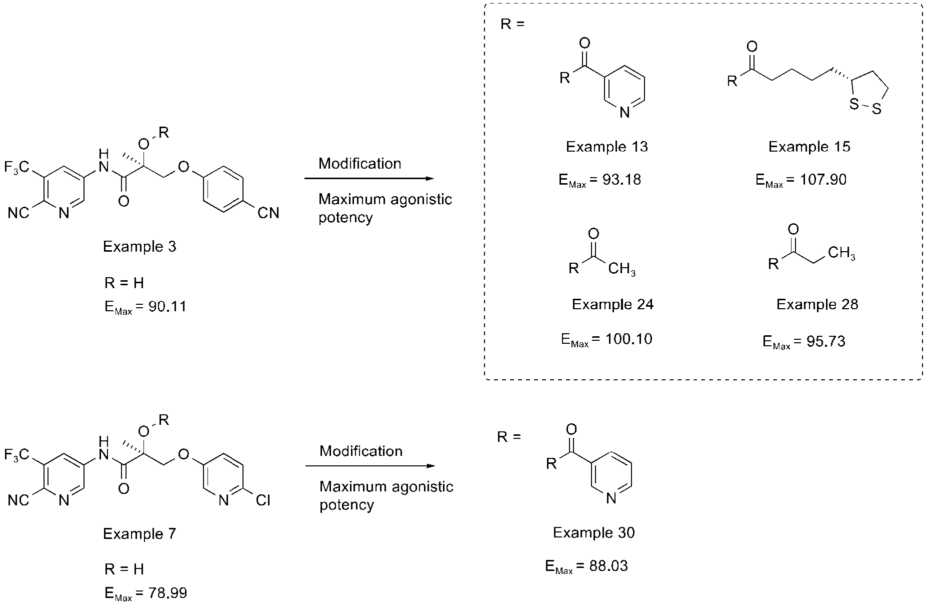

Mouse blood-brain barrier permeation assay

### 3.1 Animal experimental protocol and procedure:

(1) The experimental protocol was referenced and complied with the National Institutes of Health Guide for the Care and Use of Laboratory Animals, and was approved by the Laboratory Animal Welfare and Ethics Committee of the Chinese Institute for Brain Research (CIBR).
(2) Wild-type male C57BL/6J mice, aged 6-7 weeks and weighing 18-22 grams, were used. The mice were housed in a constant-temperature incubator with a 12-hour light-dark cycle and free access to food and water. Food was withheld one day before the experiment, while free access to water was permitted. The mice were injected intraperitoneally with the test compound at a dose of 20 mg/kg. Blood was collected by decapitation from the mice (each time point/3 mice) at the following time points: 30 min, 1 h, 2 h, 3 h, 4 h, 8 h, and brain tissue and blood were harvested at 12 h, 24 h, 36 h, 48 h and 72 h.
(3) When collecting serum, heparin sodium blood collection tubes were used. After sampling, the samples were immediately centrifuged at low speed to remove blood cells, and hemolysis was minimized as much as possible. After collection, the serum was sent to the mass spectrometry center for pretreatment.
(4) The brain tissue was rapidly dissected after treatment and frozen in liquid nitrogen for later use.

### 3.2 Mass spectrometry analysis experimental procedure:

### (1) Serum processing

The sample (100 µL) was warmed to the room temperature, acetonitrile:methanol (500 µL, v/v = 50:50) was added to the solution, and the sample was vortexed for 20 s. The sample was centrifuged at 5,000 g for 15 min at 4 °C. Subsequently, 90% of the supernatant was transferred to a glass tube and concentrated at 45 °C for 1.5 h using a vacuum concentrator. The dried sample was reconstituted in 400 µL of acetonitrile and water mixture (v/v = 50/50) and vortexed for 20 s. The resulting mixture was transferred to an EP tube and centrifuged at 5,000 g for 15 min at 4 °C. The supernatant was filtered through a 0.22 µm filter and analyzed by LC-MS/MS.

### (2) Brain sample processing

The brain sample (100 mg) was thawed, transferred to a homogenizer tube containing three zirconia beads, and homogenized. After processing as described for the serum samples, the supernatant was then filtered through a 0.22 µm filter, and analyzed by LC-MS/MS.

### 3.3 Experimental Results and Discussion

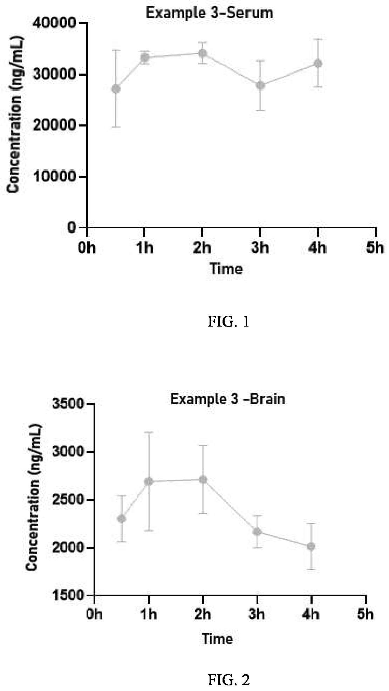

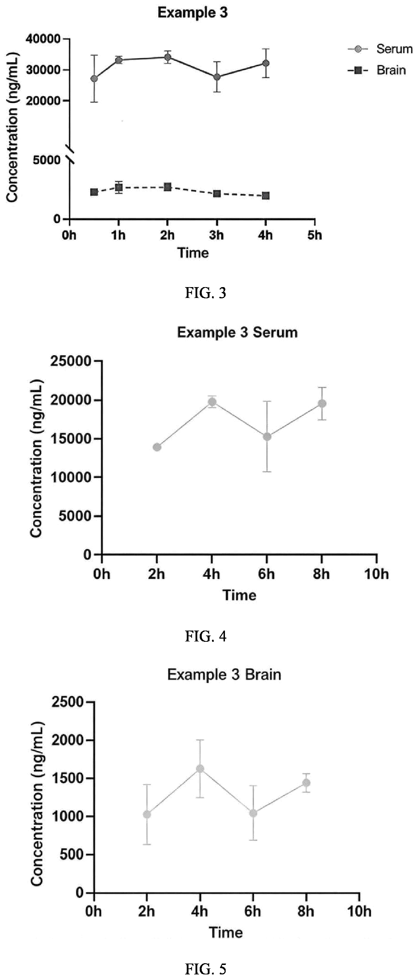

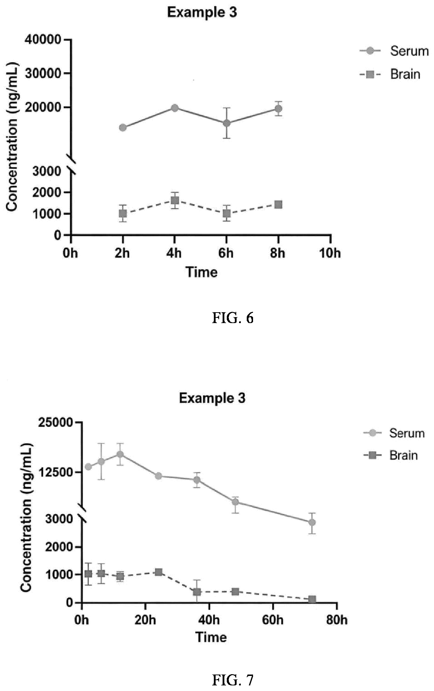

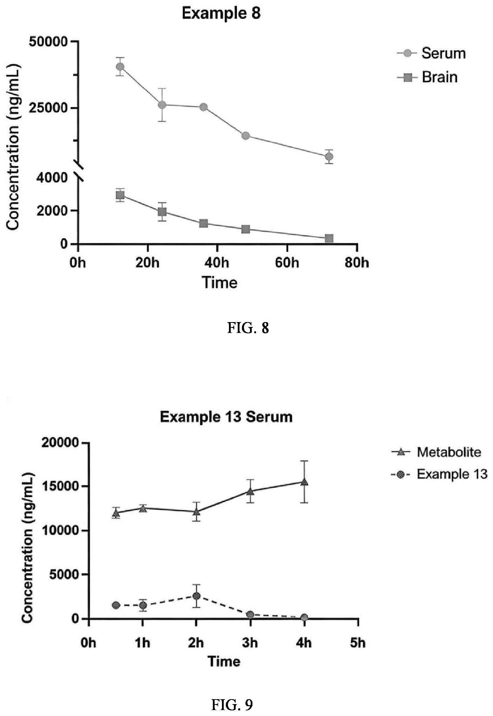

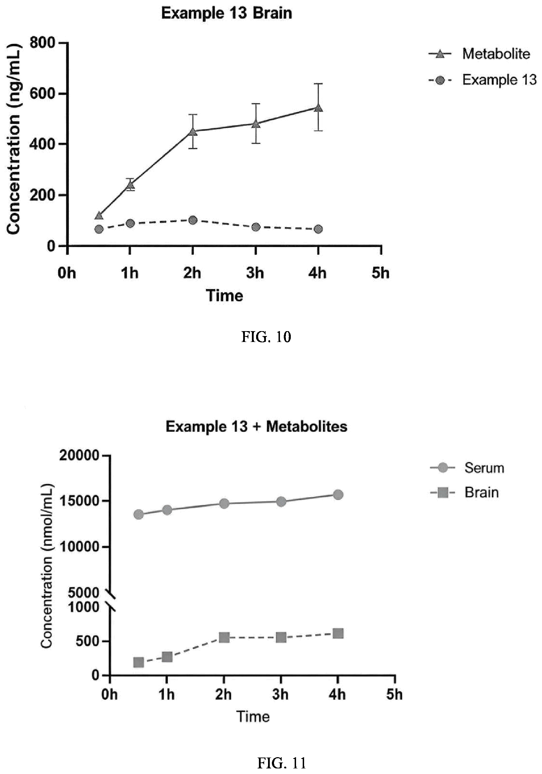

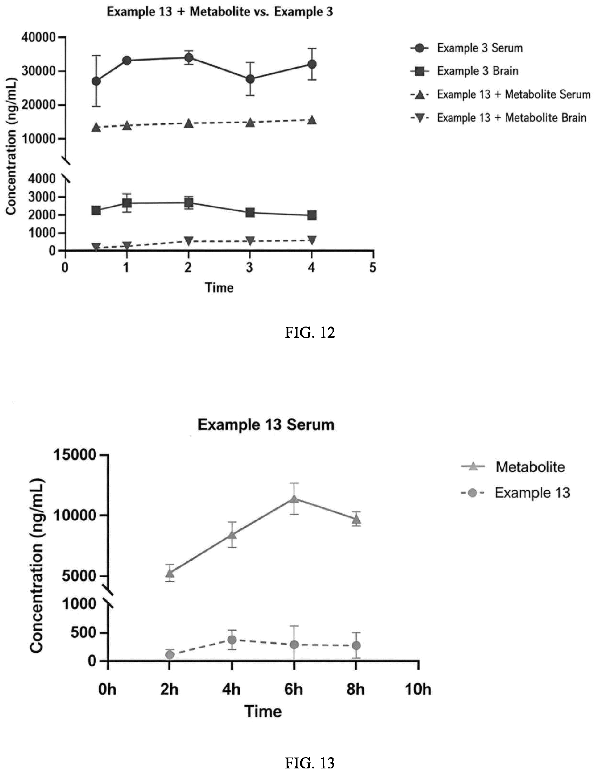

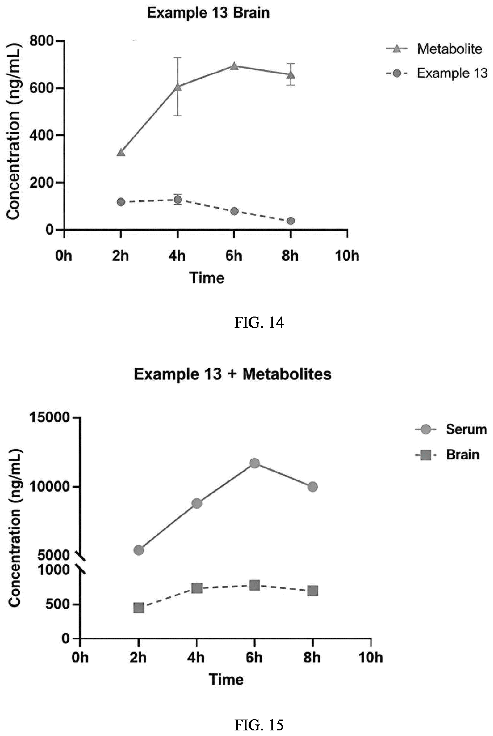

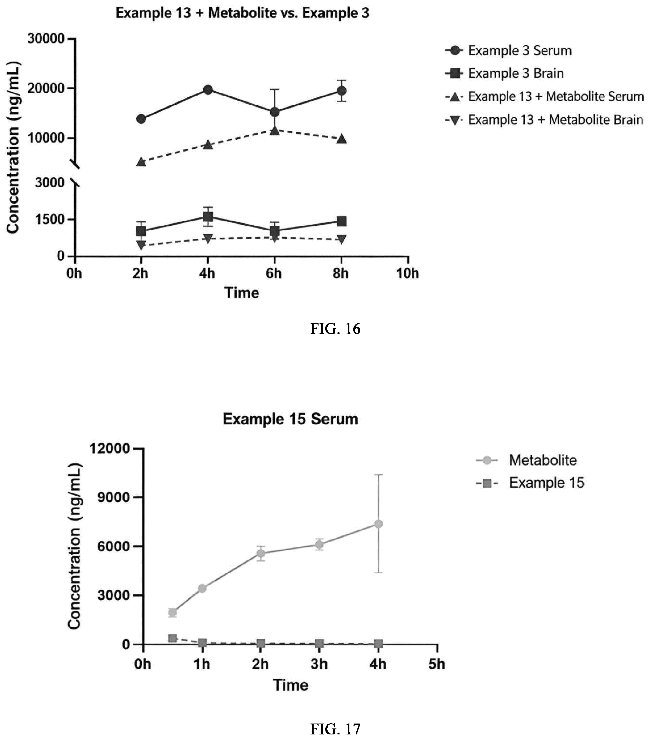

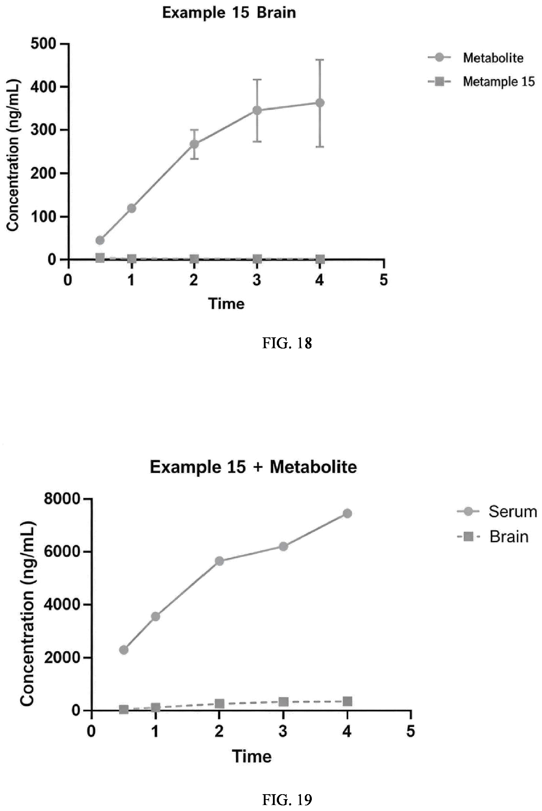

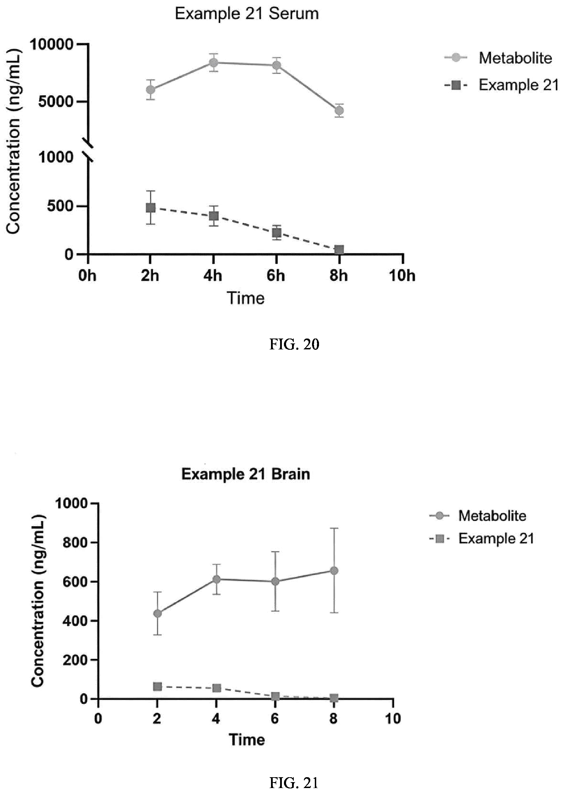

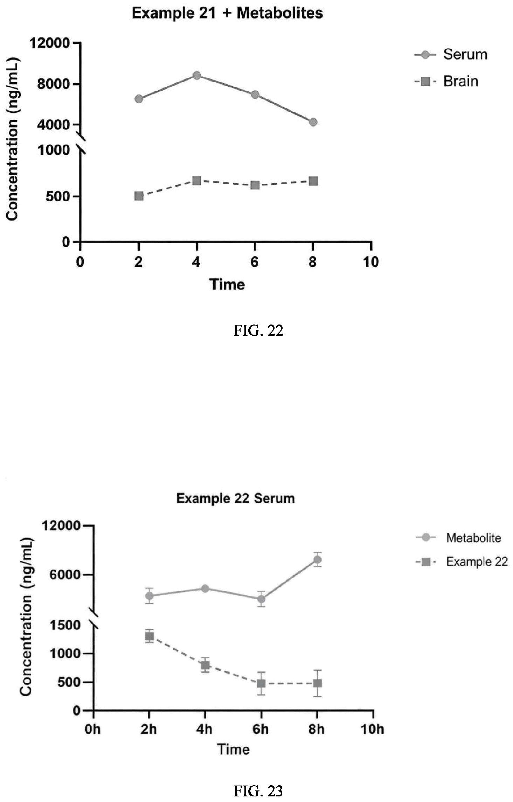

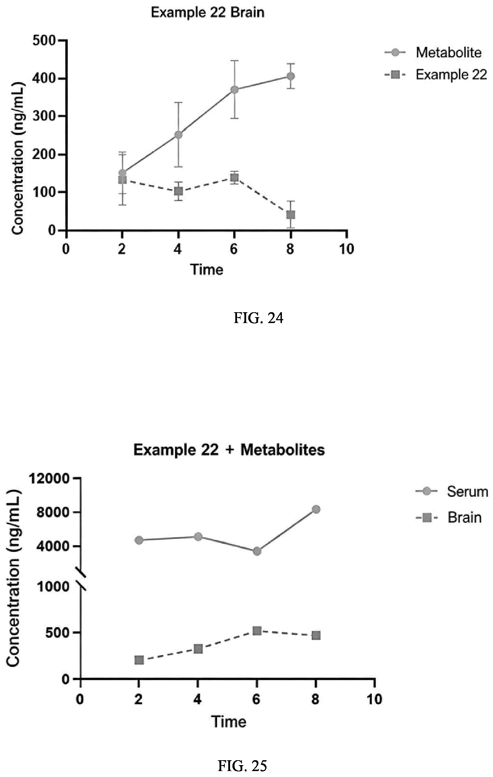

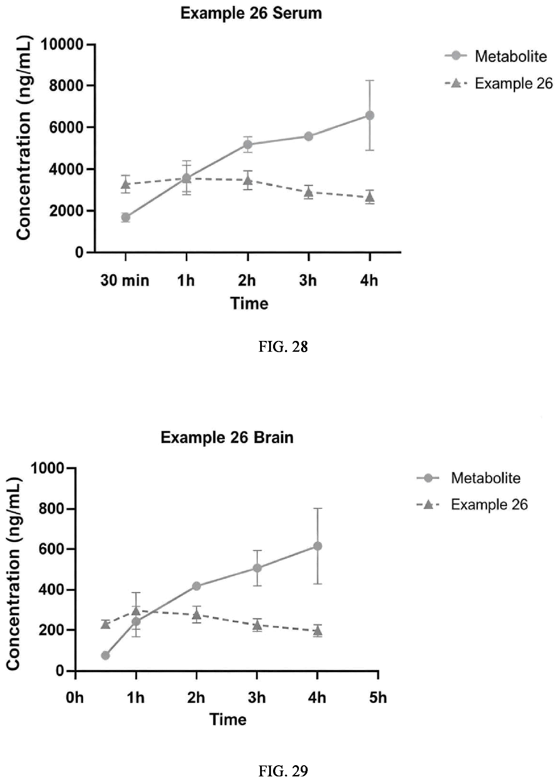

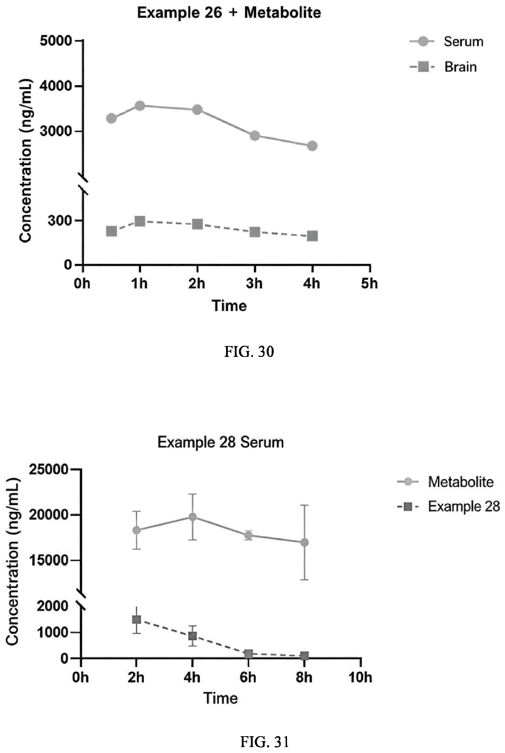

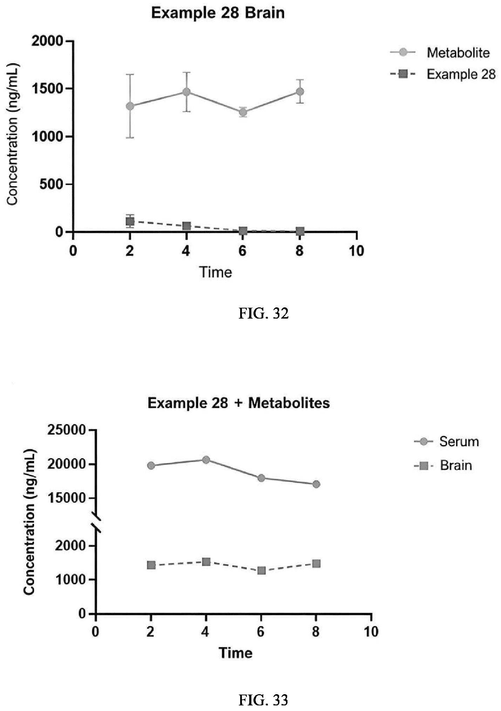

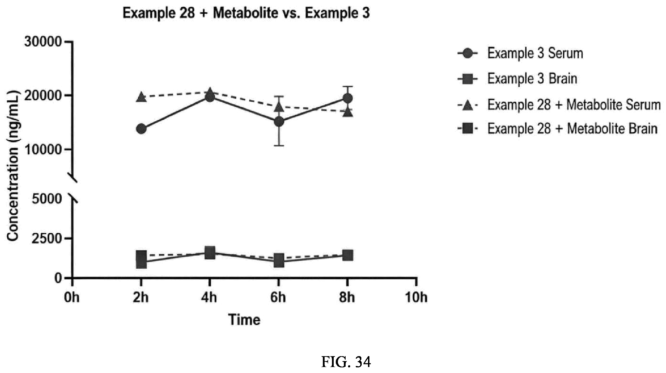

The concentration-time profiles of some example compounds in serum and brain post-dosing are shown in FIGS. 1 to 34.

Metabolites of the compounds in Examples 13, 15, 21, 22, 24, 26, and 28 were detected after administration. High-resolution mass spectrometry analysis confirmed that these metabolites corresponded to the compound of Example 3. The metabolites described herein for each example compound in the context therefore refer to the compound of Example 3.

For compounds of Formula I retaining a free hydroxyl, such as the compounds of Examples 3 and 8, certain concentrations of the compound were detected in serum and brain up to 72 h after administration (FIGS. 1-8), indicating that these compounds have good blood-brain barrier permeability. For compounds of Formula I with modified hydroxyl, such as the compounds of Examples 13, 21, 22, 26, and 28, lower concentrations of the compound and higher concentrations of metabolites were detected in serum and brain after administration, indicating that these compounds themselves have certain blood-brain barrier permeability, and were also converted into more potent metabolites in vivo through metabolic processes, thereby maintaining a certain drug concentration for a long time. For the compound of Example 15, no effective concentration of the compound was detected in serum and brain, but a higher concentration of metabolites was detected, indicating that the compound may undergo rapid metabolic processes in vivo, thereby being converted into metabolites to maintain a certain drug concentration.

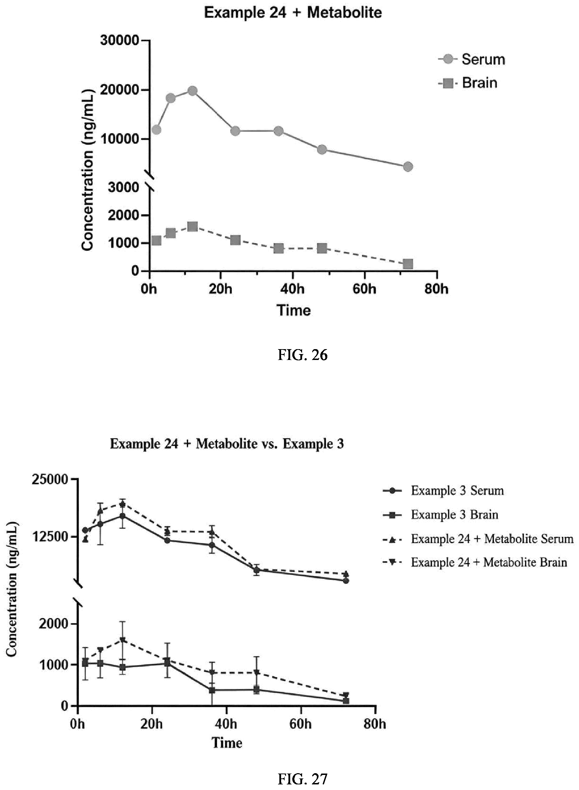

Surprisingly, although the compound of Example 24 was partially metabolized to the compound of Example 3 after administration, the total concentration of the parent drug and metabolites in the brain after administration of the compound of Example 24 was significantly higher than that of the compound of Example 3 after single administration, as shown in FIG. 27. Further, the bioavailability of the compounds of Example 24 and Example 3 after administration was compared, as shown in Table 3 below:

**Table 3. Comparison of AUC in mouse brains after administration of compounds in Ex. 3 and Ex. 24.**

| | 2h | 6h | 12h | 24h | 36h | 48h | 72h |
|---|---|---|---|---|---|---|---|
| Ex. 3 (AUC) | 1030.9 | 1048.7 | 947.7 | 1036.8 | 387.6 | 398.1 | 128.4 |
| Ex. 24 + Metabolites (AUC) | 1097.8 | 1359.0 | 1598.1 | 1113.2 | 811.8 | 811.0 | 274.1 |
| Increase percentage | +6.5% | +29.6% | +68.6% | +7.4% | +109.5% | +103.7% | +113.5% |

As can be seen from the results in the above table, the bioavailability of the compound in Example 24 in the brain was significantly improved after administration compared with the compound of Example 3.

In summary, the above bioactivity tests demonstrate that the novel arylpropionamide compounds designed in the present disclosure meet the basic requirements of neuroactive androgen receptor modulator and are a class of full androgen receptor agonists that can penetrate the blood-brain barrier.

The present disclosure has been described through the above embodiments. However, it should be understood that the above embodiments are for illustrative purposes only and are not intended to limit the disclosure to the scope of the described embodiments. Those skilled in the art will also understand that the present disclosure is not limited to the above embodiments, and many variations and modifications can be made based on the teachings of the present disclosure, all of which fall within the scope of protection claimed by the present disclosure. The scope of protection of the present disclosure is defined by the appended claims and their equivalents.

## Claims

1. Compounds of Formula I: 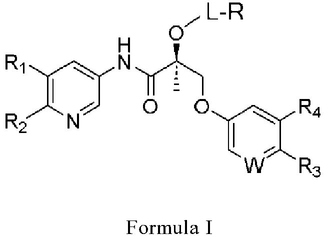 or pharmaceutically acceptable salts, stereoisomers, solvates, or isotopic derivatives thereof, wherein:
R₁ and R₂ are each independently cyano, halogen, C₁-C₆ haloalkyl, nitro or -NR₅R₆;
R₃ and R₄ are each independently hydrogen, cyano, halogen, C₁-C₆ haloalkyl, nitro, -NR₅R₆, -C(O)C₁-C₆ alkyl, -N(R₇)C(O)C₁-C₆ alkyl, -N(R₇)C(O)-C₁-C₆ haloalkyl, -C₁-C₆ alkyl-C(O)C₁-C₆ alkyl, -S(O)₂-C₁-C₆ alkyl, -N(R₇)-S(O)₂-C₁-C₆ alkyl, C₁-C₆ alkyl, or C₁-C₆ alkoxy;
W is CH or N;
L is a bond, -C(O)-, -C(O)O-, -S(O)₂-, or -C(O)NH-;
R is hydrogen, C₁-C₆ alkyl, C₃-C₈ cycloalkyl, C₆-C₁₀ aryl, 3- to 12-membered heterocyclyl, 3- to 12-membered heterocyclyl-C₁-C₆ alkyl, 5- to 12-membered heteroaryl, -C₁-C₆ alkyl-NR₅R₆, glucosyl, and amino acid, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents each independently selected from C₁-C₆ alkyl, C₁-C₆ alkoxy, halogen, and cyano; and
R₅, R₆ and R₇ are each independently hydrogen or C₁-C₆ alkyl.

2. Compounds of claim 1, or pharmaceutically acceptable salts, stereoisomers, solvates, or isotopic derivatives thereof, wherein the compounds have the following Formula II: 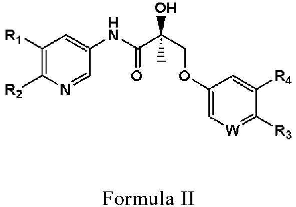 wherein R₁, R₂, R₃, R₄, and W are as defined in claim 1.

3. Compounds of claim 1, or pharmaceutically acceptable salts, stereoisomers, solvates, or isotopic derivatives thereof, wherein the compounds have the following Formula III: 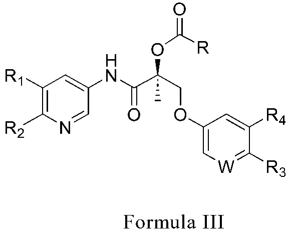 wherein R₁, R₂, R₃, R₄, W, and R are as defined in claim 1, provided that R is not hydrogen.

4. Compounds of claim 1, or pharmaceutically acceptable salts, stereoisomers, solvates, or isotopic derivatives thereof, wherein the compounds have the following Formula IV: 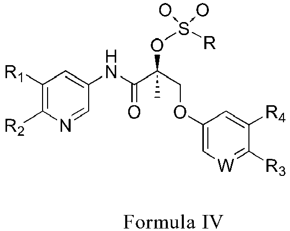 wherein R₁, R₂, R₃, R₄, W, and R are as defined in claim 1, provided that R is not hydrogen.

5. Compounds of any one of claims 1, 3 and 4, or pharmaceutically acceptable salts, stereoisomers, solvates, or isotopic derivatives thereof, wherein R is C₁-C₆ alkyl, C₃-C₈ cycloalkyl, C₆-C₁₀ aryl, 4- to 7-membered heterocyclyl, 4- to 7-membered heterocyclyl-C₁-C₆ alkyl, 5- to 10-membered heteroaryl, or -C₁-C₆ alkyl-NR₅R₆, wherein the cycloalkyl, aryl, heterocyclyl, and heteroaryl are optionally substituted by 1 or 2 substituents each independently selected from C₁-C₆ alkyl, C₁-C₆ alkoxy, halogen, or cyano.

6. Compounds of any one of claims 1-5, or pharmaceutically acceptable salts, stereoisomers, solvates or isotopic derivatives thereof, wherein R₁ and R₂ are each independently cyano, halogen, trifluoromethyl, difluoromethyl, nitro or -NH₂.

7. Compounds of any one of claims 1-6, or pharmaceutically acceptable salts, stereoisomers, solvates or isotopic derivatives thereof, wherein R₃ is cyano, halogen, C₁-C₆ haloalkyl, nitro, -NH₂, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl.

8. Compounds of any one of claims 1-7, or pharmaceutically acceptable salts, stereoisomers, solvates or isotopic derivatives thereof, wherein R₄ is hydrogen or halogen.

9. Compounds of any one of claims 1-8, or pharmaceutically acceptable salts, stereoisomers, solvates or isotopic derivatives thereof, wherein R₁ is trifluoromethyl or halogen, and R₂ is cyano.

10. Compounds of any one of claims 1-9, or pharmaceutically acceptable salts, stereoisomers, solvates or isotopic derivatives thereof, wherein R₃ is cyano or halogen, and R₄ is hydrogen or halogen.

11. Compounds of claim 1, or pharmaceutically acceptable salts, stereoisomers, solvates, or isotopic derivatives thereof, wherein:
R₁ and R₂ are each independently cyano, halogen, trifluoromethyl, difluoromethyl, nitro, or -NH₂;
R₃ is cyano, halogen, C₁-C₆ haloalkyl, nitro, -C(O)C₁-C₆ alkyl, -NHC(O)C₁-C₆ alkyl, -NHC(O)-C₁-C₆ haloalkyl, -S(O)₂-C₁-C₆ alkyl, or -NH-S(O)₂-C₁-C₆ alkyl;
R₄ is hydrogen or halogen;
W is CH or N;
L is a bond, -C(O)-or -S(O)₂-;
R is hydrogen, C₁-C₆ alkyl, C₃-C₈ cycloalkyl, C₆-C₁₀ aryl, 4- to 7-membered heterocyclyl, 4- to 7-membered heterocyclyl-C₁-C₆ alkyl, 5- to 10- membered heteroaryl, or -C₁-C₆ alkyl- NR₅R₆, wherein the heterocyclyl and heteroaryl contain 1 or 2 heteroatoms each independently selected from N, O, or S, and the cycloalkyl, aryl, heterocyclyl, and heteroaryl are optionally substituted by 1 or 2 substituents selected from C₁-C₆ alkyl; and
R₅ and R₆ are each independently hydrogen or C₁-C₆ alkyl.

12. Compounds of claim 1, or pharmaceutically acceptable salts, stereoisomers, solvates, or isotopic derivatives thereof, wherein:
R₁ is trifluoromethyl or halogen;
R₂ is cyano;
R₃ is cyano or halogen;
R₄ is hydrogen or halogen;
W is CH or N;
L is a bond, -C(O)-or -S(O)₂-; and
R is hydrogen, methyl, ethyl, n-propyl, isopropyl, cyclopentyl, phenyl, piperidinyl optionally substituted by methyl, pyrrolidinyl optionally substituted by methyl, dihydropyridinyl optionally substituted by methyl, pyridinyl, dimethylaminoethyl, and

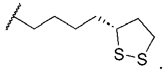

13. Compounds or pharmaceutically acceptable salts, stereoisomers, solvates, or isotopic derivatives thereof, wherein a compound is selected from the group consisting of: 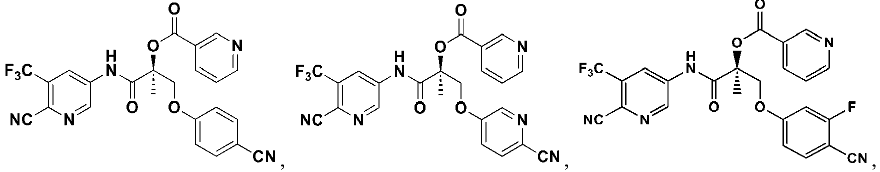 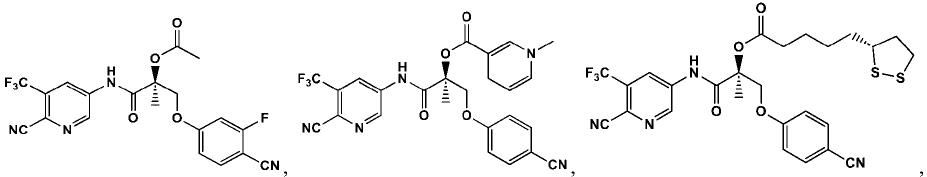 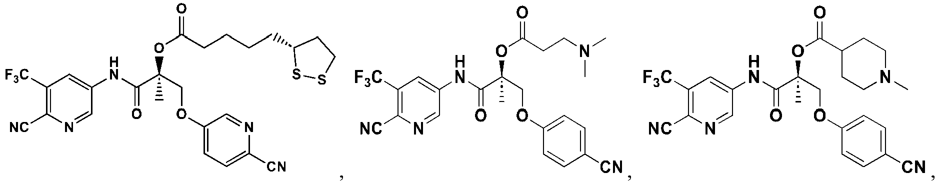 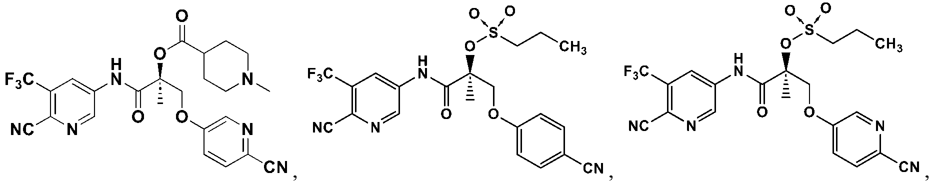 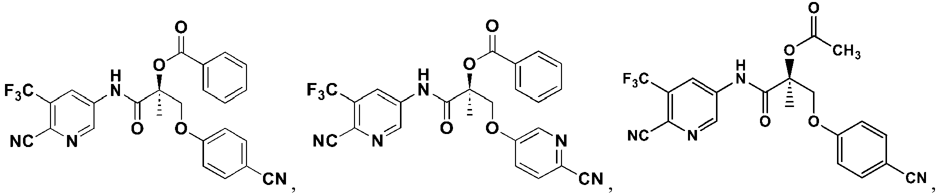 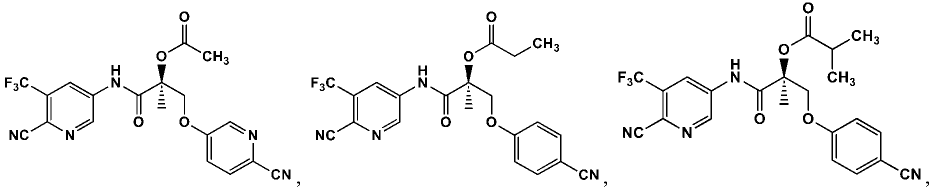 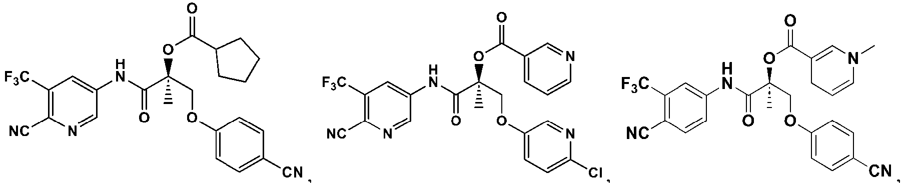 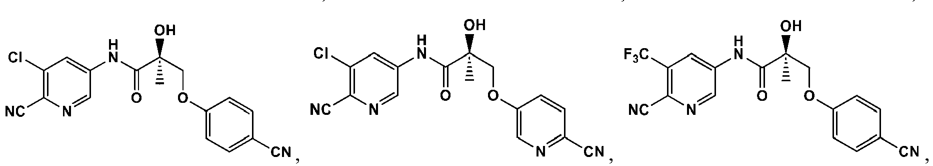 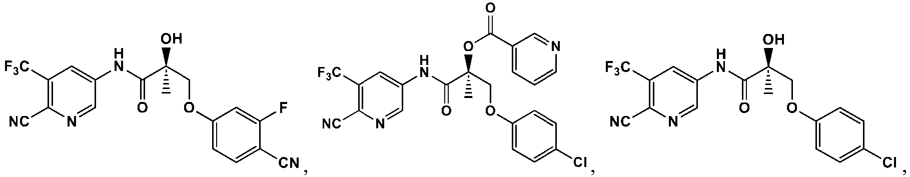 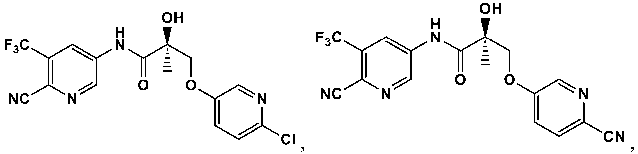 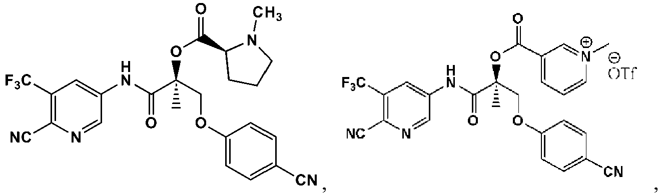 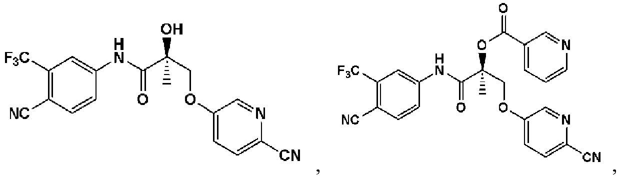 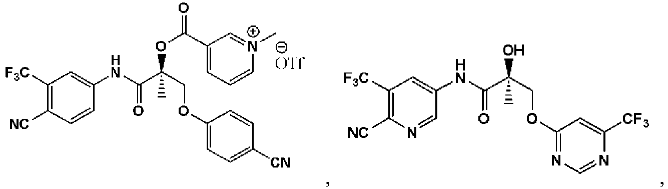 and

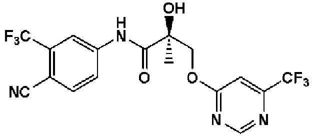

14. A pharmaceutical composition comprising the compound of any one of claims 1-13, or a pharmaceutically acceptable salt, stereoisomer, solvate or isotopic derivative thereof, and one or more pharmaceutically acceptable carriers.

15. Compounds of any one of claims 1-13, or a pharmaceutically acceptable salt, stereoisomer, solvate or isotopic derivative thereof, or a pharmaceutical composition of claim 14, for use in the preparation of a medicament for the prevention and/or treatment of androgen-related diseases.

16. The compounds for use or the pharmaceutical composition for use of claim 15, wherein the disease is a central nervous system disease selected from the group consisting of: dementia (including Alzheimer's disease), cognitive impairment associated with schizophrenia, Parkinson's disease, Huntington's disease, depression, anxiety, stroke, cerebral ischemia, amyotrophic lateral sclerosis, traumatic brain injury, Fragile X syndrome, Rett syndrome, brain tumors, and obesity.
